(19) Europäisches Patentamt / European Patent Office / Office européen des brevets

(11) EP 2 721 059 B1

(12) **FASCICULE DE BREVET EUROPEEN**

(45) Date de publication et mention de la délivrance du brevet:
**15.03.2017 Bulletin 2017/11**

(21) Numéro de dépôt: **12730867.4**

(22) Date de dépôt: **15.06.2012**

(51) Int Cl.:
***C07K 14/435*** (2006.01) ***G01N 33/542*** (2006.01)
***G01N 33/58*** (2006.01)

(86) Numéro de dépôt international:
**PCT/EP2012/061530**

(87) Numéro de publication internationale:
**WO 2012/172095 (20.12.2012 Gazette 2012/51)**

(54) **METHODE POUR GENERER DES PROTEINES FLUORESCENTES CYANS AYANT UNE SENSIBILITE REDUITE AU PH**

VERFAHREN ZUR ERZEUGUNG CYANFLUORESZIERENDER PROTEINE MIT REDUZIERTER PH-EMPFINDLICHKEIT

METHOD FOR GENERATING CYAN FLUORESCENT PROTEINS WHICH HAVE A REDUCED PH SENSITIVITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.06.2011 FR 1155227**

(43) Date de publication de la demande:
**23.04.2014 Bulletin 2014/17**

(73) Titulaires:
- **Centre National de la Recherche Scientifique (C.N.R.S.)**
  **75016 Paris (FR)**
- **Université Paris-Sud 11**
  **91400 Orsay (FR)**

(72) Inventeurs:
- **PASQUIER, Hélène**
  **91310 Montlhery (FR)**
- **MEROLA, Fabienne**
  **91190 Gif S/Yvette (FR)**
- **ERARD, Marie**
  **94250 Gentilly (FR)**
- **ESPAGNE, Agathe**
  **75014 Paris (FR)**
- **FREDJ, Asma**
  **75015 Paris (FR)**

(74) Mandataire: **Regimbeau**
**20, rue de Chazelles**
**75847 Paris Cedex 17 (FR)**

(56) Documents cités:
**EP-A1- 2 189 528**

- **TANSILA NATTA ET AL: "Rational design of analyte channels of the green fluorescent protein for biosensor applications", INTERNATIONAL JOURNAL OF BIOLOGICAL SCIENCES, vol. 3, no. 7, 2007, pages 463-470 URL, XP009154977, ISSN: 1449-2288**
- **MARKUS H. J. SEIFERT ET AL: "Backbone Dynamics of Green Fluorescent Protein and the Effect of Histidine 148 Substitution +", BIOCHEMISTRY, vol. 42, no. 9, 1 mars 2003 (2003-03-01), pages 2500-2512, XP055014707, ISSN: 0006-2960, DOI: 10.1021/bi026481b**
- **TOMOSUGI WATARU ET AL: "An ultramarine fluorescent protein with increased photostability and pH insensitivity", NATURE METHODS, NATURE PUBLISHING GROUP, GB, vol. 6, no. 5, 1 mai 2009 (2009-05-01), pages 351-353, XP002595745, ISSN: 1548-7091, DOI: 10.1038/NMETH.1317**
- **RANIERI BIZZARRI ET AL: "Green fluorescent protein based pH indicators for in vivo use: a review", ANALYTICAL AND BIOANALYTICAL CHEMISTRY, SPRINGER, BERLIN, DE, vol. 393, no. 4, 26 novembre 2008 (2008-11-26), pages 1107-1122, XP019702540, ISSN: 1618-2650**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 721 059 B1

- P.G. MAZZOLA ET AL: "Stability of Green Fluorescent Protein (GFP) in Chlorine Solutions of Varying pH", BIOTECHNOLOGY PROGRESS, vol. 22, no. 6, 1 décembre 2006 (2006-12-01), pages 1702-1707, XP055015015, ISSN: 8756-7938, DOI: 10.1021/bp060217i

## Description

### INTRODUCTION

La présente description fournit une méthode pour générer des protéines fluorescentes cyans ayant une sensibilité réduite au pH.

Les protéines fluorescentes (appelées également sondes fluorescentes), telles que la protéine fluorescente verte extraite de la méduse *Aequoria Victoria* (Green Fluorescent Protein ou GFP*av*) et ses variants, constituent un outil essentiel pour l'exploration du vivant, notamment pour des techniques d'imagerie de fluorescence, de cytométrie de flux et d'essais biologiques à haut débit. Fusionnées à diverses protéines, ces sondes fluorescentes permettent de déterminer la localisation et le trafic intracellulaire de ces protéines, et d'analyser divers processus biologiques tels que les interactions protéine-protéine, les activités enzymatiques ou les voies de signalisation second messager. Le succès de ces protéines repose notamment sur le fait qu'elles puissent être exprimées dans de très nombreux organismes vivants et compartiments cellulaires (e.g., noyau, mitochondrie, appareil de Golgi). Les nombreuses approches de mutagénèse dirigée et aléatoire conduites ces quinze dernières années ont ainsi donné lieu à une grande diversité de variants de la GFP*av* native (Day et al., 2009). Ceux-ci diffèrent de la GFP*av* par la mutation de quelques acides aminés et fluorescent dans une gamme de couleur différente allant du bleu au rouge.

Parmi les variants de la GFP*av* les plus utilisés, se trouve la protéine fluorescente cyan améliorée ou Enhanced Cyan Fluorescent Protein (ECFP*av* ou ECFP). Cette protéine possède 239 acides aminés dont 6 à 9 sont mutés par rapport à la GFP*av* native (K26R, S65T, Y66W, F64L, N146I, M153T, V163A, N164H, H231L) (Cubitt et al., 1995 ; Llopis et al., 1998 ; Cubitt et al., 1999). La substitution de l'acide aminé Tyr66 par un Tryptophane a conduit à un décalage des spectres d'excitation et d'émission vers le bleu par rapport à la GFP*av*, respectivement à 436 et 475 nm, tandis que les autres mutations ont permis d'améliorer certaines propriétés physico-chimiques, telles que le repliement protéique, la solubilité, la photostabilité, et la brillance. La brillance de cette ECFP demeure néanmoins bien inférieure à celle de la GFP améliorée (40%).

La ECFP est une des protéines fluorescentes les plus couramment employées à ce jour comme donneur dans les études d'imagerie par transfert d'énergie par résonance de type Förster (FRET), plus particulièrement en tandem avec la EYFP (Miyawaki et al., 1999) et ses variants, du fait de la superposition partielle de leurs spectres d'absorption et d'émission. Le couplage de la ECFP à ce type de protéine a ainsi permis de développer de nombreux biosenseurs FRET, notamment pour étudier le métabolisme cellulaire.

Or le FRET requiert, comme toutes les méthodes d'imagerie en temps réel, une analyse quantitative très précise des signaux de fluorescence, ce qui est rarement le cas des techniques conventionnelles de microscopie. Les propriétés d'émission complexes et peu performantes de la ECFP ne permettent donc pas d'interpréter de manière fiable les résultats obtenus par imagerie quantitative dans les cellules vivantes. En effet, la ECFP présente plusieurs états d'excitation révélant l'existence de plusieurs conformations distinctes pouvant être adoptées par cette protéine, ainsi qu'un faible rendement quantique et une durée de vie de fluorescence écourtée par rapport à la GFP*av* (Shaner et al., 2007 ; Patterson et al., 2001 ; Tramier et al. 2002).

Tout comme la plupart des sondes fluorescentes, la ECFP se caractérise par une très forte sensibilité aux facteurs environnementaux, en particulier au pH (Miyawaki et al., 2000). Sa durée de vie de fluorescence moyenne (Tau ou $\tau$) diminue légèrement entre pH 11 et pH 7 mais est réduite de manière importante de 2,5 ns à 1,5 ns lorsque le pH tombe à pH 5,5. Il est également connu que son intensité de fluorescence ($I_f$) chute quant à elle rapidement de $\sim$ 10% à pH 6,5 et de $\sim$ 40% à pH 5,5. Cette sensibilité au pH constitue un inconvénient majeur dans l'utilisation de la ECFP en imagerie quantitative dans des cellules intactes puisque le pH intracellulaire varie selon les compartiments cellulaires et les conditions expérimentales testées, telles que la stimulation mitogène ou le stress métabolique. Ainsi, lorsque la ECFP est solubilisée dans deux compartiments cellulaires de pH différent (par exemple, le pH neutre du cytosol et le pH acide des lysosomes), elle présente des propriétés d'émission distinctes indépendamment du processus biologique étudié, pouvant potentiellement conduire à des interprétations artéfactuelles. A ce jour la seule possibilité permettant de réduire ces artéfacts est de vérifier régulièrement le pH ambiant ou de le fixer (« clamping »). Il serait donc fortement souhaitable de pouvoir disposer d'une ECFP ayant une sensibilité réduite au pH et dont le pH de demi-transition ($pH_{1/2}$) serait bien inférieur au pH physiologique.

Les efforts entrepris à ce jour pour améliorer les performances photophysiques et physico-chimiques de la ECFP se sont principalement concentrés sur l'amélioration de sa brillance (ou intensité de fluorescence), de sa maturation, sa solubilisation, ainsi que sur la simplification de ses propriétés d'émission, notamment de son déclin d'émission de fluorescence (Rizzo et al. (2004) ; Nguyen et al.(2005) ; Goedhart et al. (2010) ; Sawano et al. (2000)). Toutefois, aucune étude n'a porté spécifiquement sur le développement de protéines fluorescentes cyans présentant une sensibilité réduite au pH, et dont le spectre de fluorescence caractéristique de la ECFP a pu être conservé.

Il existe donc un besoin de développer une méthode permettant de générer des protéines fluorescentes cyans ayant une sensibilité réduite au pH, tout en conservant les propriétés spectrales propres à ces protéines.

## DESCRIPTION DETAILLEE

La présente description fournit une méthode permettant de générer des protéines fluorescentes cyans ayant une sensibilité réduite au pH, en particulier aux pH acides. Outre les protéines fluorescentes cyans susceptibles d'être obtenues par ladite méthode, les autres aspects de la description concernent les acides nucléiques codant pour lesdites protéines, des vecteurs recombinants comprenant lesdits acides nucléiques, des cellules hôtes exprimant lesdites protéines, des biosenseurs comprenant lesdites protéines, ainsi que l'utilisation desdites protéines, desdits acides nucléiques, desdites cellules hôtes et desdits biosenseurs.

La présente invention a notamment pour objet une méthode telle que revendiquée dans la revendication 1.

Les présents inventeurs ont en effet découvert que la sensibilité au pH de la protéine fluorescente cyan ECFP est fortement gouvernée par la nature d'acides aminés spécifiques, et plus particulièrement des acides aminés en position 65 et 148 de la séquence protéique de la ECFP. La méthode de l'invention comprend donc une étape dans laquelle une mutation est introduite dans la protéine fluorescente cyan ECFP de séquence SEQ ID N°2, préférablement en position 65 et/ou 148 de cette séquence.

En outre, bien que l'introduction d'une simple mutation puisse affecter de manière dramatique les propriétés physico-chimiques, notamment spectrales, des protéines fluorescentes (Espagne et al., 2011 ; Sawano et al., 2000), l'introduction de certaines mutations identifiées par les inventeurs en position 65 et/ou 148 de la séquence SEQ ID N°2, ne produit pas d'effet négatif sur lesdites propriétés, et permet par ailleurs d'accroître la durée de vie moyenne de fluorescence (Tau ou $\tau$) à pH neutre de ces protéines, et d'abaisser le $pH_{1/2}$.

Les « protéines fluorescentes cyans » désignent ici toutes protéines fluorescentes mutées issues de la ECFP d'*Aequora victoria* (ECFP) de séquence protéique SEQ ID N°2, et dont le spectre d'absorption présente un maximum d'absorbance compris entre 415 et 450 nm et dont le spectre d'émission présente un maximum de fluorescence compris entre 470 et 490 nm. De manière préférée, le spectre d'absorption desdites protéines présente un maximum d'absorbance à environ 435 nm, et leur spectre d'émission présente un maximum de fluorescence à environ 476 nm. Le spectre d'absorption, correspond aux longueurs d'onde pour lesquelles la protéine fluorescente est excitée, tandis que le spectre d'émission correspond aux longueurs d'onde dans lesquelles la protéine émet de la fluorescence.

Les protéines fluorescentes cyans mutées décrites ici ont donc l'avantage de présenter un spectre d'absorption et d'émission similaire à celui de la ECFP de SEQ ID N°2. En outre, l'intensité de fluorescence de ces protéines ($I_f$) et leur durée de vie de fluorescence moyenne (Tau ou $\tau$) restent stables et plus élevées sur un domaine de pH plus étendu.

La méthode décrite ici permet ainsi d'obtenir des protéines fluorescentes cyans dont les pertes d'intensité de fluorescence ($I_f$) et de durée de vie de fluorescence moyenne ($\tau$) à pH acide sont inférieures à celles de la ECFP, c'est-à-dire respectivement inférieures à 50% et 33%, préférablement inférieures à 30%, encore plus préférablement inférieures à 20%. De manière encore plus préférée, ces pertes sont nulles.

La durée de vie moyenne à pH neutre de ces protéines mutées est par ailleurs accrue par rapport à la ECFP. Cette durée de vie est ainsi supérieure à 2,5 nanosecondes (ns), et peut atteindre 4,12 ns.

Le $pH_{1/2}$ des protéines de l'invention est également diminué à un $pH_{1/2}$ inférieur à celui de la ECFP, puisqu'il est inférieur à 5,6, et peut atteindre une valeur de $pH_{1/2}$ de 3,4, voire de 3,1.

Un autre avantage particulier de la présente méthoderéside dans le fait que ces mutations uniques peuvent être introduites directement dans les biosenseurs existants, qui sont plus particulièrement utilisés dans les applications de FRET. Les protéines fluorescentes cyans selon l'invention peuvent être notamment couplées, au sein de biosenseurs, à des protéines fluorescentes oranges (TagRFP), ou jaunes (de type YFP), dont la sensibilité au pH a été réduite. Le développement de tels biosenseurs peut ainsi permettre d'étudier divers processus biologiques dans toute condition de pH, et en particulier à pH acide, ce qui était jusque-là impossible du fait de la sensibilité au pH de la protéine ECFP. A cet égard, les inventeurs ont développé un biosenseur présentant une sensibilité réduite au pH, dans lequel une protéine fluorescente cyan de l'invention a été couplée à la protéine fluorescente orange TagRFP.

La présente méthode permet ainsi de répondre aux exigences requises par les méthodes actuelles d'imagerie quantitative en temps réel, et de pouvoir utiliser des protéines fluorescentes cyans dans des conditions de pH acide.

La présente description a pour premier aspect une méthode pour générer des protéines fluorescentes cyans présentant une sensibilité réduite au pH comprenant une étape a), selon laquelle une mutation unique est introduite dans une séquence protéique comprenant la séquence SEQ ID N°2, telle que décrite ci-dessous :

VSKGEELFTGVVPILVELDGDVNGHKFSVSGEGEGDATYGKLTLKFICTTGKLP VPWPTLVTTLTWGVQCFSRYPDHMKQHDFFKSAMPEGYVQERTIFFKDDGNYKTRAE VKFEGDTLVNRIELKGIDFKEDGNILGHKLEYNYISHNVYITADKQKNGIKANFKIRHNI EDGSVQLADHYQQNTPIGDGPVLLPDNHYLSTQSALSKDPNEKRDHMVLLEFVTAAG ITLGMDELYK (SEQ ID N°2).

Par «comprenant» ou « contenant », on entend ici que les éléments énumérés sont requis ou obligatoires, mais que d'autres éléments optionnels peuvent être ou non présents.

Ainsi, par «séquence protéique comprenant la séquence SEQ ID N°2 », on entend ici que la séquence SEQ ID N°2 peut comprendre d'autres acides-aminés en ses extrémités N- ou C-terminal, tels qu'une méthionine en N-terminal, une séquence peptide signal ou encore une séquence d'acides aminés permettant la purification de la protéine. Ces dernières pourront être choisies par l'homme du métier parmi les séquences peptidiques n'affectant pas les propriétés fonctionnelles de protéines, telle qu'une séquence polyhistidine et /ou une séquence de site de clivage par une protéase (e.g. SEQ ID N°70 de séquence MSYYHHHHHHDYDIPTTENLYFQGA). Ainsi la séquence protéique à muter peut comprendre ou consister en la séquence SEQ ID N°4.

Selon un autre mode de réalisation préféré, ladite séquence SEQ ID N°2 ne comprend pas d'autres acides aminés, et ladite séquence protéique consiste alors uniquement en la séquence SEQ ID N°2.

De manière préférée, ladite mutation est introduite en position 65 ou 148 de la séquence SEQ ID N°2.

Selon un mode particulier, la méthode comprend une étape supplémentaire b) selon laquelle une mutation additionnelle est introduite en position 65 de la séquence SEQ ID N°2 si la mutation de l'étape a) est introduite en position 148. Réciproquement, l'étape b) de ladite méthode consiste en l'introduction d'une mutation additionnelle en position 148 de la séquence SEQ ID N°2 si la mutation de l'étape a) est introduite en position 65.

Selon un autre mode particulier, la méthode consiste en l'étape a) et l'étape b), telles que définies ci-dessus.

Selon un mode avantageux, la méthode consiste uniquement en l'étape a) telle que définie ci-dessus.

De manière préférée, selon les différents modes de réalisation de la méthode telle que décrite ci-dessus, l'acide aminé en position 65 est substitué par une sérine et/ou l'acide aminé en position 148 est substitué par une glycine, une alanine, une sérine, ou un acide glutamique. De manière encore plus préférée, ledit acide aminé en position 148 est substitué par une glycine, une alanine, ou une sérine. Selon un autre mode préféré, l'acide aminé en position 65 est substitué par une sérine, et l'acide aminé en position 148 est substitué par une glycine, un acide aspartique, un acide glutamique ou une sérine, préférentiellement par une glycine, un acide aspartique ou une sérine. De manière encore plus préférée, l'acide aminé en position 65 est substitué par une sérine et l'acide aminé en position 148 est substitué par une glycine.

Il est entendu que la présente méthode, qu'elle comprenne ou consiste en l'étape

a) ou en l'étape a) et b), permet de générer des protéines fluorescentes cyans telles que définies ci-dessus et présentant une sensibilité réduite au pH.

Dans le cas où l'acide aminé en position 65 est substitué par une sérine, les protéines fluorescentes cyans obtenues présentent également un rendement quantique accru, une cinétique de fluorescence simplifiée, un photoblanchiment réversible réduit ainsi qu'un photoblanchiment irréversible ralenti. De par ces propriétés photophysiques avantageuses, les protéines fluorescentes cyans de l'invention comprenant au moins la mutation 65S sont particulièrement utiles dans les applications d'imagerie dans des cellules vivantes, telles que les applications de type FRET ou FLIM.

Selon la méthode ici décrite, on entend par « mutation» une altération de la séquence d'acides aminés SEQ ID N°2 de la protéine ECFP, suite à la modification de la séquence nucléotidique SEQ ID N°1 codant pour ladite protéine. La mutation ici décrite peut être une addition, une délétion ou une substitution d'un acide aminé par un autre acide aminé par rapport à la séquence protéique d'origine. De manière préférée, ladite mutation est une substitution.

Les méthodes permettant d'introduire une mutation dans une séquence nucléotidique sont connues de l'homme du métier. Par exemple, il est possible d'introduire une mutation par mutagénèse aléatoire ou dirigée, par PCR en utilisant des oligonucléotides dégénérés, par radiation ou en utilisant un agent mutagène. Lesdites techniques sont notamment décrites par Sambrook et al. dans "Molecular Cloning : A laboratory Manual", 3ème édition, Cold Spring Harbor Laboratory Press, (2001), et par Ausubel et al. dans "Current Protocols in Molecular Biology", John Wiley & Sons (2011). De manière préférée, la mutation est introduite par mutagénèse dirigée. Il est bien entendu que pour introduire lesdites mutations, l'homme du métier pourra utiliser des codons (ou triplets de nucléotides) fonctionnellement équivalents, c'est-à-dire des codons qui codent les mêmes acides aminés, ou des acides aminés biologiquement équivalents.

Par ailleurs, si l'homme du métier souhaite optimiser l'expression de la protéine fluorescente cyan mutée de l'invention, il pourra se référer à la base de données référencée sur le site internet http://www.kazusa.or.jp/codo/, qui répertorie l'usage optimal de ces codons dans divers organismes et organelles.

Par « acide aminé », on entend ici tous les résidus des acides $\alpha$-aminés naturels (par exemple alanine (Ala, A), arginine (Arg, R), asparagine (Asn, N), acide aspartique (Asp, D), cystéine (Cys), glutamine (Gln, Q), acide glutamique (Glu, E), glycine (Gly, G), histidine (His, H), isoleucine (Ile, I), leucine (Leu, L), lysine (Lys, K), méthionine (Met, M), phénylalanine (Phe, F), proline (Pro, P), sérine (Ser, S), thréonine (Thr, T), tryptophane (Trp, W), tyrosine (Tyr, Y) et valine (Val, V) sous la forme D ou L), ainsi que les acides aminés non naturels.

Un autre aspect de l'invention porte sur les protéines fluorescentes cyans ayant une sensibilité réduite au pH susceptibles d'être obtenues selon la méthode décrite plus haut, ladite méthode comprenant ou consistant en l'étape a), ou en l'étape a) et b).

Par « sensibilité au pH » ou dépendance au pH, on entend ici la perte d'intensité de fluorescence ($I_f$) et/ou la diminution de la durée de vie de fluorescence (Tau ou $\tau$) d'une protéine fluorescente cyan lorsque le pH du milieu dans lequel se trouve la protéine passe d'un pH basique à un pH acide. La sensibilité au pH peut être définie par au moins un des deux critères ci-dessus.

De manière préférée, on entend par « sensibilité au pH » la perte d'intensité de fluorescence ($I_f$) et la diminution de la durée de vie de fluorescence (Tau ou $\tau$) d'une protéine fluorescente cyan lorsque le pH du milieu dans lequel se trouve la protéine passe d'un pH basique à un pH acide. Selon la présente description, les termes perte, abaissement, diminution, déclin ou réduction sont synonymes, et peuvent être utilisés alternativement.

Les termes intensité de fluorescence ($I_f$), rendement quantique, ou brillance sont interdépendants. Dans le contexte de la description, le terme « intensité de fluorescence » signifie le nombre de photons émis par une protéine fluorescente par unité de volume et par unité de temps à une longueur d'onde d'émission donnée. Le terme de « rendement quantique » désigne le rapport de l'intensité de fluorescence émise sur l'ensemble du spectre d'émission et de l'intensité absorbée de ladite protéine. Le terme de « brillance » désigne quant à lui le produit du rendement quantique avec le coefficient d'absorption molaire de ladite protéine.

L'« intensité de fluorescence » d'une protéine fluorescente peut être obtenue à l'aide d'un spectrofluorimètre, tel que par exemple le Fluorolog®3 (HORIBA Jobin Yvon), et dans des conditions de concentrations faibles telles que $\varepsilon(\lambda_{ex})\cdot C\cdot 1<0{,}05$, a pour expression :

$$I_{em}(\lambda_{ex},\lambda_{em}) = k\cdot I_0(\lambda_{ex})\cdot f(\lambda_{em})\cdot \varepsilon(\lambda_{ex})\cdot C\cdot 1$$

où

- $\varepsilon(\lambda_{ex})$ et $I_0(\lambda_{ex})$ désignent respectivement le coefficient d'absorption molaire de la dite protéine et l'intensité du faisceau incident à la longueur d'onde d'excitation $\lambda_{ex}$ ;
- $f(\lambda_{em})$ représente l'intensité de fluorescence à la longueur d'onde d'émission $\lambda_{em}$. Intégré sur l'ensemble du spectre d'émission, ce paramètre est égal au rendement quantique ;
- C est la concentration de la dite protéine ;
- et 1 la longueur du trajet optique dans l'échantillon.

Une méthode permettant de mesurer ces paramètres est notamment décrite par Valeur, B. dans « Molecular Fluorescence : Principles and Applications », 3ème édition. (2006), Wiley-VCH.

En outre, la mesure de l'intensité de fluorescence peut permettre de rendre compte de variations :

- du coefficient d'absorption molaire, et donc du spectre d'absorption ;
- de la probabilité d'émission à la longueur d'onde d'émission, et donc par extension du spectre d'émission, lorsque la protéine est étudiée dans différentes conditions de pH.

Le terme « durée de vie de fluorescence » (Tau, $<\tau>$) signifie le temps moyen pendant lequel une protéine fluorescente demeure à l'état excité avant de retourner à son état basal. Cette durée se mesure préférentiellement en nanosecondes. Ici, la durée de vie de fluorescence est une durée de vie moyenne ($<\tau>$) et est obtenue par ajustement des déclins d'émission de fluorescence I(t) à l'aide d'une somme d'exponentielle selon l'expression :

$$< \tau >= \frac{\sum_i a_i \cdot \tau_i}{\sum_i a_i}$$

avec

$$I(t) = \sum_i a_i \cdot e^{-t/\tau_i}$$

Ici, les déclins d'émission de fluorescence sont obtenus à l'aide de la technique de comptage de photon unique décrite par O'Connor et al. (1984).
Parmi les méthodes permettant de mesurer la durée de vie de fluorescence, on peut citer la microscopie d'imagerie de la durée de vie de fluorescence (FLIM), et le comptage monophotonique corrélé au temps (TCSPC).

Les méthodes telles que définies ci-dessus peuvent être préférentiellement mises en oeuvre dans une gamme de température comprise entre 0°C et 100°C, préférablement sur une gamme comprise entre 1°C et 90°C, 2°C et 80°C, 3°C et 70°C, 4°C et 60°C, 5°C et 50°C, encore plus préférablement entre 6°C et 40°C, 7°C et 30°C, 8°C et 25°C, 9°C et 24°C, 10°C et 23°C, et de manière encore plus préférentielle entre 11°C et 22°C, 12°C et 21°C. De manière avantageuse, ladite gamme de température est de 20°C +/-0,1°C.

Par pH basique, on entend un pH compris entre 7 et 14, et par pH acide, on entend ici un pH compris entre 0 et 7. Ladite sensibilité au pH est donc étudiée sur une gamme de pH allant de 0 à 14, préférablement sur une gamme de pH 1 à pH 13, de pH 2 à pH 12, plus préférablement sur une gamme de pH 2,5 à pH 11, de pH 3 à 10, de pH 4 à pH 9, et de manière encore plus préférentielle sur une gamme de pH 5 à pH 8, et de pH 5,5 à pH 7,5. De manière avantageuse, la gamme de pH testée va de pH 5,5 à pH 7,4. Les pertes, diminutions, ou modifications mentionnées ci-dessus se mesurent toujours entre le pH le plus basique et le pH le plus acide.

Par « sensibilité réduite au pH », on entend que la perte d'intensité de fluorescence ($I_f$) d'une protéine susceptible d'être obtenue selon la méthode de l'invention est inférieure à 50%, de préférence inférieure à 33%, de manière encore plus préférée inférieure à 30%, 25%, 20%, 15%, 10%, 5% et de manière avantageuse égale à 0%, quand le pH du milieu dans lequel se trouve ladite protéine passe d'un pH basique à un pH acide. Plus particulièrement, ladite perte d'intensité de fluorescence est inférieure à 50%, de préférence inférieure à 33%, de manière encore plus préférée inférieure à 30%, 25%, 20%, 15%, 10%, 5% et de manière avantageuse égale à 0%, quand ledit pH passe d'un pH 7,4 à un pH 5,5.

Par « sensibilité réduite au pH », on peut également entendre que la perte de durée de vie de fluorescence (Tau ou $\tau$) d'une protéine susceptible d'être obtenue selon la présente méthode est inférieure à 33%, de préférence inférieure à 32%, de manière en encore plus préférée inférieure à 30%, 25%, 20%, 15%, 10%, 5% et de manière avantageuse égale à 0%, quand le pH du milieu dans lequel se trouve ladite protéine passe d'un pH basique à un pH acide. Plus particulièrement, ladite perte de durée de vie de fluorescence est inférieure à 33%, de préférence inférieure à 32%, de manière en encore plus préférée inférieure à 30%, 25%, 20%, 15%, 10%, 5% et de manière avantageuse égale à 0%, quand ledit pH passe d'un pH 7,4 à un pH 5,5.

La sensibilité au pH d'une protéine fluorescente cyan est réduite dès lors qu'au moins un des deux critères tels que définis ci-dessus est rempli.

De manière préférée, la sensibilité au pH d'une protéine fluorescente cyan est réduite si les deux critères tels que définis ci-dessus sont remplis.

En sus de ces critères, les protéines fluorescentes cyans ayant une « sensibilité réduite au pH » peuvent présenter une éventuelle diminution de la valeur de leur pH de demi-transition ($pH_{1/2}$) par rapport à la valeur de $pH_{1/2}$ de la ECFP. Ainsi la diminution de la valeur du $pH_{1/2}$ est d'au moins 0,1 ; 0,2 ; 0,3 ; 0.4 unité de pH, préférentiellement d'au moins 0,5 ; 1 ; 1,5, 2 unités de pH et encore plus préférentiellement d'au moins 2,2 unités de pH.

Par « $pH_{1/2}$ », il faut entendre ici la valeur de pH pour laquelle la somme des intensités de fluorescence à 474 nm de la protéine au pH le plus basique et au pH le plus acide tels que définis ci-dessus est réduite de moitié. L'intensité de fluorescence est mesurée par la méthode définie ci-dessus. De préférence, le $pH_{1/2}$ est la valeur de pH pour laquelle la somme des intensités de fluorescence à 474 nm de ladite protéine à pH 7,4 et à pH 2,5 est réduite de moitié. Une méthode équivalente permettant de déterminer le pH de demi-transition d'une protéine fluorescente consiste à mesurer à chaque pH testé l'intensité de fluorescence totale émise par ladite protéine en calculant l'aire sous le spectre d'émission de fluorescence obtenu en excitant à 420 nm. Cette intensité totale est alors corrigée de l'absorbance à 420 nm. Le pH de demi-transition peut également être estimé à partir de la variation en fonction du pH de l'intensité totale corrigée ainsi obtenue selon un mode de calcul similaire (valeur de pH telle que la somme de ces intensités totales au pH le plus basique et au pH le plus acide est réduite de moitié).

En outre, ces protéines peuvent également présenter une éventuelle augmentation de leur durée de vie de fluorescence (Tau ou $\tau$) à pH 7,4 par rapport à la durée de vie de la ECFP. Ainsi l'augmentation de la valeur $\tau$ est d'au moins 0,1 ns, préférentiellement d'au moins 0,5 ns, 1 ns et encore plus préférentiellement d'au moins 1,5 ns.

La propriété de « sensibilité réduite au pH » ne doit cependant pas être assimilée à celle de stabilité thermodynamique ou cinétique, plus communément appelée « stabilité ». La stabilité d'une protéine se traduit en effet par le maintien de la structure native de cette protéine dans une certaine gamme de conditions physico-chimiques externes (température, pression, etc), et est une notion complexe pouvant être difficile à apprécier car elle dépend non seulement des conditions externes utilisées pour perturber la structure de la protéine mais aussi des paramètres sélectionnés pour évaluer l'état dénaturé de cette protéine.

La convention utilisée pour décrire les protéines fluorescentes cyans suit la règle suivante : ECFP (numéro de l'acide aminé muté - nom de l'acide aminé introduit).

La numérotation des acides aminés utilisée se fait non pas de manière conventionnelle à partir d'un acide aminé N-terminal méthionine, mais à partir de l'acide aminé valine de la séquence SEQ ID N°2 de la ECFP. Ainsi, la séquence SEQ ID N°2 sert de référence pour affecter le numéro ou position de l'acide aminé muté, et le nom de l'acide aminé introduit peut être déterminé en effectuant un alignement optimal, tel que décrit ultérieurement, de la séquence SEQ ID N°2 avec celle de la protéine fluorescente cyan obtenue selon la présente méthode.

Les exemples de protéines fluorescentes cyans ayant une sensibilité réduite au pH comprennent, sans limitation, ou consistent en une séquence protéique sélectionnée dans le groupe consistant en les séquences SEQ ID N°6, SEQ ID N°8, SEQ ID N°58, SEQ ID N°10, SEQ ID N°12, SEQ ID N°14, SEQ ID N°60, SEQ ID N° 72, SEQ ID N° 74 et SEQ ID N° 76 (voir Tableau 1 ci-après).

Selon un mode préféré, les protéines fluorescentes cyans comprennent, sans limitation, ou consistent en une séquence protéique sélectionnée dans le groupe consistant en les séquences SEQ ID N°12, SEQ ID N°14, SEQ ID N°60, SEQ ID N° 74 et SEQ ID N° 76.

Selon un autre mode avantageux, la protéine fluorescente cyan comprend ou consiste en une séquence protéique de séquence SEQ ID N°12.

L'invention concerne également les acides nucléiques codant les protéines fluorescentes cyans telles que définies ci-dessus.

Par « acide nucléique », ou séquence nucléotidique, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN simple brin ou double brin choisi parmi un ADNc, un ADN génomique et un ADN plasmidique, qu'aux produits de transcription dudit ADN.

Les exemples d'acides nucléiques comprennent, sans limitation, ou consistent en une séquence nucléotidique sélectionnée dans le groupe consistant en les séquences SEQ ID N°5, SEQ ID N°7, SEQ ID N°57, SEQ ID N°9, SEQ ID N°11, SEQ ID N°13, SEQ ID N°59, SEQ ID N° 71, SEQ ID N° 73 et SEQ ID N° 75 (voir Tableau 1 ci-après).

Selon un mode préféré, les acides nucléiques comprennent, sans limitation, ou consistent en une séquence nucléotidique sélectionnée dans le groupe consistant en les séquences SEQ ID N°11, SEQ ID N°13 SEQ ID N°59, SEQ ID N° 73 et SEQ ID N° 75.

Selon un autre mode avantageux, l'acide nucléique comprend ou consiste en une séquence nucléotidique de séquence SEQ ID N°11.

Les acides nucléiques tels que définis ci-dessus peuvent ou non comprendre un codon d'initiation à l'extrémité 5', et/ou un codon de terminaison à l'extrémité 3' de leur séquence. Les codons d'initiation incluent, sans limitation, les codons (ou trinucléotides, ou triplets de nucléotides) ATG, AUG, TTG, UUG, GTG, GUG, CTG and CUG et peuvent être sélectionnés par l'homme du métier en fonction de la cellule hôte dans laquelle ledit acide nucléique sera traduit. Les codons de terminaison incluent notamment les codons TAG, UAG, TAA, UAA, TGA and UGA et peuvent être sélectionnés par l'homme du métier en fonction de la cellule hôte dans laquelle ledit acide nucléique sera traduit.

Avantageusement, ces acides nucléiques ne comprennent pas de codon d'initiation et/ou de codon de terminaison dans le cas où la protéine fluorescente cyan pour laquelle ils codent est fusionnée, directement ou indirectement, à une autre protéine.

Ces acides nucléiques peuvent également comprendre en leur extrémité 5' et/ou 3' une séquence nucléotidique codant pour un peptide signal et/ou une séquence nucléotidique codant pour une séquence d'acides aminés permettant la purification de la protéine pour laquelle ils codent. Ces dernières pourront être choisies par l'homme du métier parmi les séquences nucléotidiques n'affectant pas les propriétés fonctionnelles de protéines, telle qu'une séquence polyhistidine et /ou une séquence de site de clivage par une protéase (e.g. SEQ ID N°69 de séquence ATGTCGTACT ACCATCACCA TCACCATCAC GATTACGATA TCCCAACGAC CGAAAACCTG TATTTTCAGG GCGCC).

Selon un autre mode de réalisation de l'invention, la méthode comprend en plus de l'étape a), ou de l'étape a) et de l'étape b), une étape c) selon laquelle au moins une autre mutation sélectionnée parmi le groupe 9G, 11I, 19E, 26R, 68L, 72A, 87V, 145A, 164H, 167A, 172T, 175G, 1941, et 206K est introduite dans la séquence SEQ ID N°2, ladite étape c) pouvant être préalable ou postérieure à l'étape a) et/ou b).

Les « protéines fluorescentes cyans » selon l'invention peuvent donc également présenter une ou plusieurs mutations supplémentaires (ou additionnelles) à des positions autres que les positions 65 et 148, à condition que ces mutations permettent de conserver les spectres d'absorption et d'émission des protéines de l'invention tel que décrit précédemment. De telles mutations conservatrices sont connues de l'homme du métier et peuvent être sélectionnées, de manière non limitative, parmi les mutations 9G, 11I, 19E, 26R, 68L, 72A, 87V, 145A, 164H, 167A, 172T, 175G, 1941, et 206K, et de manière préférée parmi les mutations 26R, 72A, 145A, 164H et 206K. Ces mutations supplémentaires peuvent par ailleurs être introduites avant ou après les mutations en position 65 et/ou 148 caractéristiques de l'invention, selon une méthode identique. Ainsi, la méthode de l'invention comprenant ou consistant en l'étape a), b) et c) permet également de générer des protéines fluorescentes cyans telles que définies ci-dessus et présentant une sensibilité réduite au pH.

Les séquences d'acides aminés des protéines fluorescentes cyans ainsi mutées peuvent alors présenter au moins environ 60%, 65%, 70%, 75%, 80%, 85%, 90%, 91%, 92%, 93%, 94% 95%, 96%, 97%, 98%, 99% d'identité, avec la séquence SEQ ID N°2 de la ECFP, de manière contiguë ou non-contiguë. De préférence, les séquences d'acides aminés présentent au moins environ 85%, avantageusement environ au moins 90% d'identité, avec la séquence SEQ ID N°2, de manière contiguë ou non-contiguë. De manière encore plus préférée, les séquences d'acides aminés présentent au moins environ 95% d'identité, avantageusement au moins environ 97% et encore plus préférentiellement au moins environ 99% d'identité, avec la séquence SEQ ID N°2, de manière contiguë ou non-contiguë.

Le pourcentage d'identité décrit ci-dessus peut être déterminé en effectuant un alignement optimal des séquences d'acides aminés à comparer (ici avec la séquence SEQ ID N°2), ce pourcentage étant purement statistique et les différences entre les deux séquences étant réparties sur toute leur longueur. Cet alignement peut être réalisé en utilisant un algorithme connu de l'homme du métier, tels que l'alignement global de Needleman et Wunsch (J.Mol. Biol., 1970, 48:443) et ses implémentations informatiques, ou encore par simple inspection. Le meilleur alignement (c'est-à-dire, celui produisant le pourcentage le plus élevé d'identité) est alors sélectionné. Le pourcentage d'identité entre deux séquences d'acides aminés est déterminé en comparant ces deux séquences alignées de manière optimale dans laquelle la séquence d'acides aminés à comparer peut comprendre des additions ou des délétions par rapport à la séquence de référence (soit ici la séquence SEQ ID N°2) pour un alignement optimal entre ces deux séquences. Le pourcentage d'identité est calculé en déterminant le nombre de positions identiques pour lesquelles le résidu d'acide aminé est identique entre les deux séquences, en divisant ce nombre de positions identiques par le nombre total de positions et en multipliant le résultat obtenu par 100 pour obtenir le pourcentage d'identité entre ces deux séquences.

De manière préférée, 1 à 20 mutations supplémentaires peuvent être introduites dans la séquence SEQ ID N°2, préférablement 1 à 10 mutations, encore plus préférablement 1 à 5 mutations, et de manière avantageuse 1 à 2 mutations.

Selon un mode préféré de l'invention, les mutations additionnelles introduites dans la séquence SEQ ID N°2 sont uniquement sélectionnées parmi les mutations 9G, 11I, 19E, 26R, 68L, 72A, 87V, 145A, 164H, 167A, 172T, 175G, 1941, et 206K. De manière encore plus préférée, ces mutations additionnelles sont uniquement sélectionnées parmi les mutations 26R, 72A, 145A, 164H et 206K.

Les exemples de protéines pouvant être ainsi produites, comprennent, sans limitation, ou consistent en une séquence protéique sélectionnée dans le groupe consistant en les séquences SEQ ID N°16, SEQ ID N°18, SEQ ID N°20, SEQ ID N°62, SEQ ID N°22, SEQ ID N°24, SEQ ID N°26, SEQ ID N°64, SEQ ID N°28, SEQ ID N°30, SEQ ID N°32, SEQ ID N°66, SEQ ID N°34, SEQ ID N°80 et SEQ ID N°82 (voir Tableau 1 ci-après).

Selon un mode préféré, les protéines fluorescentes cyans comprennent, sans limitation, ou consistent en une séquence protéique sélectionnée dans le groupe consistant en les séquences SEQ ID N°18, SEQ ID N°20, SEQ ID N°62, SEQ ID N°22, SEQ ID N°24, SEQ ID N°26, SEQ ID N°64, SEQ ID N°28, SEQ ID N°30, SEQ ID N°32, SEQ ID N°66, SEQ ID N°34, SEQ ID N°80 et SEQ ID N°82.

Selon un mode encore plus préféré, les protéines fluorescentes cyans comprennent, sans limitation, ou consistent en une séquence protéique sélectionnée dans le groupe consistant en les séquences SEQ ID N°18, SEQ ID N°20, SEQ ID N°62, SEQ ID N°22, SEQ ID N°24, SEQ ID N°26, SEQ ID N°64, SEQ ID N°28, SEQ ID N°30, SEQ ID N°32, SEQ ID N°66, SEQ ID N°80 et SEQ ID N°82.

De manière encore plus préférée, les protéines fluorescentes cyans comprennent, sans limitation, ou consistent en une séquence protéique sélectionnée dans le groupe consistant en les séquences SEQ ID N°22, SEQ ID N°24, SEQ ID N°26, SEQ ID N°64, SEQ ID N°28, SEQ ID N°30, SEQ ID N°32, SEQ ID N°66, SEQ ID N°80 et SEQ ID N°82.

Selon un mode encore plus avantageux, les protéines fluorescentes cyans comprennent, sans limitation, ou consistent en une séquence protéique sélectionnée dans le groupe consistant en les séquences SEQ ID N°22, SEQ ID N°24, SEQ ID N°26, et SEQ ID N°64.

Selon un autre mode avantageux de l'invention, les protéines fluorescentes cyans comprennent, sans limitation, ou consistent en une séquence protéique sélectionnée dans le groupe consistant en les séquences SEQ ID N°28, SEQ ID N°30, SEQ ID N°32, et SEQ ID N°66.

Selon un autre mode avantageux, la protéine fluorescentes cyan comprend ou consiste en la séquence protéique SEQ ID N°80 ou SEQ ID N°82.

L'invention concerne également les acides nucléiques codant les protéines fluorescentes cyans telles que décrites ci-dessus.

Les acides nucléiques codant lesdites protéines, ou séquences nucléotidiques, peuvent alors présenter au moins environ 60%, 65%, 70%, 75%, 80%, 85%, 90%, 91%, 92%, 93%, 94% 95%, 96%, 97%, 98%, 99% d'identité, avec la séquence SEQ ID N°1 de la ECFP, de manière contiguë ou non-contiguë. De préférence, les séquences nucléotidiques présentent au moins environ 85%, avantageusement environ au moins 90% d'identité, avec la séquence SEQ ID N°1, de manière contiguë ou non-contiguë. De manière encore plus préférée, les séquences nucléotidiques présentent au moins environ 95% d'identité, avantageusement au moins environ 97% et encore plus préférentiellement au moins environ 99% d'identité, avec la séquence SEQ ID N°1, de manière contiguë ou non-contiguë.

Le pourcentage d'identité décrit ci-dessus peut être déterminé en effectuant un alignement optimal des séquences nucléotidiques à comparer (ici avec la séquence SEQ ID N°1), ce pourcentage étant purement statistique et les différences entre les deux séquences étant réparties sur toute leur longueur. Cet alignement peut être réalisé en utilisant un algorithme connu de l'homme du métier, tels que l'alignement global de Needleman et Wunsch (J.Mol. Biol., 1970, 48:443) et ses implémentations informatiques, ou encore par simple inspection. Le meilleur alignement (c'est-à-dire, celui produisant le pourcentage le plus élevé d'identité) est alors sélectionné. Le pourcentage d'identité entre deux séquences nucléotidiques est déterminé en comparant ces deux séquences alignées de manière optimale dans laquelle la séquence nucléotidique à comparer peut comprendre des additions ou des délétions par rapport à la séquence de référence (soit ici la séquence SEQ ID N°1) pour un alignement optimal entre ces deux séquences. Le pourcentage d'identité est calculé en déterminant le nombre de positions identiques pour lesquelles l'acide nucléique est identique entre les deux séquences, en divisant ce nombre de positions identiques par le nombre total de positions et en multipliant le résultat obtenu par 100 pour obtenir le pourcentage d'identité entre ces deux séquences.

Les exemples d'acides nucléiques codant les protéines ici décrites présentant des mutations additionnelles comprennent, sans limitation, ou consistent en une séquence nucléotidique sélectionnée dans le groupe consistant en les séquences SEQ ID N°15, SEQ ID N°17, SEQ ID N°19, SEQ ID N°61, SEQ ID N°21, SEQ ID N°23, SEQ ID N°25, SEQ ID N°63, SEQ ID N°27, SEQ ID N°29, SEQ ID N°31, SEQ ID N°65, SEQ ID N°33, SEQ ID N°79 et SEQ ID N°81 (voir Tableau 1 ci-après).

Selon un mode préféré, les acides nucléiques codant les protéines fluorescentes cyans comprennent, sans limitation, ou consistent en une séquence nucléotidique sélectionnée dans le groupe consistant en les séquences SEQ ID N°17, SEQ ID N°19, SEQ ID N°61, SEQ ID N°21, SEQ ID N°23, SEQ ID N°25, SEQ ID N°63, SEQ ID N°27, SEQ ID N°29, SEQ ID N°31, SEQ ID N°65, SEQ ID N°33, SEQ ID N°79 et SEQ ID N°81.

Selon un mode encore plus préféré, les acides nucléiques comprennent, sans limitation, ou consistent en une séquence nucléotidique sélectionnée dans le groupe consistant en les séquences SEQ ID N°17, SEQ ID N°19, SEQ ID N°61, SEQ ID N°21, SEQ ID N°23, SEQ ID N°25, SEQ ID N°63, SEQ ID N°27, SEQ ID N°29, SEQ ID N°31, SEQ ID N°65, SEQ ID N°79 et SEQ ID N°81.

De manière encore plus préférée, les acides nucléiques comprennent, sans limitation, ou consistent en une séquence nucléotidique sélectionnée dans le groupe consistant en les séquences SEQ ID N°21, SEQ ID N°23, SEQ ID N°25, SEQ ID N°63, SEQ ID N°27, SEQ ID N°29, SEQ ID N°31, SEQ ID N°65, SEQ ID N°79 et SEQ ID N°81.

Selon un mode encore plus avantageux, les acides nucléiques comprennent, sans limitation, ou consistent en une séquence nucléotidique sélectionnée dans le groupe consistant en les séquences SEQ ID N°21, SEQ ID N°23, SEQ ID N°25, et SEQ ID N°63.

Selon un autre mode avantageux de l'invention, les acides nucléiques comprennent, sans limitation, ou consistent en une séquence nucléotidique sélectionnée dans le groupe consistant en les séquences SEQ ID N°27, SEQ ID N°29, SEQ ID N°31, et SEQ ID N°65.

Selon un autre mode avantageux, l'acide nucléique comprend ou consiste en la séquence nucléotidique SEQ ID N°79 ou SEQ ID N°81.

**Tableau 1 :** liste des mutations pouvant être introduites dans la séquence SEQ ID N°2, avec les séquences d'acides aminés et séquences nucléotidiques correspondantes.

| Mutation(s) introduite(s) dans la séquence SEQ ID N°2 | Référence de la séquence protéique | Référence de la séquence d'acide nucléique codant pour ladite séquence protéique |
|---|---|---|
| 148G | SEQ ID N°6 | SEQ ID N°5 |
| 148S | SEQ ID N°8 | SEQ ID N°7 |
| 148A | SEQ ID N°58 | SEQ ID N°57 |

(suite)

| Mutation(s) introduite(s) dans la séquence SEQ ID N°2 | Référence de la séquence protéique | Référence de la séquence d'acide nucléique codant pour ladite séquence protéique |
|---|---|---|
| 148E | SEQ ID N°72 | SEQ ID N°71 |
| 65S | SEQ ID N°10 | SEQ ID N°9 |
| 65S, 148G | SEQ ID N°12 | SEQ ID N°11 |
| 65S, 148S | SEQ ID N°14 | SEQ ID N°13 |
| 65S, 148A | SEQ ID N°60 | SEQ ID N°59 |
| 65S, 148D | SEQ ID N°74 | SEQ ID N°73 |
| 65S, 148E | SEQ ID N°76 | SEQ ID N°75 |
| 72A, 145A, 148D | SEQ ID N°16 | SEQ ID N°15 |
| 72A, 145A, 148G | SEQ ID N°18 | SEQ ID N°17 |
| 72A, 145A, 148S | SEQ ID N°20 | SEQ ID N°19 |
| 72A, 145A, 148A | SEQ ID N°62 | SEQ ID N°61 |
| 65S, 72A, 145A, 148D | SEQ ID N°22 | SEQ ID N°21 |
| 65S, 72A, 145A, 148G | SEQ ID N°24 | SEQ ID N°23 |
| 65S, 72A, 145A, 148S | SEQ ID N°26 | SEQ ID N°25 |
| 65S, 72A, 145A, 148A | SEQ ID N°64 | SEQ ID N°63 |
| 65S, 72A, 148D, 206K | SEQ ID N°28 | SEQ ID N°27 |
| 65S, 72A, 148G, 206K | SEQ ID N°30 | SEQ ID N°29 |
| 65S, 72A, 148S, 206K | SEQ ID N°32 | SEQ ID N°31 |
| 65S, 72A, 148A, 206K | SEQ ID N°66 | SEQ ID N°65 |
| 65S, 72A, 206K | SEQ ID N°34 | SEQ ID N°33 |
| 26R, 65S, 148G, 164H, 206K | SEQ ID N°80 | SEQ ID N°79 |

Un autre aspect de l'invention concerne les vecteurs recombinants comprenant l'acide nucléique codant la protéine fluorescente cyan mutée selon l'invention, tel que défini ci-dessus. Ledit acide nucléique peut être ainsi inséré dans un vecteur capable de réplication dans le but d'amplifier cet acide nucléique, ou pour exprimer la protéine fluorescente cyan selon l'invention. Parmi les vecteurs sont compris les vecteurs de clonage qui permettent d'amplifier un acide nucléique, et les vecteurs d'expression qui permettent d'exprimer une protéine ; ces vecteurs sont bien connus de l'homme du métier. De tels vecteurs incluent, sans limitation, les vecteurs plasmidiques, cosmidiques, YACS, viraux (adénoviraux, rétroviraux, épisome EBV), et phagiques. L'homme pourra par ailleurs utiliser tout autre vecteur approprié permettant d'exprimer la protéine fluorescente cyan de l'invention.

Des méthodes d'insertion d'acide nucléique dans de tels vecteurs sont connues de l'homme du métier. De manière générale, un acide nucléique est inséré dans au moins un site de restriction d'endonucléase approprié en utilisant des techniques connues de l'art (voir, par exemple, les techniques décrites dans Sambrook et al. (2001) et Ausubel et al. (2011).

Des séquences nucléotidiques permettant la transcription de l'acide nucléique de l'invention, l'expression et/ou la purification de la protéine fluorescente cyan de l'invention sont par ailleurs contenues dans le vecteur recombinant de l'invention. Ces séquences incluent, de manière générale et sans limitation, une ou plusieurs séquence(s) peptide-signal, une origine de réplication, un ou plusieurs gène(s) marqueur(s) de sélection, un élément amplificateur, un promoteur, une séquence de terminaison de transcription, et éventuellement une séquence permettant la purification d'une protéine. L'insertion de telles séquences dans ledit vecteur peut se faire via des techniques de ligation standard connues de l'homme du métier.

Selon l'origine de réplication sélectionnée, ledit vecteur de clonage ou d'expression pourra se répliquer dans une ou plusieurs cellules hôtes. Ainsi, l'origine de réplication du plasmide pBR322 est adapté à la plupart des bactéries Gram-négatives, celle du plasmide 2$\mu$ est spécifique de la levure, et diverses origines de réplication virales (SV40, polyome, adénovirus, VSV ou BPV) sont utiles pour les vecteurs de clonage dans les cellules de mammifères.

Selon le promoteur sélectionné, l'acide nucléique pourra être transcrit et la protéine correspondante exprimée dans une ou plusieurs cellules hôtes. Ainsi, les promoteurs T7, Lac, trp, tac, $\lambda$PL sont spécifiques des bactéries de genre *E. coli* ; les promoteurs PHO5, GAP, TPI1, ADH sont adaptés à la levure ; les promoteurs de la polyhédrine et P10 et leurs équivalents sont utilisés chez les cellules d'insecte ; et enfin les promoteurs CMV, MT1, de SV40, SR$\alpha$, rétroviraux, et les promoteurs de gènes d'une protéine de choc thermique sont adaptés aux cellules de mammifères.

Parmi les gènes marqueurs de sélection typiquement contenus dans les vecteurs de clonage ou d'expression, on peut citer, sans limitation : (a) les gènes conférant une résistance à un antibiotique ou à une toxine (par exemple, la néomycine, la zéocine, l'hygromycine, l'ampicilline, la kanamycine, la tétracycline, le chloramphénicol), et (b) les gènes permettant la compensation d'une déficience auxotrophique (par exemple le gène codant pour la dihydrolofate réductase DHFR permettant la résistance au méthotrexate, ou encore le gène TPI de *S.pombe*).

Parmi les séquences nucléotidiques permettant la purification d'une protéine, sont inclues, sans limitation, la séquence Histidine (Histidine Tag ou Hisx6), la séquence FLAG, et la séquence GST. Une séquence de clivage d'une protéase, telle que la TEV, peut par ailleurs être présente afin de pouvoir supprimer ultérieurement la séquence de purification.

Selon un mode préféré, une séquence nucléotidique de 6 Histidines et une séquence de clivage de la protéase TEV sont présentes dans le vecteur recombinant, et plus particulièrement une séquence nucléotidique codant pour la séquence d'acides aminés MSYYHHHHHHDYDIPTTENLYFQGA (SEQ ID N°70).

Selon un mode particulièrement préféré, le vecteur d'expression est le vecteur pPROEX™ HTa (GibcoBRL).

Des méthodes permettant de connecter l'acide nucléique selon l'invention et les séquences additionnelles mentionnées ci-dessus sont connues de l'homme du métier.

La présente invention porte également sur une cellule hôte transformée par l'acide nucléique codant ladite protéine, ou par le vecteur recombinant comprenant ledit acide nucléique.

Tel qu'il est utilisé ici, le terme «cellule hôte», « cellule », et «lignée cellulaire », peut être utilisé de façon interchangeable. Tous ces termes comprennent aussi leur descendance, qui comprend toutes les générations suivantes. Il est entendu que tous les descendants ne sont pas nécessairement identiques en raison de mutations délibérées ou accidentelles.

Par «cellule hôte», on fait référence ici à une cellule procaryote ou eucaryote, capable de répliquer l'acide nucléique codant pour la protéine fluorescente mutée de l'invention ou le vecteur recombinant tels que décrits précédemment, et ainsi d'exprimer la protéine fluorescente mutée de l'invention. Une cellule hôte peut être « transfectée» ou «transformée», selon un processus connu de l'homme du métier par lequel ledit acide nucléique ou ledit vecteur est transféré ou introduit dans la cellule hôte. Des exemples de telles méthodes incluent, sans limitation, l'électroporation, la lipofection, la transfection par phosphate de calcium, la transfection via DEAE-Dextran, la microinjection, et la biolistique.

Parmi les cellules hôtes sont inclues, sans limitation, des bactéries, des levures, des champignons, ou toute autre cellule eucaryote supérieure. L'homme du métier pourra ainsi choisir la cellule hôte appropriée parmi les nombreuses lignées cellulaires disponibles, notamment via l'American Type Culture Collection (ATCC) (www.ATCC.org). Des exemples de cellule hôte incluent, sans limitation, des micro-organismes tels que les bactéries Gram négatives du genre *Escherichia* (par exemple, *E. coli* RR1, LE392, B, X 1776, W3110, DH5 alpha, JM109, KC8), *Serratia, Pseudomonas, Erwinia, Methylobacterium, Rhodobacter, Salmonella* ou *Zymomonas,* les bactéries Gram positives du genre *Corynebacterium, Brevibacterium, Bacillus, Arthrobacter,* ou *Streptomyces,* les levures du genre *Saccharomyces,* les cellules de champignon du genre *Aspergillus, Neurospora, Fusarium* et *Trichoderma,* les cellules animales dont les cellules HEK293, NIH3T3, Jurkat, MEF, Vero, HeLa, CHO, W138, BHK, COS-7, MDCK, C127, Saos, PC12, HKG, et les cellules d'insecte Sf9, Sf21, Hi Five™ ou de *Bombyx mori.* L'utilisation de cellules d'insecte est notamment décrite dans le manuel « Baculovirus Expression vectors, A Laboratory Manual », de David R. O'Reilly et al., Oxford University Press, USA, (1992).

Dans le cas où la cellule hôte est transformée par le vecteur recombinant de l'invention tel que défini ci-dessus, le choix de ladite cellule hôte peut être dicté par le choix dudit vecteur, et en fonction de l'utilisation choisie, à savoir le clonage de l'acide nucléique ou l'expression de la protéine fluorescente cyan mutée selon l'invention.

Un autre aspect ici décrit concerne une méthode pour exprimer et purifier la protéine fluorescente cyan mutée de l'invention. Selon cette méthode, la cellule hôte telle que décrite ci-dessus est cultivée dans un milieu de culture approprié dans des conditions permettant l'expression de la protéine de l'invention. L'homme du métier pourra utiliser toute méthode conventionnelle lui permettant d'isoler et de purifier ladite protéine. Par exemple, dans le cas où ladite protéine a été exprimée sous une forme solubilisée dans les cellules hôtes, ces dernières sont récupérées par centrifugation et suspendues dans un tampon, puis un extrait cellulaire est obtenu en détruisant les cellules au moyen par exemple d'un homogénéisateur à ultrasons. A partir du surnageant obtenu par centrifugation de cet extrait, un échantillon purifié peut être obtenu en utilisant une méthode classique ou une combinaison de méthodes classiques pour isoler et purifier la protéine de l'invention. Ces méthodes incluent, sans limitation, l'extraction par solvant, le relargage au sulfate d'ammonium, le dessalage, la précipitation par solvant organique, la filtration sur gel, l'électrophorèse préparative, la focalisation isoélectrique, l'ultrafiltration, de nombreuses méthodes de chromatographie telles que la chromatographie échangeuse d'ions (soit anionique, par exemple à l'aide d'une résine telle que le diéthylaminoéthyle (DEAE) Sépharose, soit cationique en utilisant par exemple une résine telle que le S-Sépharose (Pharmacia)), la chromatographie hydrophobe (en utilisant par exemple une résine telle que le Butyl Sépharose ou le Phényl Sépharose), la chromatographie d'affinité au moyen d'anticorps, la chromatographie d'adsorption, la chromatofocalisation, la chromatographie en phase liquide à haute performance (HPLC), et la HPLC en phase inverse.

Par ailleurs, si une séquence nucléotidique permettant la purification de la protéine, ladite séquence étant fusionnée à une séquence de clivage par une protéase, est présente dans le vecteur recombinant, la protéine produite peut être récupérée grâce à un traitement par une protéase spécifique de ladite séquence de clivage (thrombine, trypsine, protéase TEV, etc).

Un autre aspect de l'invention concerne un biosenseur comprenant la protéine fluorescente cyan selon l'invention.

Par « biosenseur », il faut entendre ici une molécule comprenant un capteur biosensible lié, par une liaison covalente ou non-covalente, à une autre molécule, et permettant de convertir une réponse biologique en un signal électrique, chimique, physique, photophysique ou photochimique. Un « capteur biosensible », tel qu'on l'entend ici, est une molécule naturelle ou synthétique, permettant de détecter la présence, et/ou de mesurer la concentration ou l'activité d'un analyte tel qu'un ion, un sucre, une enzyme, un acide nucléique, un anticorps, un cofacteur ou une protéine naturelle ou modifiée (par exemple par glycosylation ou phosphorylation), lorsque le biosenseur est exprimé dans une cellule hôte telle que définie ci-dessus. Ainsi, l'interaction entre ledit analyte présent dans ladite cellule et ledit capteur entraîne un réarrangement structural du biosenseur, qui se traduit par un signal tel que décrit ci-dessus. Ledit capteur peut être, par exemple, un sucre, un lipide, une protéine, un acide nucléique. De manière préférée, ledit capteur est une molécule de nature peptidique.

Selon un mode préféré, les capteurs biosensibles incluent, sans limitation, des peptides telles que la mellitine, des polypeptides hydrides (c'est-à-dire des polypeptides qui résultent de la fusion d'au moins deux protéines), des anticorps, des enzymes ou encore des substrats enzymatiques. Parmi lesdits polypeptides hydrides, on peut citer des enzymes fusionnées à des domaines de liaison (par exemple, la calmoduline fusionnée au peptide M13, ou encore la GTPase fusionnée à un domaine pouvant reconnaitre sa conformation active). On peut également citer comme exemple de capteur biosensible une séquence peptidique substrat d'une protéine kinase, une séquence peptidique substrat d'une protéase, un domaine de liaison à l'AMPc, un domaine de liaison aux acides aminés phosphorylés (FHA), un domaine de liaison au glutamate (YbeJ), un domaine de liaison à un sucrose (par exemple le MBP, qui lie le maltose), et un domaine de liaison au $Ca^{2+}$ telle que la Troponine C et ses fragments.

Un biosenseur tel que décrit ici peut ainsi permettre d'étudier le métabolisme cellulaire ou les événements de signalisation cellulaire.

Dans le cadre de la présente description, le biosenseur comprend une protéine fluorescente cyan présentant une sensibilité réduite au pH liée, par une liaison covalente ou non-covalente, audit capteur biosensible. Ainsi, lorsque ce biosenseur est exprimé dans une cellule hôte, l'interaction entre l'analyte présent dans ladite cellule et ledit capteur entraîne un réarrangement structural du biosenseur, qui se traduit par une modification de l'émission de fluorescence de la part de la protéine fluorescente à laquelle il est fusionné. Par « modification de l'émission de fluorescence », on entend ici une variation de l'intensité de fluorescence émise par ladite protéine, caractérisée en ce que ladite variation est proportionnelle à la concentration ou à l'activité dudit analyte, ou traduit la présence de cet analyte. L'intensité de fluorescence peut être mesurée telle que décrite précédemment.

Selon un mode préféré de l'invention, le biosenseur de l'invention comprend en outre une protéine fluorescente dont le spectre d'absorption se recouvre partiellement avec le spectre d'émission de la protéine cyan dudit biosenseur.

Selon un mode particulier, la protéine fluorescente dont le spectre d'absorption se recouvre partiellement avec le spectre d'émission de la protéine cyan est liée directement audit biosenseur. Par « liée directement », on entend ici que ladite protéine est liée, de manière covalente ou non-covalente, au capteur biosensible dudit biosenseur.

Selon un autre mode particulier, la protéine fluorescente dont le spectre d'absorption se recouvre partiellement avec le spectre d'émission de la protéine cyan est liée indirectement au biosenseur. Par « liée indirectement », on entend ici que ladite protéine est liée au capteur biosensible dudit biosenseur via l'intermédiaire d'une molécule, par exemple via des liaisons non-covalentes entre ladite molécule et ledit capteur.

De manière préférée, la protéine fluorescente dont le spectre d'absorption se recouvre partiellement avec le spectre d'émission de la protéine cyan de l'invention présente un maximum d'absorbance compris entre 495 et 600 nm, de manière avantageuse entre 500 et 590 nm, et encore plus avantageusement entre 510 et 580 nm, et de manière encore plus avantageuse entre 520 et 570 nm, et entre 550 et 570 nm. Des exemples de protéines fluorescentes pouvant être ainsi fusionnées à la protéine de l'invention via un capteur biosensible incluent, sans limitation, les protéines fluorescentes jaunes telles que la YFP, la Topaz, la EYFP, la YPET, la SYFP2, la Citrine, la Venus, la cp-Venus, les protéines fluorescentes oranges telles que la Kusabira Orange, la Kusabira Orange2, la mOrange, la mOrange2, la dTomato, la dTomato-Tandem, la DsRed et ses variants (DsRed2, DsRed-Express (T1), DsRed-Express2, DsRed-Max, DsRed-Monomer), la TagRFP et la TagRFP-T, les protéines fluorescentes rouges telles que la mRuby, la mApple, la mStrawberry, l'AsRed2, la mRFP1, la JRed, la mCherry, l'eqFP611, la tdRFP611, la HcRed1, la mRaspberry, ainsi que les protéines fluorescentes émettant dans le rouge lointain telles que la tdRFP639, la mKate, la mKate2, Katushka, tdKatushka, la HcRed-Tandem, la mPlum et la AQ143 (Day et al., 2009). De manière préférée, la protéine fluorescente dont le spectre d'absorption se recouvre partiellement avec le spectre d'émission de la protéine cyan de l'invention est sélectionnée parmi les protéines fluorescentes jaunes ou oranges telles que décrites ci-dessus.

De manière particulièrement avantageuse, la protéine fluorescente dont le spectre d'absorption se recouvre partiellement avec le spectre d'émission de la protéine cyan décrite ici présente elle-même une sensibilité réduite au pH. Cette protéine peut alors être sélectionnée parmi les protéines Citrine, Venus, cp-Venus, TagRFP et TagRFP-T, de manière préférée parmi la TagRFP et TagRFP-T, et de manière encore plus préférée cette protéine est la TagRFP (Merzlyak et al., 2007).

Selon un mode particulièrement avantageux de l'invention, le biosenseur de l'invention comprend, outre une protéine fluorescente cyan de l'invention dont la séquence comprend au moins les mutations 65S et 148G, la protéine fluorescente TagRFP. Selon un mode préféré, ladite protéine fluorescente cyan comprend ou consiste en une séquence protéique sélectionnée dans le groupe consistant en les séquences SEQ ID N°12, SEQ ID N°80 ou SEQ ID N°82, et de manière encore plus préférée, en la séquence protéique SEQ ID N°82.

La fusion du capteur biosensible à une protéine fluorescente pour construire le biosenseur de l'invention se fait par des techniques d'ingénierie génétique, enzymatiques ou de couplage chimique connues de l'homme du métier. De telles techniques sont décrites dans Sambrook et al. (2001) et Ausubel et al. (2011).

Selon un mode particulièrement avantageux, le biosenseur peut être généré en mutant des biosenseurs existants selon la méthode de l'invention, c'est-à-dire en y introduisant au moins une mutation unique permettant de réduire la sensibilité au pH. Parmi les biosenseurs existants, qui sont par ailleurs connus de l'homme du métier, on peut citer, sans limitation, les biosenseurs Epac, Epac2, ECFP-YbeJ-Venus, ECFP-MBP-EYFP, CFP-TnC-Citrine, Aktus, Atomic, Erkus, Phocus, Picchu, AKAR, AKAR1 (Zhang et al., 2001), AKAR2 (Zhang et al., 2005), AKAR2.2 (Dunn et al., 2006), AKAR3, BKAR, CKAR et DKAR. De manière préférée, le biosenseur muté est un biosenseur de type AKAR, et de manière plus préférée le biosenseur est AKAR2.2.

Selon un mode avantageux, les mutations 65S et 148G sont introduites dans ledit biosenseur selon la méthode de l'invention. Ainsi, lorsque les mutations 65S et 148G sont introduites dans le biosenseur AKAR2.2, ce dernier comprend une protéine fluorescente cyan comprenant la séquence SEQ ID N°82. De manière encore plus préférée, les mutations 65S et 148G sont introduites dans le biosenseur AKAR2.2 selon la méthode de l'invention et la Citrine exprimée par celui-ci est remplacée par la protéine TagRFP à l'aide de techniques classiques d'ingénierie génétique, enzymatiques ou de couplage chimique connues de l'homme du métier, ledit biosenseur comprenant ainsi une protéine fluorescente cyan comprenant la séquence SEQ ID N°82 et la protéine fluorescente orange TagRFP.

Pour de plus amples informations concernant les biosenseurs, l'homme du métier pourra se référer aux publications de Morris M.C. (2010) et Frommer et al. (2009).

Les biosenseurs décrits ici peuvent être utilisés selon les techniques décrites ci-dessous, qui sont plus préférentiellement selon l'invention, le FRET et/ou le FLIM.

Un autre aspect de la présente description concerne l'utilisation des produits décrits ici (notamment les protéines fluorescentes mutées, acides nucléiques codant pour ces protéines, vecteurs recombinants, cellules hôtes et biosenseurs tels que définis ci-dessus).

Celles-ci incluent, sans limitation, la cytométrie de flux (FACS), les méthodes d'imagerie conventionnelles telles que la microscopie photonique ou confocale, les méthodes d'imagerie quantitative en temps réel telles que la spectroscopie de corrélation de fluorescence (FCS) et les variations de cette méthode (par exemple le FCCS), le transfert d'énergie par résonance de type Förster (FRET), le transfert d'énergie bioluminescente par résonance (BRET), la microscopie d'imagerie de la durée de vie de fluorescence (FLIM), la redistribution de fluorescence après photoblanchiment (FRAP), la perte de fluorescence induite par photoblanchiment (FLIP), le comptage monophotonique corrélé au temps (TCSPC), l'anisotropie et la dépolarisation de fluorescence, la microscopie de localisation par photoactivation (PALM), la microscopie de reconstruction optique stochastique (STORM) et la microscopie de déplétion par émission stimulée (STED). Ceci inclut également les méthodes de détection haut débit telles que le criblage haut contenu, la microscopie haut débit et les méthodes microfluidiques conventionnelles ou à ultra haut débit.

Les méthodes d'imagerie utilisées le plus préférentiellement sont le FRET et le FLIM.

Le terme «transfert d'énergie par résonance de type Förster» (FRET) se réfère ici à un transfert non radiatif de l'énergie d'excitation provenant d'une molécule fluorescente « donneur » de photons à une molécule fluorescente « accepteur », ce transfert n'étant possible que lorsque le « donneur » de photons est suffisamment proche de la molécule « accepteur », soit à une distance de 10 à 100 Å selon la géométrie des molécules et du système d'observation (Heyduk (2002), Truong et al. (2001), Issad et al. (2003), Boute et al. (2002)). Cette méthode permet de quantifier soit la diminution de la fluorescence du « donneur » de photons (en mesurant par exemple l'intensité ou la durée de vie de fluorescence de ce « donneur »), soit l'augmentation de la fluorescence de l' « accepteur » (en mesurant par exemple l'intensité de fluorescence de cet « accepteur »). Toute méthode dérivée du FRET s'applique également à l'invention présente. Dans le cas du FRET, la protéine fluorescente cyan mutée de l'invention est préférentiellement utilisée en tant que « donneur » de photons.

Le « transfert d'énergie bioluminescente par résonance », ou BRET, se diffère du FRET en ce que l'énergie du « donneur » provient d'une molécule bioluminescente, telle que la luciférine (ex: coelenterazine), qui excitée en présence d'une enzyme (par exemple, la luciférase), va émettre des photons. Ces photons seront alors transférés sur une molécule fluorescente « accepteur » de type GFP qui va émettre une fluorescence si les conditions de proximité et de géométrie pour un transfert d'énergie sont remplies. La protéine fluorescente mutée de l'invention devra donc être utilisée comme molécule fluorescente « accepteur » dans ce type particulier d'application.

Le FLIM (ou microscopie d'imagerie de la durée de vie de fluorescence) est une technique permettant de mesurer le déclin de fluorescence d'une molécule fluorescente et de quantifier la durée de vie de fluorescence de cette molécule. Cette technique peut être utilisée seule ou en combinaison avec le FRET, en particulier pour localiser les interactions protéine-protéine ou pour étudier la signalisation cellulaire. Toute méthode dérivée du FLIM telle que le FLIM tomographique, le FLIM multiplexe, le FLIM automatisé en plaque multi-puits, ou l'endomicroscopie confocale FLIM est comprise dans la présente description. Dans le cas où la technique FLIM est utilisée dans la présente invention, la protéine fluorescente cyan mutée est préférentiellement un « donneur » de photons.

Pour tout autre détail concernant les techniques mentionnées ci-dessus, l'homme du métier pourra se référer aux articles de Day et al. (2009), Trugnan et al. (2004), et Kumar et al. (2011).

Un mode particulier de réalisation concerne l'utilisation des produits de l'invention dans des méthodes de criblage de composés chimiques et/ou de cellules. De manière préférée, lesdites méthodes de criblage sont des méthodes de détection haut débit telles que le criblage haut contenu, la cytométrie de flux, la microscopie haut débit, les méthodes microfluidiques (conventionnelles ou à ultra haut débit), et les dosages en lecteur de plaque.

Un autre mode particulier de réalisation concerne l'utilisation desdits produits dans des tests de toxicologie, de génotoxicité ou de détection de pollution environnementale réalisés en solution, plus particulièrement à partir d'un prélèvement, d'un extrait biologique, d'une cellule, d'un tissu ou d'un organisme vivant.

La présente invention sera mieux comprise à la lumière des exemples ci-après.

**FIGURES:**

**Figure 1 : Dépendance en pH des propriétés de fluorescence de la ECFPr purifiée (SEQ ID N°4).** Graphe A : Spectres d'absorption normalisés à la même aire. Graphe B : Spectres d'émission de fluorescence normalisés à la même aire ($\lambda$ex=420 nm). Graphe C : Intensité de fluorescence émise à 474 nm ($\Delta\lambda$= 6 nm) normalisée à la valeur maximum, en fonction du pH.. Graphe D: Durée de vie de fluorescence $<\tau>$ (ns), en fonction du pH.

**Figure 2 : Dépendance en pH des propriétés de fluorescence de la ECFPr (148G) purifiée.** Graphe A : Spectres d'absorption normalisés à la même aire. Graphe B : Spectres d'émission de fluorescence normalisés à la même aire ($\lambda$ex=420 nm). Graphe C : Intensité de fluorescence émise à 474 nm ($\Delta\lambda$= 6 nm) normalisée à la valeur maximum, en fonction du pH- comparaison avec la ECFPr. Graphe D: Durée de vie de fluorescence $<\tau>$ (ns), en fonction du pH - comparaison avec la ECFPr.

**Figure 3 : Dépendance en pH des propriétés de fluorescence de la ECFPr (148S) purifiée**. Graphe A : Spectres d'absorption normalisés à la même aire. Graphe B: Spectres d'émission de fluorescence normalisés à la même aire ($\lambda$ex=420nm). Graphe C: Intensité de fluorescence émise à 474 nm ($\Delta\lambda$=6nm) normalisée à la valeur maximum-comparaison avec la ECFPr. Graphe D : Durée de vie de fluorescence-Ccomparaison avec la ECFPr.

**Figure 4 : Dépendance en pH des propriétés de fluorescence de la ECFPr (148A) purifiée.** Graphe A : Spectres d'absorption normalisés à la même aire. Graphe B: Spectres d'émission de fluorescence normalisés à la même aire ($\lambda$ex=420nm). Graphe C: Intensité de fluorescence émise à 474 nm ($\Delta\lambda$=6nm) normalisée à la valeur maximum-comparaison avec la ECFP. Graphe D : Durée de vie de fluorescence-comparaison avec la ECFPr.

**Figure 5 : Dépendance en pH des propriétés de fluorescence de la ECFPr (65S) purifiée.** Graphe A : Spectres d'absorption normalisés à la même aire. Graphe B: Spectres d'émission de fluorescence normalisés à la même aire ($\lambda$ex=420nm). Graphe C: Intensité de fluorescence émise à 474 nm ($\Delta\lambda$=6nm) normalisée à la valeur maximum comparaison avec la ECFPr. Graphe D : Durée de vie de fluorescence- comparaison avec la ECFPr.

**Figure 6 : Dépendance en pH des propriétés de fluorescence de la ECFPr (65S, 148G) purifiée.** Graphe A : Spectres d'absorption normalisés à la même aire. Graphe B: Spectres d'émission de fluorescence normalisés à la même aire ($\lambda$ex=420nm). Graphe C : Intensité de fluorescence émise à 474 nm ($\Delta\lambda$=6nm) normalisée à la valeur maximum-comparaison avec la ECFPr. Graphe D : Durée de vie de fluorescence-comparaison avec la ECFPr.

**Figure 7 : Dépendance en pH des propriétés de fluorescence de la ECFPr (72A, 145A, 148D) purifiée.** Graphe A : Spectres d'absorption normalisés à la même aire. Graphe B : Spectres d'émission de fluorescence normalisés à la même aire (λex=420 nm). Graphe C : Intensité de fluorescence émise à 474 nm (Δλ= 6 nm) normalisée à la valeur maximum, en fonction du pH - comparaison avec la ECFPr. Graphe D: Durée de vie de fluorescence $<\tau>$ (ns), en fonction du pH - comparaison avec la ECFPr.

**Figure 8 : Dépendance en pH des propriétés de fluorescence de la ECFPr (72A, 145A, 148G) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum- comparaison avec la ECFPr. Graphe D : Durée de vie de fluorescence- comparaison avec la ECFPr.

**Figure 9 : Dépendance en pH des propriétés de fluorescence de la ECFPr (65S, 72A, 145A, 148D) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum- comparaison avec la ECFPr. Graphe D : Durée de vie de fluorescence- comparaison avec la ECFPr.

**Figure 10 : Dépendance en pH des propriétés de fluorescence de la ECFPr (65S, 72A, 148D, 206K) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ =6nm) normalisée à la valeur maximum- comparaison avec la ECFPr. Graphe D : Durée de vie de fluorescence- comparaison avec la ECFPr.

**Figure 11 : Dépendance en pH des propriétés de fluorescence de la ECFPr (65S, 72A, 148G, 206K) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum-comparaison avec la ECFPr. Graphe D : Durée de vie de fluorescence- comparaison avec la ECFPr.

**Figure 12 : Dépendance en pH des propriétés de fluorescence de la ECFPr (65S, 72A, 206K) purifiée.** Graphe A : Spectres d'absorption normalisés à la même aire. Graphe B : Spectres d'émission de fluorescence normalisés à la même aire (λex=420 nm). Graphe C : Intensité de fluorescence émise à 474 nm (Δλ= 6 nm) normalisée à la valeur maximum, en fonction du pH - comparaison avec la ECFPr ; Graphe D: Durée de vie de fluorescence $<\tau>$ (ns), en fonction du pH - comparaison avec la ECFPr.

**Figure 13 : Dépendance en pH des propriétés de fluorescence de la ECFPr (148D) purifiée.** Graphe A : Spectres d'absorption normalisés à la même aire. Graphe B: Spectres d'émission de fluorescence normalisés à la même aire (λex=420nm). Graphe C: Intensité de fluorescence émise à 474 nm (Δλ=6nm) normalisée à la valeur maximum-comparaison avec la ECFPr. Graphe D : Durée de vie de fluorescence-comparaison avec la ECFPr.

**Figure 14 : Dépendance en pH des propriétés de fluorescence de la ECFPr (148N) purifiée.** Graphe A : Spectres d'absorption normalisés à la même aire. Graphe B: Spectres d'émission de fluorescence normalisés à la même aire (λex=420nm). Graphe C: Intensité de fluorescence émise à 474 nm (Δλ=6nm) normalisée à la valeur maximum-comparaison avec la ECFPr.

**Figure 15 : Dépendance en pH des propriétés de fluorescence de la ECFPr (148E) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum-comparaison avec la ECFPr.

**Figure 16 : Dépendance en pH des propriétés de fluorescence de la ECFPr (148R) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum-comparaison avec la ECFPr.

**Figure 17: Dépendance en pH des propriétés de fluorescence de la ECFPr (65S, 148D) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum- comparaison avec la ECFPr. Graphe D : Durée de vie de fluorescence-comparaison avec la ECFPr.

**Figure 18: Dépendance en pH des propriétés de fluorescence de la ECFPr (65S, 148S) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum- comparaison avec la ECFPr. Graphe D : Durée de vie de fluorescence-comparaison avec la ECFPr.

**Figure 19 : Dépendance en pH des propriétés de fluorescence de la ECFPr (65S, 148E) purifiée.** Graphe A : Spectres d'absorption normalisés à la même aire. Graphe B: Spectres d'émission de fluorescence normalisés à la même aire (λex=420nm). Graphe C : Intensité de fluorescence émise à 474 nm (Δλ=6nm) normalisée à la valeur maximum -comparaison avec la ECFPr. Graphe D : Durée de vie de fluorescence-comparaison avec la ECFPr.

**Figure 20 : Durée de vie moyenne de fluorescence de la protéine ECFPr comprenant au moins les mutations**

**65S et 148G exprimée seule ou incorporée dans un biosenseur - en fonction du pH intracellulaire.** Des cellules BHK (baby hamster kidney) ont été transfectées soit avec un plasmide codant uniquement pour la ECFPr (65S, 148G), soit avec le biosenseur pHAKAR.

**Figure 21 : Propriétés spectrales des protéines fluorescentes cyans ECFPr, ECFPr (65S), ECFPr (72A, 145A, 148D) et ECFPr (65S, 72A, 145A, 148D).** Les spectres d'absorption (lignes hachurées) et spectres d'émission (lignes solides) ont été normalisées à la même aire (maximum de la bande du chromophore). Les spectres d'émission ont été enregistrés à une longueur d'onde d'excitation λex, de 420 nm.

**Figure 22 : Propriétés photophysiques de protéines fluorescentes cyans, comprenant ou non la mutation 65S.** ε : coefficient d'extinction molaire ; Rend.Q.: rendement quantique d'émission de fluorescence ; $\tau_L$ : valeur de la durée de vie la plus longue dans la distribution de vie de fluorescence ; $c_L$ : amplitude relative de la composante de temps de durée de vie de fluorescence la plus longue dans la distribution de vie de fluorescence ; % Rev :pourcentage de perte de l'intensité de fluorescence initiale après une illumination instantanée de densité de puissance 0,2W/cm2 ; $\tau_{Rev}$ (s) : constante de temps du déclin initial de l'intensité de fluorescence ; $\tau_{Irrev}$ (s) : constante de temps exponentielle de la perte d'intensité de fluorescence irréversible sous illumination prolongée de densité de puissance 0.2W/$cm^2$.

**Figure 23 : Distribution de la durée de vie de fluorescence de protéines fluorescentes cyans, comprenant ou non la mutation 65S.** Les distributions ont été obtenues via l'analyse, par la méthode de maximum d'entropie, des déclins de fluorescence des protéines ECFPr, ECFPr (65S), ECFPr (72A, 145A, 148D) et ECFPr (65S, 72A, 145A, 148D). Pour chaque protéine, la distribution a été établie à partir de 6 expériences indépendantes réalisées à pH 7,4 et à 20°C.

**Figure 24 : Dépendance avec le pH des propriétés spectrales de la ECFPr.** (A) Spectre d'absorption normalisé à l'unité au maximum d'absorbance, et (B) Spectre d'émission normalisé à la même aire.

**Figure 25 : Spectres d'émission de fluorescence de protéines fluorescentes cyans, comprenant ou non la mutation 65S, à pH acide, neutre et basique.** (A): ECFPr; (B): ECFPr (72A, 145A, 148D); (C): ECFPr (65S); (D) ECFPr (65S, 72A, 145A, 148D).

**Figure 26 : Spectres d'absorption de protéines fluorescentes cyans, incluant ou non la mutation 65S , à pH acide, neutre et basique.** (A): ECFPr; (B): ECFPr (72A, 145A, 148D); (C): ECFPr (65S); (D) ECFPr (65S, 72A, 145A, 148D).

**Figure 27 : Photoblanchiment réversible de protéines fluorescentes cyans, comprenant ou non la mutation 65S.** (A) Cinétique de blanchiment réversible effectué sur des billes d'agarose marquées avec des protéines fluorescentes purifiées. Après avoir été équilibrées au préalable dans l'obscurité, une illumination instantanée et constante avec une densité de puissance de 0.2W/$cm^2$ a été appliquée pendant moins de 15 secondes, tandis que des images ont été prises toutes les 200 ms. L'illumination a alors été arrêtée, et, après un minimum de 3 min dans l'obscurité, une série d'images de fluorescence ont été recueillies pour vérifier la réversibilité. Les lignes continues correspondent aux meilleurs ajustements sur la base du modèle $F^{norm} = y_0 + y_1 t + y_2 \exp(-t/\tau Rev)$. (B) Amplitudes du photoblanchiment réversible des protéines fluorescentes cyans.

**Figure 28 : Retour photoactivé de la fluorescence de la protéine fluorescente cyan ECFPr après photoblanchiment transitoire.** La protéine ECFPr a d'abord été photoblanchie en utilisant la puissance maximale de la lampe pendant moins de 1 min. Le retour de l'intensité de fluorescence après avoir stoppé l'illumination a alors été évaluée sous différents régimes d'illumination: les données expérimentales ont été normalisées (marqueurs) et les meilleurs ajustements (lignes continues) ont été évalués sur la base du modèle $F^{norm} = y_0 + y_1 t + y_2 \exp(-t/\tau_{Back})$.

**Figure 29 : Photoblanchiment réversible de protéines fluorescentes cyans, comprenant ou non la mutation 65S, dans des cellules vivantes MDCK.** Les conditions expérimentales sont identiques à celles utilisées pour les expériences sur billes d'agarose. Chaque courbe représente une moyenne de 4 à 6 déclins collectés à partir de différentes cellules individuelles. Les lignes continues sont les meilleurs ajustements sur la base du modèle $F^{norm} = y_0 + y_1 t + y_2 \exp(-t/\tau_{Rev})$.

**Figure 30 : Photoblanchiment irréversible de protéines fluorescentes cyans, comprenant ou non la mutation 65S, dans des cellules vivantes MDCK.** (A) Une illumination constante de densité de puissance 0.2W/$cm^2$ a été appliquée et la prise d'images a eu lieu toutes les 20 s durant cette illumination. Chaque courbe est la moyenne de 4 à 6 déclins collectés à partir de différentes cellules individuelles. Les lignes continues sont les meilleurs ajustements des déclins à partir d'un modèle exponentiel avec la constante de temps $\tau_{Irrev}$. (B) Dépendance de la constante de vitesse de blanchiment irréversible de la ECFPr en fonction de la densité de puissance.

## EXEMPLES

### A- MATERIEL ET METHODES

### I. Clonage de la protéine fluorescente cyan

Le plasmide d'expression de la ECFP (pHis-ECFP) a été construit à partir du vecteur pECFPN1 (Clontech) de la manière suivante : la séquence entière ECFP (SEQ ID N°1) du vecteur pECFPN1 a été amplifiée par PCR en utilisant les amorces 5'-AAGGCGCCGTGAGCAAGGGCGAGGAGCTG-3' (amorce sens) de séquence SEQ ID N°35 et 5'- TTAAGCTTACTTGTACAGCTCGTCCATGCC- 3' (amorce antisens) de séquence SEQ ID N°36.

Le produit de PCR obtenu a ensuite été digéré par HindIII et EheI, ligué dans le vecteur d'expression pPROEX-HTa (GibcoBRL), puis vérifié par cartographie de restriction et séquençage. Le clonage a été réalisé de façon à ce que la séquence MSYYHHHHHHDYDIPTTENLYFQGA (SEQ ID N°70) (qui comprend 6 Histidines et le site de clivage de la protéase TEV) du vecteur pPROEX-HTa soit insérée en N-Terminal de la ECFP, ceci afin de faciliter la purification de la protéine recombinante obtenue (ici de SEQ ID N°4). Les propriétés de fluorescence de la ECFP ne sont pas affectées par cette séquence.

### II. Méthode permettant de réduire la sensibilité au pH de la protéine fluorescente cyan

#### II.1. Mutagénèse dirigée

Des mutations uniques ont été introduites par mutagénèse dirigée dans la protéine fluorescente cyan selon le protocole « Quickchange mutagenesis » de Stratagène. Des amorces spécifiques ont été désignées pour chaque mutation (voir Tableau 2).

Ainsi, la mutation monomérique 148G a été introduite en utilisant les amorces 148Gf (SEQ ID N°37) et 148Gr (SEQ ID N°38) ; la mutation monomérique 148S a été introduite en utilisant les amorces 148Sf (SEQ ID N°39) et 148Sr (SEQ ID N°40); la mutation monomérique 65S a été introduite via les amorces 65Sf (SEQ ID N°49) et 65Sr (SEQ ID N°50).

Brièvement, les conditions de réaction suivies ont été les suivantes: à 39$\mu$L d'eau ont été ajoutés, 1$\mu$L de vecteur plasmidique exprimant la protéine fluorescente cyan à muter (e.g. pHis-ECFP) (10ng/$\mu$L), 1,5$\mu$L de chaque amorce sens et antisens spécifique de la mutation à introduire (100 ng/$\mu$L), $\mu$L de dNTPs (10 mM), 5$\mu$L de tampon de réaction 10x, et 1$\mu$L d'ADN polymérase *Pfu ultra HF* (2.5U/$\mu$L). L'amplification de la séquence nucléotidique mutée a été ensuite obtenue en soumettant ce mélange aux conditions suivantes dans un thermocycleur: un premier cycle de 2 minutes à 95°C, suivi par 18 cycles d'amplification dont chaque cycle est constitué de 30s à 95°C, 30s à 58°C et 10 minutes à 72°C, achevée par un dernier cycle de 10 minutes à 72°C. Enfin, la présence de la mutation dans le produit de PCR a été vérifiée par digestion avec l'enzyme DpnI (2,5U ; 1heure à 37°C) et séquençage de l'ADN.

**Tableau 2 :** mutations introduites par mutagénèse dirigée dans la protéine fluorescente cyan afin d'évaluer leur impact respectif sur la sensibilité au pH de la protéine.

| Mutation | Amorce sens (5'to 3') | Amorce antisens (5'to 3') |
|---|---|---|
| 148G | 148Gf : SEQ ID N°37<br>CAACTACATCAGCGGCAACGTCTATATCACC | 148Gr : SEQ ID N°38<br>GGTGATATAGACGTTGCCGCTGATGTAGTTG |
| 148S | 148Sf : SEQ ID N°39<br>CAACTACATCAGCTCCAACGTCTATATCACC | 148Sr :SEQ ID N°40<br>GGTGATATAGACGTTGGAGCTGATGTAGTTG |
| 148D | 148Df: SEQ ID N°41<br>CAACTACATCAGCGACAACGTCTATATCACC | 148Dr : SEQ ID N°42<br>GGTGATATAGACGTTGTCGCTGATGTAGTTG |
| 148R | 148Rf : SEQ ID N°43<br>CAACTACATCAGCCGCAACGTCTATAT CACC | 148Rr :SEQ ID N°44<br>GGTGATATAGACGTTGCGGCTGATGTAGTTG |
| 148N | 148Nf : SEQ ID N°45<br>GTACAACTACATCTCCAACAACGTCTATATC | 148Nr :SEQ ID N°46<br>GATATAGACGTTGTTGGAGATGTAGTTGTAC |
| 148E | 148Ef : SEQ ID N°47<br>CAACTACATCAGCGAGA ACGTCTATATCACC | 148Er : SEQ ID N°48<br>GGTGATATAG ACGTTCTCGC TGATGTAGTTG |
| 148A | 148Af : SEQ ID N°67<br>CAACTACATCAGCGCCAACGTCTATATCACC | 148Ar : SEQ ID N°68<br>GGTGATATAGACGTTGGCGCTGATGTAGTTG |
| 65S | 65Sf : SEQ ID N°49<br>CGTGACCACCCTGAGCTGGGGCGTGCAGTGC | 65Sr :SEQ ID N°50<br>GCACTGCACGCCCCAGCTCAGGGTGGTCACG |

Les mutations additionnelles suivantes ont été introduites avant ou après les mutations décrites ci-dessus, selon la même méthode de mutagénèse dirigée:

- 72A, en utilisant les amorces de séquences SEQ ID N°51 et SEQ ID N°52 ;
- 145A, en utilisant les amorces de séquences SEQ ID N°53 et SEQ ID N°54 ;
- 206K, en utilisant les amorces de séquences SEQ ID N°55 et SEQ ID N°56.

**II.2. Production et purification des mutants de la protéine fluorescente cyan**

La production et purification des mutants de la protéine fluorescente cyan a été réalisée selon le protocole décrit ci-dessous.

Les protéines fluorescentes cyan mutées ont été préparées en transformant des cellules compétentes TOP10 (Invitrogen) avec le vecteur d'intérêt (c'est-à-dire codant pour la protéine fluorescente mutée) selon les instructions du fabricant. Un volume préchauffé de 1,5 L de milieu Luria-Bertani (LB) contenant 100 $\mu$g/mL d'ampicilline a été inoculé avec 25 mL de culture de départ exprimant le vecteur d'intérêt et précédemment cultivée pendant la nuit. La production de protéines a été induite en ajoutant de l'isopropyl-D-thiogalactopyranoside (IPTG, 1 mM) aux cellules transformées lorsque l'$OD_{600}$ de ces cellules atteint une valeur de 0,6. Après avoir été cultivées pendant 18 h à 30 °C, les cellules induites ont été récoltées par centrifugation et congelées. Les cellules ont ensuite été resuspendues dans 30 ml de tampon de lyse (50 mM Tris-HCl, 5 mM de 2-mercaptoéthanol, 1 mM PMSF et 0,02 mg/mL de DNase) et soniquées. Enfin, une centrifugation (40,000 rpm, 1h30 à 6°C) a permis d'éliminer les débris cellulaires afin de ne récolter que le surnageant.

Pour la purification de chaque protéine fluorescente cyan mutée, le surnageant a été filtré à l'aide d'un filtre de 0,22 $\mu$m et dilué par un facteur 2 dans un tampon phosphate de pH 7,5 (30 mM $NaH_2PO_4$, 700 mM NaCl, 30 mM imidazole). Cette solution a été déposée dans une colonne contenant 5 mL de nickel nitro-acétique (Ni-NTA) agarose (15 mL, Sigma) pendant 1h. La colonne a été ensuite lavée (30 mM $NaH_2PO_4$, 100 mM NaCl, 10 mM imidazole, pH 7,5), et la protéine d'intérêt éluée (30 mM $NaH_2PO_4$, 100 mM NaCl, 150 mM imidazole, pH 7,5). Avant d'être stockée à -20°C, pour les expériences de spectroscopies, la solution de chaque protéine purifiée a été dialysée dans un tampon de concentration 2mM et de pH 7,4, constitué en concentration équimolaire de CAPS, de 2 - (N-morpholino) éthane acide (MES) et de Bis-tris propane puis concentrée de façon à obtenir une solution de protéine de concentration de l'ordre de 150 $\mu$M.

La concentration de protéines purifiées a ensuite été déterminée par un test utilisant l'acide bicinchoninique avec la BSA (bovine sérum albumine, 1 mg/mL) comme standard.

La pureté de la ECFPr a été estimée, via la spectrométrie de masse, comme étant supérieure à 99%. La pureté des mutants, purifiés selon le même protocole, a été estimée comme étant semblable (aucune différence de comportement n'a été observée sur les gels d'électrophorèse).

**II.3. Spectroscopie de fluorescence et évaluation de la sensibilité au pH**

Toutes les mesures spectroscopiques d'absorption et d'émission de fluorescence ont été réalisées sur des solutions de protéines purifiées dont la concentration ne dépassait pas 10 $\mu$M. La concentration en protéines était généralement de 10 $\mu$M aussi bien pour les mesures de spectroscopie stationnaire (spectres d'absorption et d'émission) que pour l'acquisition des déclins d'émission de fluorescence.

Pour les études de pH, des aliquots d'une solution concentrée de la protéine d'intérêt ont été dilués dans des tampons distincts préalablement ajustés au pH adéquat (précision de la mesure : 0,1 unité pH). La nature du tampon utilisé dépend de la zone de pH étudiée :

(i) pour les pH supérieurs à 5,5 (pH 5,5 à 11), le tampon utilisé est le MCBtP 33mM (mélange équimolaire des tampons usuels MES, CAPS et Bis-trispropane) dont le pH a été ajusté par l'ajout de $H_2SO_4$ ou de NaOH (Aldrich) ;
(ii) pour les pH inférieurs à 5,5 (pH 2,5 à 5,5), le tampon utilisé est l'acide citrique 50mM / NaOH, dont le pH a également été ajusté par l'ajout de NaOH.

L'ajout d'acide concentré directement à la solution de protéine fluorescente a été évité afin de ne pas entraîner une agrégation irréversible de la protéine.

II.3.a. <u>Spectroscopie de fluorescence stationnaire</u>

Les spectres d'absorption UV-visible ont été effectués sur un spectrophotomètre Perkin Elmer Lambda 900 à l'aide de cuvettes de quartz de 1 cm d'épaisseur, ayant des parois latérales noires (Hellma).

Les spectres d'émission de fluorescence de chaque protéine purifiée ont été mesurés sur un spectrofluorimètre Fluorolog3 de HORIBA Jobin Yvon, à température contrôlée (T = 20°C +/-0,1°C), en utilisant des cuvettes de quartz de 0,3 cm d'épaisseur dont les parois sont noires (Hellma 105-251-QS, Hellma Ltd). La largeur des fentes des monochromateurs d'excitation et d'émission a été fixée à 1 nm. Les spectres ont été alors collectés avec des temps d'intégration de 1s et des incréments de 1 nm. Le bruit de fond émis par le tampon fut soustrait.

Pour chaque protéine fluorescente purifiée, à partir des spectres d'émission de fluorescence, l'intensité à 474 nm (maximum du spectre d'émission de la ECFPr) a été mesurée et son évolution en fonction du pH déterminée. Ces intensités de fluorescence ont ensuite été corrigées des variations d'absorbance à 420nm (longueur d'onde d'excitation) ce qui permet une mesure de la variation du rendement quantique d'émission de fluorescence à 474 nm. Le pH de demi-transition, $pH_{1/2}$, a alors été mesuré comme étant la valeur de pH pour laquelle la somme de ces intensités de fluorescence à pH 7,4 et pH 2,5 correspondant à l'intensité de fluorescence la plus faible est réduite de moitié.

II.3.b. Spectroscopie de fluorescence résolue en temps

Les déclins d'émission de fluorescence ont été enregistrés à l'aide de la technique de comptage de photons unique (TCSPC) avec comme source d'excitation un laser Ti:Sapphire accordable, mode locké (MIRA 900, Coherent, Watford, UK) pompé optiquement par une diode laser à 532 nm (10W Verdi, Coherent).

Le taux de répétition des impulsions laser de 76 MHz a été réduit à 3,8 MHz à l'aide d'un sélecteur d'impulsions (cristal SiO2, APE, Berlin, Allemagne). Après le sélecteur d'impulsions, la longueur d'onde d'excitation de 420 nm a été obtenue par doublage de fréquence du rayonnement laser à 840 nm en utilisant un cristal doubleur BBO. Après le doublement de fréquence, la lumière d'excitation laser a été envoyée sur l'échantillon placé dans un porte-échantillon multi-position à température contrôlée. La puissance moyenne du laser sur l'échantillon a été typiquement de 1 à 1,5 $\mu$W (taille du faisceau d'environ 1-2 mm). Les courbes de décroissance de la fluorescence ont été recueillies par un dispositif électronique rapide (Ortec, Phillips & Tennelec). La fonction de réponse instrumentale (IRF) obtenue en mesurant la lumière diffusée par une solution de LUDOX (Dupont) a typiquement une largeur à mi-hauteur de 60-70 ps. L'excitation a été polarisée verticalement et la fluorescence de l'échantillon a été passée à travers un polariseur orienté à angle magique (54,7°), avant le monochromateur d'émission. La résolution spectrale du monochromateur est de 6 nm pour la plupart des expériences, à l'exception des études de pH réalisées pour la ECFPr (H148D), où cette résolution était de 24 nm. La fluorescence de l'échantillon et l'IRF ont été collectés alternativement sur plusieurs dizaines de cycles afin d'obtenir des statistiques suffisantes: environ $20.10^6$ couts totaux ont été collectées pour chaque courbe de décroissance, à des taux d'environ $10^4$ cts par seconde.

**III. Développement d'un biosenseur de sensibilité réduite au pH**

**III.1. Construction du biosenseur pHAKAR**

Le biosenseur pHAKAR de sensibilité réduite au pH a été construit à partir du biosenseur AKAR2.2 (Dunn et al., 2006), qui est constitué de la Citrine et d'une protéine fluorescente cyan (comprenant notamment les mutations 26R, 164H et 206K) en tant qu'accepteur et donneur respectifs d'énergie, ainsi que d'un capteur biosensible pris en sandwich entre ces deux protéines, lui-même constitué d'un domaine de liaison aux acides aminés phosphorylés (FHA) et d'une séquence substrat de la protéine kinase A (dite PKA). La phosphorylation de la thréonine de la séquence substrat par la PKA entraîne la reconnaissance de cette dernière par le domaine de liaison FHA, ce qui conduit à un changement conformationel du biosenseur, qui entraîne en retour une augmentation du signal de FRET entre la protéine fluorescente cyan et la Citrine.

La séquence nucléotidique codant pour le biosenseur AKAR2.2 a été tout d'abord insérée dans le vecteur pcDNA3 (Life Technologies).

Les mutations 65S et 148G ont ensuite été introduites dans le plasmide pcDNA3-AKAR2.2 par mutagénèse ponctuelle via le kit « Quickchange » (Stratagene) en utilisant les couples d'amorces de séquence SEQ ID N°49 et SEQ ID N°50, et celles de séquence SEQ ID N°37 et SEQ ID N°38, respectivement. La protéine fluorescente cyan du biosenseur comprend ainsi les mutations 65S et 148G de l'invention.

La protéine monomérique à émission fluorescente orange TagRFP (Merzlyak et al., 2007) a ensuite été clonée en lieu et place de la Citrine de la manière suivante : la séquence nucléotidique codant pour la protéine fluorescente cyan mutée du biosenseur et pour le capteur biosensible a été tout d'abord clonée dans le vecteur pPROEXHTa (GibcoBRL) entre les sites de restriction HindIII et SacI ; puis la séquence nucléotidique codant pour la protéine TagRFP a été amplifiée par PCR à partir du vecteur commercial pTagRFP-C (Evrogen) en utilisant les amorces TagRFPf de séquence SEQ ID N°77 (5'ATTAGAGCTCATGGTGTCTAAGGGCGAA 3') et TagRFPr de séquence SEQ ID N°78 (5' ATAATGAATTCTTAATTAAGTTTGTGCC CC 3') et clonée entre les sites SacI et EcoRI du vecteur pPROEXHTa [ECFPr de séquence SEQ ID N°82 - capteur biosensible].

Le biosenseur à protéine kinase A ainsi obtenu, dénommé pHAKAR, contenant le couple de protéines fluorescentes ECFPr de séquence SEQ ID N°82 / TagRFP, ainsi que le capteur biosensible, a alors été introduit entre les sites de restrictions HindIII et EcoRI du vecteur plasmidique pCDNA3. Chaque clonage a été vérifié par séquençage.

Les plasmides codant pour la protéine ECFPr (65S, 148G) (pECFPN1, Clontech) et pour le biosenseur pHAKAR (pcDNA3-pHAKAR) ont été amplifiés dans *E.Coli* et stockés à une concentration de 1 μg/μL.

### III.2. Spectroscopie de fluorescence et évaluation de la sensibilité au pH du biosenseur pHAKAR

Des cellules de hamster BHK ont été cultivées dans des flasques de 25cm$^2$ en présence de milieu DMEM supplémenté avec 10% de sérum foetal de veau (Gibco). A l'occasion d'un repiquage, ces cellules ont été déposées sur des lamelles de verre de 25 mm de diamètre posées au fond d'une plaque 6 puits, puis transfectées par lipotransfection à 90% de confluence avec le plasmide pECFPNlou pcDNA3-pHAKAR en suivant le protocole du fabricant (4 μg de plasmide et 10 μL de Lipofectamine2000 pour 2 mL de milieu ; Life Technologies). 24h après transfection, les lamelles ont été montées dans une chambre métallique attofluor (Life Technologies). Le milieu utilisé pour les mesures contient 140 mM de KCL, 15 mM de tampon MES et 15mM de tampon Hepes dont le pH a été ajusté à la valeur désirée, 7,4 ou 5,9. Le pH intracellulaire a été ajusté au pH extérieur au moyen d'un ionophore, la nigéricine (concentration finale 10 μM). Après 5 min d'incubation avec la nigéricine à 37°C, les cellules transfectées ont été placées à 20°C sur la platine du microscope.

La durée de vie fluorescence émise par la protéine ECFPr comprenant au moins les mutations 65S et 148G, seule ou incorporée dans le biosenseur pHAKAR, a alors pu être observée à différents pH (7,40 et 5,9) à l'aide d'un microscope NIKON TE2000 équipé d'un objectif à immersion à eau (x60, NA 1,2), d'une lampe à mercure et d'un système de détection de fluorescence permettant d'identifier les cellules fluorescentes (filtres : Oméga XF114-2 pour la ECFPr et TRITC-B-NTE Semrock pour la TagRFP), ainsi que d'une voie d'excitation et de détection pour les mesures de durées de vie de fluorescence. L'excitation a été réalisée à l'aide d'une diode pulsée à 442 nm (LDH 440 et PDL 800D driver, PicoQuant) qui est fibrée et injectée dans une tête de scan de type C1 (Nikon) initialement utilisée pour la microscopie confocale. La tête de scan a été pilotée par le logiciel EZ-C1 et permet le contrôle spatial de l'excitation de l'échantillon. Sous la tourelle de l'objectif, les photons de fluorescence ont été réfléchis par un miroir dichroïque escamotable (SWP-500 à 45°, Lambda Research) vers une galette de microcanaux (Hamamastu) après filtrage (un filtre 480AF30 Omega et deux Razor Edge Longpass à 458nm Semrock). Les signaux ont ensuite été collectés par un module de comptage de photons PicoHarp300 (Picoquant) qui est aussi synchronisé avec les impulsions du laser d'excitation. Les données ont été analysées par le logiciel SymPhoTime (Picoquant). Chaque cellule a été identifiée comme une région d'intérêt, l'histogramme des durées de vie de fluorescence a alors été calculé sur l'ensemble des pixels de cette région. Le nombre total de coups est compris entre 1 et 5.10$^6$, et la durée de vie moyenne a été calculée par SymPhoTime. 5 à 10 cellules ont été mesurées pour chaque condition de pH.

## IV. <u>Etude de l'influence de la mutation 65S sur le rendement quantique, le déclin de fluorescence et la photostabilité de la protéine fluorescente cyan ECFP</u>

Les protéines ECFPr, ECFPr (65S), ECFPr (72A, 145A et 148D) et ECFPr (65S, 72A, 145A et 148D) ont été générées, produites et purifiées selon le protocole du point II décrit ci-dessus.

### IV.1. Analyse des données de spectroscopie

Chaque déclin d'émission de fluorescence F(t) avec la fonction d'appareil correspondante (IRF) a été analysé individuellement en utilisant la méthode du maximum d'entropie décrit par Mérola et al. (1989) et Couprie et al. (1994). Cette analyse suppose que le déclin F(t) expérimental soit le produit de convolution suivant :

$$\mathrm{F(t)} = g(t) * I_m(t)$$

où g(t) est l'IRF mesuré, et $I_m(t)$ représente la loi d'émission de fluorescence I(t) consécutive à une excitation infiniment brève. L'analyse suppose que la loi du déclin de fluorescence soit composée d'un grand nombre de termes exponentiels. Le déclin total correspond ainsi à l'équation suivante :

$$I_m(t) = I_0 \int_0^{\infty} \alpha(\tau) \exp^{-t/\tau} d\tau$$

où $\alpha(t)$ est la distribution des amplitudes pré-exponentielles normalisées (i.e. $\int \alpha(\tau)\, d\tau = 1$) et $I_o$ est un facteur arbitraire incorporant les conditions expérimentales de mesure. Un décalage dans le temps entre F(t) et g(t) a également été optimisé, et la valeur du $\chi^2$ réduit se trouve dans la gamme de valeur 0,97 à 1,05, avec des résidus et des fonctions d'autocorrélation distribués de façon aléatoire.

A partir de la distribution de vie de fluorescence $\alpha(t)$ récupérée par cette méthode, un petit nombre de composants individuels ($\tau_i$) et leurs amplitudes pré-exponentielles correspondantes ($c_i$) ont été obtenus par intégration de chaque pic séparé observé dans la distribution. La distribution $\alpha(t)$ a été utilisée pour calculer la durée de vie moyenne de fluorescence $<\tau_f>$ qui doit être proportionnel au rendement quantique de fluorescence (Valeur B., 2006):

$$<\tau_f> = \sum_i c_i \cdot \tau_i = \int_0^{\infty} \alpha(\tau)\ \tau\ d\tau$$

Les incertitudes de mesure sur $c_i$, $\tau_i$ et $<\tau_f>$ ont été déterminées à partir des déviations standard sur plusieurs expériences identiques répétées.

**IV.2. Rayonnement synchrotron de dichroïsme circulaire**

Les mesures ont été effectuées sur la ligne de lumière DISCO au synchrotron Soleil (Gif sur Yvette, France) (Giuliani et al., 2009), en utilisant des cuvettes circulaires de fluorure de calcium (Hellma) de 11 $\mu$m (Wien et al., 2005).

Les concentrations en protéines étaient typiquement de 8 mg/L à pH 2,5 et de 18 mg/L à pH 7,4. Tous les échantillons ont été préparés la veille dans leur tampon (30 mM CAPS, 30 mM MES et 30 mM de Bis-tris propane à pH 7,4 et 30 mM d'acide citrique à un pH de 2,5). Pour chaque protéine, 3 scans, de 170 à 280 nm avec des intervalles de 1 nm par seconde ont été enregistrés puis moyennés. Trois balayages consécutifs de la ligne de base (en utilisant le tampon) ont été obtenus de la même manière et moyennés. Pour toutes les protéines, les expériences ont été enregistrées à 25°C. Le spectre du tampon a été soustrait à celui des échantillons correspondants. La région de 260-270 nm a été réglée à zéro, et les spectres résultant ont été calibrées avec du CSA (acide D-10-camphosulfonique) en utilisant le logiciel CDtool (Lees et al., 2004). Le dichroïsme circulaire moyen par résidu s'exprime en $M^{-1}.cm^{-1}$ (Kelly et al., 2005). La détermination de la structure secondaire a été réalisée sur DICHROWEB (Whitmore et al., 2004) en utilisant les algorithmes CDSSTR et CONTINLL ainsi que la base de données SP175 (Lees et al., 2006). Les deux algorithmes fournissent des résultats similaires. Les résultats ont été obtenus à l'aide de l'algorithme CDSSTR. Le paramètre d'ajustement NRMSD varie de 0,030 à 0,050 pour toutes les protéines.

**IV.3. Expériences de photoblanchiment**

IV.3.a. Marquage de billes d'agarose avec les protéines fluorescentes cyans

Des billes d'agarose chargées de nickel (Sigma) ont été marquées avec les protéines recombinantes mutées ci-dessus mentionnées. 100 $\mu$Lde billes sédimentées, préalablement lavées et équilibrées avec un tampon phosphate (pH 7,5) ont été incubées avec 1 à 5 $\mu$M de protéine purifiée dans un volume total de 1 mL pendant 1h sous agitation douce dans le froid. Les billes ont ensuite été centrifugées pendant 5 min à 5000 rpm, lavées deux fois et remises en suspension dans du PBS. Quelques microlitres de la suspension de billes ont été déposés sur une lamelle de microscope de 25 mm de diamètre pour le photoblanchiment.

IV.3.b. Expression des protéines fluorescentes cyans cytosoliques

Des cellules MDCK cultivées sur des lamelles de 25 mm de diamètre ont été transitoirement transfectées avec les plasmides d'expression eucaryotes codant pour la protéine fluorescente cyan d'intérêt à l'aide de Lipofectamine2000, conformément aux recommandations du fabricant (Invitrogen). Ces cellules ont été étudiées 24 à 48 heures après leur transfection.

IV.3.c. Illumination et conditions d'imagerie

Les expériences de photoblanchiment de fluorescence des protéines fluorescentes cyans ont été effectuées à 20°C+/-0,5°C à l'aide d'un microscope à épifluorescence équipé d'un objectif à immersion à eau (x60, NA 1.2 ; Nikon), d'une lampe HBO 100W Hg, et en utilisant un filtre dichroïque ECFP pour détecter la fluorescence (Oméga XF114-2). La puissance d'éclairage a été ajustée à l'aide de filtres de densité neutre, la puissance maximale mesurée sur l'échantillon sans aucune atténuation était approximativement de 200 μW (FieldMaster avec détecteur 13M41, Coherent), et le rayon du champ illuminé a été estimé à 185 μm, conduisant à un éclairement moyen sur la échantillon d'environ 0,2 W/cm2. Des billes uniques ou des groupes de cellules MDCK ont été placées au centre du champ d'imagerie, et l'intensité de fluorescence mesurée via une caméra CCD refroidie (ORCA-AG Hamamatsu) a été quantifiée en utilisant le logiciel NIH ImageJ. Aucune dépendance significative des taux de photoblanchiment mesurés en fonction de la taille des billes, ou de la densité de marquage des billes n'a pu être observée.

IV.3.d. Modélisation et analyse des expériences de photoblanchiment

Modèle cinétique

Il est supposé que la protéine fluorescence cyan ECFP subit une réaction réversible photoactivée entre deux états, un état fluorescent et un non fluorescent, caractérisés par des constantes de vitesse $k_{off}$ et $k_{on}$.

$$CFP_{on} \underset{k_{on}}{\overset{k_{off}}{\rightleftarrows}} CFP_{off}$$

Ce modèle néglige la relaxation thermique entre ces états, ainsi que le photoblanchiment irréversible (ceux-ci ayant lieu sur des échelles de temps plus lent, selon les conditions expérimentales ici testées), et suppose une absorption identique des deux formes. Il en résulte les équations suivantes:

$$\frac{d[CFP_{on}]}{dt} = -k_{off}[CFP_{on}] + k_{on}[CFP_{off}]$$

$$\frac{d[CFP_{off}]}{dt} = -k_{on}[CFP_{off}] + k_{off}[CFP_{on}],$$

En posant x, la fraction relative de l'état fluorescent

$$x = \frac{[CFP_{on}]}{[CFP_{on}] + [CFP_{off}]}$$

et en prenant $x_0$ =1 (pas d'état non-fluorescent au temps zéro, lorsque le système est à l'équilibre thermique dans l'obscurité,), on obtient :

$$x = \left(1 - \frac{k_{on}}{k_{on} + k_{off}}\right)\exp\left(-\left(k_{on} + k_{off}\right)t\right) + \frac{k_{on}}{k_{on} + k_{off}}$$

c'est-à-dire, sous une illumination instantanée, la fraction molaire de l'état fluorescent diminue suivant une monoexponentielle avec une constante de temps $\tau_{Rev}$ = 1/($k_{on}$+$k_{off}$), et vers un niveau stationnaire $y_0$ = $k_{on}$/( $k_{on}$+$k_{off}$).

Ajustement des expériences de photoblanchiment réversible

Après normalisation de l'intensité initiale à 1, les courbes de photoblanchiment transitoires ont été ajustées par le modèle

$$F^{Norm} = y_0 + y_1 t + y_2 \exp\left(-t / \tau_{\mathrm{Rev}}\right)$$

où $y_0$ représente le niveau de fluorescence stationnaire, $y_1$ la constante de vitesse de photoblanchiment irréversible supposant une loi linéaire (les valeurs de $y_1$ sont généralement ≈ $-1{*}10^{-3}s^{-1}$, en accord avec les constantes de vitesse de photoblanchiment irréversible obtenues (voir la Figure 22), tandis que $y_2$ et $\tau_{Rev}$ représentent respectivement l'amplitude relative et la constante de temps du blanchiment réversible.

Le retour de la fluorescence après photoblanchiment transitoire est accéléré sous illumination.

Après normalisation de l'intensité initiale à zéro et de l'intensité maximale à 1, les courbes de retour de l'intensité de fluorescence ont été ajustées suivant le même modèle analytique :

$$F^{Norm} = y_0 + y_1 t + y_2 \exp\left(-t / \tau_{Back}\right)$$

Les temps de relaxation pour le retour de la fluorescence $\tau_{Back}$ sont en excellent accord avec les temps de relaxation pour la perte de fluorescence $\tau_{Rev}$ (Figure 28).

Toutes les constantes de vitesse $k_{on}$ et $k_{off}$ dépendent de l'intensité de l'illumination

Les constantes de vitesse $k_{on}$ et $k_{off}$ sont obtenues directement à partir de $y_0$ et $\tau_{Rev}$:

$$k_{on} = \frac{y_0}{\tau_{\mathrm{Rev}}} \qquad k_{off} = \frac{1 - y_0}{\tau_{\mathrm{Rev}}}$$

Selon le modèle cinétique à deux états, supposant une dépendance linéaire de $k_{on}$ et $k_{off}$ vis-à-vis de la puissance d'illumination, le temps de relaxation $\tau_{Rev}$ devrait également dépendre de manière linéaire de la puissance d'excitation (ou irradiance, en W/cm2), ce qui ne devrait pas être le cas de la fraction de perte de fluorescence réversible (1 - $y_0$), (ceci est vérifié approximativement pour une puissance d'excitation faible ; voir notamment la Figure 28).

Les rendements quantiques de photoconversion de la protéine fluorescente cyan

Les rendements quantiques $\phi_{off}$ et $\phi_{on}$ des réactions de photoconversion de la protéine fluorescente cyan ECFPr sont donnés par les équations suivantes :

$$\Phi_{on} = \frac{k_{on}}{k_{exc}} \qquad \Phi_{off} = \frac{k_{off}}{k_{exc}}$$

où $k_{exc}$ est le vitesse d'excitation par molécule, donnée par le produit de la section efficace d'absorption $\sigma_{exc}$ par le flux de photons $N_{exc}$ à longueur d'onde d'excitation $\lambda_{exc}$:

$$k_{exc} = \sigma_{exc}(cm^2) \times N_{exc}\ (Photons/s/cm^2)$$

Pour la protéine fluorescente cyan ECFPr, avec $I_{exc}$ = 0,05 W/cm2, et en supposant que $\varepsilon_{on} = \varepsilon_{off} \approx 30000\ M^{-1}cm^{-1}$ à $\lambda_{exc}$=440 nm, on obtient $k_{exc}$= 13 $s^{-1}$, avec $k_{off}$ = 0.18 $s^{-1}$ et $k_{on}$= 0.77 $s^{-1}$, donnant $\phi_{off}$= 1.4% et $\phi_{on}$ =6.1%.

**B- RESULTATS**

**I. Comparaison de la sensibilité au pH de la ECFP, et des protéines fluorescentes cyan mutées selon l'invention, en fonction du pH**

Les inventeurs ont souhaité étudier l'impact de diverses mutations sur la sensibilité au pH de la ECFP, dans le but de générer des protéines fluorescentes cyans ayant une sensibilité réduite au pH, et plus particulièrement aux pH acides.

Ils ont ainsi introduit diverses mutations uniques dans la ECFP sous forme recombinante (ECFPr), et étudié leur effet sur la perte d'intensité de fluorescence et la diminution de durée de vie de fluorescence habituellement observées chez cette protéine lorsque le pH passe d'un pH basique à un pH acide. Les résultats de cette étude sont répertoriés dans le Tableau 3 ci-après.

En effet, comme démontré sur la Figure 1 et dans le Tableau 3 (ci-dessous), la ECFPr présente une perte d'intensité de fluorescence de 50%, et une diminution de durée de vie de fluorescence de 33% lorsque le pH diminue de 7,4 à 5,5. Des modifications spectrales sont également visibles lorsque le pH devient acide. Ces propriétés limitent fortement la fiabilité de l'utilisation de la ECFPr dans des méthodes d'imagerie quantitative en cellules vivantes, comme le FRET, qui sont particulièrement sensibles à la moindre variation du signal de fluorescence. En effet, le pH intracellulaire varie selon les compartiments cellulaires dans lesquels la ECFPr est solubilisée et selon les conditions expérimentales testées.

Par ailleurs, le pH de demi-transition ($pH_{1/2}$) de la ECFPr mesuré par les inventeurs est de 5,6, et sa durée de vie de fluorescence à pH neutre de 2,5 ns. Il faut noter que la valeur du $pH_{1/2}$ de la ECFPr déterminé par les inventeurs diffère de celle mentionnée dans la littérature, qui est de environ 4,6-4,7. Seule une analyse objective telle que réalisée ici permet donc d'étudier convenablement les réelles différences de $pH_{1/2}$ entre les protéines de l'invention et celles de l'art antérieur.

Afin de résoudre le problème de sensibilité au pH de la ECFP, les inventeurs ont donc tout d'abord introduit une mutation unique en position 148 de la ECFPr.

L'introduction de la mutation 148D a généré une protéine fluorescente cyan présentant une perte d'intensité de fluorescence d'environ 50%, et une diminution de la durée de vie de fluorescence de 40%, lorsque le pH passe de 7,4 à 5,5. Sa durée de vie de fluorescence à pH neutre est toutefois plus élevée que celle de la ECFPr puisqu'elle atteint 3,3 ns alors que celle de la ECFPr est de 2,5 ns (Tableau 3). Cette mutation n'a donc pas permis d'atténuer la perte d'intensité et de durée de vie de fluorescence à pH acide par rapport à la ECFPr, mais elle améliore significativement la durée de vie à pH neutre.

Les inventeurs ont alors introduit la mutation 148G dans la ECFPr, générant ainsi la protéine ECFPr (148G). Cette protéine présente une perte d'intensité de fluorescence de seulement 18%, et une diminution de la durée de vie de fluorescence de 6% à pH acide. Son $pH_{1/2}$ est de 4,9, et sa durée de vie de fluorescence à pH neutre de 3,37 ns (Figure 2 et Tableau 3). La mutation 148G permet donc de réduire la sensibilité au pH de la ECFP.

Les inventeurs ont ensuite testé d'autres mutations en position 148 de la ECFP, et en particulier les mutations 148E, 148S et 148A.

La protéine ECFPr (148E) présente une perte d'intensité de fluorescence de 27%, et une diminution de la durée de vie de fluorescence de 18%, entre pH 7,4 et pH 5,5. Son $pH_{1/2}$ est de 5,1 et sa durée de vie de fluorescence à pH neutre de 3,15 ns (Figure 15 et Tableau 3).

La protéine ECFPr (148S) ainsi générée montre une perte d'intensité de fluorescence de seulement 9%, et une diminution de la durée de vie de fluorescence de 15%, entre pH 7,4 et pH 5,5. Par ailleurs, le $pH_{1/2}$ de cette protéine est de 4,5 et sa durée de vie de fluorescence à pH neutre de 3,17 ns (Figure 3 et Tableau 3). L'introduction de la mutation 148S dans la ECFP permet donc d'obtenir des protéines fluorescentes cyan ayant une sensibilité réduite au pH.

La protéine ECFPr (148A) montre, quant à elle, une perte d'intensité de fluorescence de seulement 7%, et une diminution de la durée de vie de fluorescence de 4%, entre pH 7,4 et pH 5,5. Par ailleurs, le $pH_{1/2}$ de cette protéine est de 4,5 et sa durée de vie de fluorescence à pH neutre de 3,15 ns (Figure 4 et Tableau 3). L'introduction de la mutation 148A dans la ECFP permet donc elle aussi d'obtenir des protéines fluorescentes cyan ayant une sensibilité réduite au pH.

L'acide aminé en position 148 de la ECFP semble donc jouer un rôle important dans la sensibilité au pH de cette protéine.

Par la suite, les inventeurs se sont intéressés à l'acide aminé en position 65 de la ECFP. Ils y ont introduit par mutagénèse dirigée la mutation 65S, générant ainsi la protéine ECFPr (65S). Cette protéine présente des propriétés avantageuses puisque sa perte d'intensité de fluorescence est seulement de 15% et sa diminution de la durée de vie de fluorescence de 16% lorsque le pH passe de 7,4 à 5,5. Le $pH_{1/2}$ de cette protéine est par ailleurs de 4,5 et sa durée de vie de fluorescence de 3,3 ns (Figure 5 et Tableau 3). L'acide aminé en position 65 semble donc également influer les propriétés de sensibilité au pH de la ECFP.

Des mutations additionnelles à celles introduites en position 148 ou 65 ont été par ailleurs évaluées. Celles-ci ont été choisies parmi les mutations 72A, 145A, et 206K.

Ainsi, la protéine fluorescente cyan ECFPr (72A, 145A, 148D) présente des modifications spectrales, tout comme la ECFPr. Ses pertes d'intensité de fluorescence et de durée de vie sont respectivement de 33% et 32% lorsque le pH passe de 7,4 à 5,5, et donc inférieures à celles de la ECFPr. Son pH de demi-transition ($pH_{1/2}$) est quant à lui de 5,2 (Figure 7 et Tableau 3). Néanmoins, la réduction de sensibilité au pH conférée par la combinaison de ces mutations est moins avantageuse que celle conférée par la mutation unique 148G ,148S, 148A, ou 65S.

Les inventeurs ont alors remplacé la mutation 148D par la mutation 148G, générant ainsi la ECFPr (72A, 145A, 148G). Cette combinaison de mutations a permis de réduire la sensibilité au pH puisque les pertes d'intensité et de durée de vie de fluorescence sont seulement de 13% et 3%, respectivement, lorsque le pH passe de 7,4 à 5,5. En outre, le $pH_{1/2}$ de cette protéine est inférieur à celui de la ECFPr, et sa durée de vie de fluorescence à pH neutre est augmentée (Tau= 3,13 ns) (Figure 8 et Tableau 3).

La combinaison de la mutation 65 S aux mutations 72A et 206K permet également de réduire la sensibilité au pH (voir Figure 12 et Tableau 3) par rapport à la ECFP. Toutefois, les pertes d'intensité et de durée de vie fluorescence observées chez cette protéine (ECFPr (65S, 72A, 206K)) sont plus importantes que celles observées avec la mutation unique 65 S.

Les inventeurs ont alors testé des doubles mutations en position 148 et 65 en présence ou non de mutations additionnelles, qui sont ici les mutations 72A et 206K.

L'introduction de ces doubles mutations en présence ou non de mutations additionnelles permet de réduire drastiquement la sensibilité au pH de la ECFP, voire même de l'abolir (voir Tableau 3).

En effet, les pertes d'intensité et de durée de vie de fluorescence sont proches de 0%, voire complètement nulles, lorsque le pH passe d'un pH basique à un pH acide pour les protéines ECFPr (65S, 72A, 145A, 148D), ECFPr (65S, 72A, 148D, 206K) et ECFPr (65S, 72A, 148G, 206K), ECFPr (65S, 148G), ECFPr (65S, 148D), et ECFPr (65S, 148S).

Parmi ces protéines, la ECFPr présentant seulement les doubles mutations (65S, 148G) et (65S, 148S) ainsi que la ECFPr (65S, 72A, 148G, 206K) présentent les meilleures propriétés de toutes les protéines fluorescentes cyans mutées générées par les inventeurs: en effet, les pertes d'intensité et de durée de vie de fluorescence observées sont nulles lorsque le pH passe de 7,4 à 5,5, leur $pH_{1/2}$ respectif est de 3,4 ; 3,6 et 3,1, et leur durée de vie moyenne respective de fluorescence à pH neutre est de 4,12 ; 3,86 et 4,06 ns.

La ECFPr (65S,148G) est en outre la meilleure de toutes les protéines fluorescentes cyans générées par les inventeurs en termes de durée de vie de fluorescence.

En conclusion, l'introduction de mutation(s) unique(s) en position 148 et/ou 65 dans la ECFP, et plus particulièrement des mutations 148G/A et/ou 65S, permet de générer des protéines fluorescentes cyans dont la sensibilité au pH est réduite, voire abolie. L'utilisation de telles protéines dans des applications d'imagerie quantitative permettra donc de quantifier de manière plus fiable les signaux de fluorescence qu'elles émettent.

**Tableau 3 : Comparaison de la dépendance au pH de différents mutants de**

| Protéines étudiées | $<\tau>$ (ns) à pH 7,4 | $pH_{1/2}$ | Pourcentage de perte d'intensité (If) entre pH 7,4 et 5,5 | Pourcentage de perte de $<\tau>$ pH 7,4 à 5,5 |
|---|---|---|---|---|
| ECFPr (SEQ ID N° 4) | 2,5 | 5,6 | 50% | 33% |
| ECFPr (148G) | 3,37 | 4,9 | 18% | 6% |
| ECFPr (1485) | 3,17 | 4,5 | 9% | 15% |
| ECFPr (148A) | 3,15 | 4,5 | 7% | 4% |
| ECFPr (148D) | 3,3 | 5,5 | 50% | 40% |
| ECFPr (148N) | 2,93 | 5,7- | 59%- | - |
| ECFPr (148E) | 3,15 | 5,1- | 27%- | 18% |
| ECFPr (148R) | 2,04 | 5,7 | 45% | - |

(suite)

| Protéines étudiées | <τ> (ns) à pH 7,4 | $pH_{1/2}$ | Pourcentage de perte d'intensité (If) entre pH 7,4 et 5,5 | Pourcentage de perte de <τ> pH 7,4 à 5,5 |
|---|---|---|---|---|
| ECFPr (65S) | 3,3 | 4,5 | 15% | 16% |
| ECFPr (72A, 145A, 148D) | 3,06 | 5,2 | 33% | 32% |
| ECFPr (72A, 145A, 148G) | 3,13 | 4,4 | 13% | 3% |
| ECFPr (65S, 72A, 145A, 148D) | 3,95 | 3,6 | 0% | 0% |
| ECFPr (65S, 72A, 148D, 206K) | 4,05 | 3,9 | 0% | 0% |
| ECFPr (65S, 72A, 148G, 206K) | 4,06 | 3,1 | 0% | 0% |
| ECFPr (65S, 72A, 206K) | 3,38 | 4,8 | 23% | 15% |
| ECFPr (655, 148G) | 4,12 | 3,4 | 0% | 0% |
| ECFPr (655, 148D) | 4,02 | 4 | 0% | 0% |
| ECFPr (655, 148E) | 3,86 | 4,1 | 0% | 0% |
| ECFPr (65S, 148S) | 3,86 | 3,6 | 0% | 0% |

**la protéine fluorescente cyan.** (<τ> = durée de vie de fluorescence moyenne, en nanosecondes (ns) ; If = Intensité de fluorescence).

**II. <u>Etude de la sensibilité au pH d'un nouveau biosenseur</u>**

A pH physiologique (7,4), les inventeurs ont constaté que la durée de vie moyenne de fluorescence émise par la protéine fluorescente cyan ECFPr incorporée dans le biosenseur pHAKAR et dont la séquence comprend entre autres les mutations 65S et 148G est réduite de 16% par rapport à la protéine ECFPr (65S, 148G) exprimée seule (3,32 ns versus 3,96 ns). Cette baisse de la durée de vie de fluorescence est liée au transfert d'énergie entre la protéine fluorescente cyan et la TagRFP, et non à la présence des mutations additionnelles 26R, 164H et 206K qui sont des mutations silencieuses d'un point de vue des propriétés photophysiques des protéines fluorescentes cyans.

Cependant, lorsque le pH intracellulaire est abaissé à 5,9, la durée de vie moyenne de fluorescence de la protéine ECFPr mutée du biosenseur pHAKAR n'est que très légèrement modifiée, passant de 3,32 ns à 3,23 ns, soulignant l'insensibilité au pH du biosenseur pHAKAR.

Au vu de ces résultats, le couple de protéines fluorescentes ECFPr (65S,148G)

- pouvant par ailleurs comprendre des mutations additionnelles qui n'affectent pas ses propriétés photophysiques - et TagRFP semble donc constituer un couple de FRET performant et de sensibilité réduite au pH.

**III. <u>Etude de l'influence de la mutation 65S sur le rendement quantique, le déclin de fluorescence et la photostabilité des protéines fluorescentes cyans</u>**

Comme discuté ci-dessus, l'introduction de la mutation 65S a pour effet de réduire la sensibilité au pH de la protéine fluorescente cyan ECFP. Les inventeurs ont également découvert que l'introduction de cette mutation permet également d'augmenter le rendement quantique, de simplifier la cinétique d'émission de fluorescence, et d'améliorer la photostabilité de cette protéine au photoblanchiment.

**III.1. La mutation 65S améliore l'homogénéité et le rendement quantique des** protéines fluorescentes cyans

Les propriétés d'absorption et d'émission de fluorescence des protéines purifiées ECFPr et ECFPr (72A, 145A, 148D) ont été comparées, à pH neutre et température ambiante à celles des protéines comprenant la mutation 65S, à savoir les protéines ECFPr-65S et ECFPr (65S, 72A, 145A, 148D). Les inventeurs ont constaté que les spectres d'absorption et de fluorescence de ces protéines cyans sont très similaires, et présentent des spectres d'absorption et d'émission bimodales typiques des chromophores de type indole, avec deux maximas pour l'absorption à 430 nm et 445nm, et deux maximas pour l'émission à 474nm et 500nm (Figure 21). En prenant en compte les incertitudes expérimentales, des coefficients d'absorption très semblables ont été observés pour toutes les protéines étudiées, dans la plage de 32000 $\pm$ 4000 $M^{-1}.cm^{-1}$ à 430 nm (Tableau de la Figure 22). Malgré une similitude spectrale étroite, ces protéines diffèrent nettement par leur rendement quantique (Figure 22), ainsi que par leur distribution de durée de vie de fluorescence, tels que calculés à partir de leur déclin d'émission de fluorescence (Figure 23). Ces distributions de durée de vie comprennent dans tous les cas une durée de vie de fluorescence majeure et longue, qui est associée à des composantes de temps court de population variable (Figure 22). La protéine ECFPr (72A, 145A, 148D) a un rendement quantique accru par rapport à celui de la protéine ECFPr, mais conserve une émission de fluorescence hétérogène, sa plus longue durée de vie contribuant seulement à 64% de l'amplitude du déclin total (Figures 22 et 23).

La mutation unique 65S introduite selon la méthode de l'invention améliore de manière considérable les performances des protéines ECFPr et ECFPr (72A, 145A, 148D). Ainsi, le rendement quantique de fluorescence de la protéine ECFPr-65S est augmenté de 48% par rapport à ECFPr, tandis que celui de ECFPr (65S, 72A, 145A, 148D) augmente de 25% par rapport à ECFPr (72A, 145A, 148D) (Figure 22). La mutation 65S se traduit également par une simplification notable de la distribution de durée de vie de fluorescence (Figure 23). Dans le cas de la protéine ECFPr (72A, 145A, 148D), la mutation 65S conduit à un rendement quantique de fluorescence et de durée de vie moyenne élevés, et le déclin de fluorescence suit une cinétique quasi-monoexponentielle avec une amplitude de durée de vie de 83 % (Figure 22).

### III.2. La mutation 65S supprime les formes intermédiaires des protéines fluorescentes cyans détectées lors de la transition acide

L'analyse détaillée des spectres d'absorption et d'émission de fluorescence de la ECFPr et de ses mutants lors de la transition acide apporte de nombreuses preuves de l'existence de formes intermédiaires. Notamment, dans le cas de la ECFPr, un déplacement vers le rouge du maximum du spectre d'émission de fluorescence est observé suite à une acidification du milieu, et ce jusqu'à un pH de 4,7 (Figure 24). Lorsque le pH est encore abaissé, le maximum d'émission se déplace de nouveau vers le bleu. Ce comportement ne peut pas être représenté par une simple transition entre deux états, et fournit la preuve de l'existence d'un intermédiaire, dont l'émission de fluorescence décalée vers le rouge est clairement distincte des deux états neutre et dénaturé à pH très acide. Cet intermédiaire « rouge » disparaît lorsque la mutation 65 S est introduite dans la ECFPr (Figure 25C). De même, dans le cas de la protéine ECFPr (72A, 145A, 148D), il existe un intermédiaire en dessous du $pK_{1/2}$, qui absorbe et émet dans le bleu (Figures 25B et 26B) ; cet état intermédiaire isomérisé (plus connu sous le nom d'isomère *cis-trans*) devient indétectable suite à l'introduction de la mutation 65S (Figures 25D et 26D). En effet, les spectres d'absorption et d'émission de fluorescence de la protéine ECFPr (65S,72A, 145A, 148D) présentent des mélanges de forme neutre et acide, sans aucune perturbation spectrale intermédiaire. La perte d'intensité de fluorescence et de la structure fine des spectres d'absorption et d'émission, ainsi que le déplacement des spectres vers le bleu ont lieu presque simultanément dans une gamme de pH très étroite.

### III.3. La mutation 65S réduit la vitesse et l'amplitude du photoblanchiment réversible des protéines fluorescentes cyans

Beaucoup de protéines fluorescentes naturelles ou génétiquement modifiées subissent une conversion réversible induite par la lumière entre des états optiquement distincts (aussi connues sous le nom de protéines à photocommutation réversible ou RSFP). Il est de plus en plus reconnu que de telles photoréactions réversibles sont, à des degrés divers, communes à la plupart des protéines fluorescentes, et que cela peut avoir des conséquences majeures pour leur utilisation dans l'imagerie biologique : ainsi, dans les applications standards de FRET, les fluorophores doivent être, autant que possible, dépourvus de telles photoréactions. Toutefois, les données quantitatives sur les propriétés de photoconversion réversible des protéines fluorescentes cyans et jaunes sont assez rares. Ces réactions sont en général plus faciles à observer sur des protéines fluorescentes purifiées et immobilisées, afin d'éliminer les interférences par diffusion brownienne ou liées aux mouvements de cellules vivantes exprimant ces protéines.

Les inventeurs ont ici pu constater que la protéine ECFPr purifiée liée à des billes d'agarose subit un photoblanchiment réversible transitoire et prononcé sous illumination dans la bande d'absorption de son chromophore. Ainsi, sous illumination brève en champ large et en utilisant la puissance maximale d'une lampe à mercure, l'intensité de fluorescence diminue de 23% suite une monoexponentielle de constante de temps de moins de une seconde (Figures 22 et 27). Suite à cette réponse transitoire, il y a une diminution plus lente de l'intensité de fluorescence d'environ 0,1% par seconde, très probablement due à un photoblanchiment irréversible (voir ci-dessous). Si le temps d'illumination est maintenu suffisamment court, et après plusieurs minutes dans l'obscurité complète, l'intensité de fluorescence revient à son niveau initial (Figure 27A).

Les inventeurs ont en outre observé que le recouvrement de la fluorescence de la ECFPr après le blanchiment transitoire est accéléré par une illumination modérée aux mêmes longueurs d'onde (Figure 28). Cela montre que le retour de la ECFPr à son état fluorescent est également activé par la lumière. En conséquence, le niveau stationnaire de fluorescence atteint au bout de quelques secondes d'éclairage doit correspondre à un régime d'état constant, où les deux photoréactions "*off*" (état non fluorescent) et "*on*" (état fluorescent) auront lieu à des vitesses équivalentes. Un modèle cinétique impliquant deux états minimum peut être utilisé pour décrire ce système : ce modèle est caractérisé par les deux constantes de vitesse $k_{off}$ et $k_{on}$, qui décrivent les réactions élémentaires de blanchiment réversible et de retour photoactivé, respectivement. Selon ce modèle, le temps de relaxation apparent $\tau_{rev}$ dans les expériences de blanchiment est la somme inverse des deux constantes de vitesse $(k_{on} + k_{off})^{-1}$, alors que le niveau stationnaire de l'intensité de fluorescence atteint au bout de quelques secondes est donnée par $k_{on} / (k_{on} + k_{off})$, à partir de laquelle les constantes de vitesse $k_{on}$ et $k_{off}$ peuvent être obtenues (Tableau 4). A partir de ces deux constantes de vitesse, on peut également estimer les rendements quantiques de photoconversion dans les deux directions : les valeurs obtenues, $\varphi_{off}$ = 1% et $\varphi_{on}$ = 6%, montrent que la ECFPr est une protéine à photocommutation réversible très efficace (RSFP).

Le blanchiment transitoire d'autres protéines fluorescentes cyans comprenant ou non la mutation 65S a été également étudié selon le même protocole expérimental. Sous illumination instantanée, toutes les protéines fluorescentes subissent une chute réversible de l'intensité de fluorescence sur des échelles de temps similaires de quelques secondes, mais avec des amplitudes très différentes (Figure 27). Ainsi, il est constaté que la protéine ECFPr (72A, 145A, 148D) affiche une diminution prononcée de 33%. De manière plus notable, la mutation 65S introduite dans ECFPr ou ECFPr (72A, 145A, 148D) réduit nettement l'amplitude de la diminution transitoire, à moins de 3% dans les deux cas (Figure 27B). En comparant les constantes de vitesse $k_{on}$ et $k_{off}$ de chacune de ces protéines (Tableau 4), on constate que les protéines comprenant la mutation 65S présentent une vitesse de blanchiment réversible $k_{off}$ 10 fois plus faible par rapport aux protéines dépourvues de cette mutation, avec seulement des changements modérés concernant le retour photoactivé, (Tableau 4). Par conséquent, l'effet majeur de la mutation 65S est de ralentir considérablement la vitesse élémentaire de blanchiment réversible $k_{off}$, ce qui conduit à une réduction de l'amplitude du blanchiment transitoire.

Les photoréactions des protéines fluorescentes cyans sont également observables dans des cellules MDCK exprimant ces protéines dans leur cytosol (Figure 29). Cependant, en utilisant des conditions d'illumination plein champ identiques, les amplitudes de réponses apparaissent sensiblement réduites par rapport à celles obtenues *in vitro,* et les temps de relaxation sont sensiblement plus courts (Tableau 4), cela pouvant provenir des conditions d'acquisition. Néanmoins, la comparaison des différentes protéines fluorescentes cyans révèle des tendances très similaires à celles observées *in vitro,* à savoir que la mutation 65S diminue fortement les réponses transitoires (Figure 29).

**Tableau 4 : Paramètres de blanchiment réversible des protéines fluorescentes cyans, comprenant ou non la mutation 65S, sur des billes d'agarose ou dans des cellules.**

| Protéines étudiées | Billes d'agarose | | | | Cellules vivantes | | |
|---|---|---|---|---|---|---|---|
| | % Rev ± 2% | $\tau_{Rev}$ (s) ± 0.1 | $k_{off}$ ($S^{-1}$) | $k_{on}$ ($s^{-1}$) | % Rev ± Std Dev | $\tau_{Rev}$ ± Std Dev (s) | N |
| ECFPr | 23.1 | 0.6 | 0.404 | 1.35 | 5±3 | 0.28±0.04 | 21 |
| ECFPr-65S | 3 | 1.0 | 0.029 | 0.94 | 0.8±0.4 | 0.6±0.3 | 15 |
| ECFPr (72A, 145A, 148D) | 33 | 1.0 | 0.337 | 0.68 | 14±2 | 0.6±0.1 | 21 |
| ECFPr (65S, 72A,145A,148D) | 2.5 | 0.8 | 0.033 | 1.30 | - | - | - |
| % Rev : pourcentage de perte de l'intensité de fluorescence initiale après une illumination instantanée de densité de puissance 0,2W/cm$^2$ ; $\tau_{Rev}$ : constante de temps du déclin initial de l'intensité de fluorescence ; $k_{off}$ : constante de vitesse de formation de l'état non fluorescent de la protéine ; $k_{on}$ : constante de vitesse de formation de l'état fluorescent de la protéine. | | | | | | | |

**III.4. La mutation 65S ralentit le photoblanchiment irréversible dans les protéines fluorescentes cyans**

Les réactions irréversibles de blanchiment des protéines fluorescentes cyans, comprenant ou non la mutation 65S, ont également été étudiées. Pour ce faire, des conditions identiques d'illumination plein champ mais prolongée ont été utilisées à la fois sur des billes d'agarose immobilisées et dans le cytosol de cellules. Toutes les protéines fluorescentes cyans étudiées présentent entre 85% et 95% de perte de fluorescence irréversible après 30 min d'illumination à la puissance maximale de la lampe à mercure. Le déclin de l'intensité de fluorescence est approximativement exponentiel (Figure 30), avec moins de 1% de retour de l'intensité dans l'obscurité et aucun retour photo-activé détectable, et ce jusqu'à 10 min après avoir stoppé l'illumination. Les expériences menées sur des billes d'agarose immobilisées et sur les cellules ont donné des constantes de temps de blanchiment irréversibles assez semblables (Figure 22): ainsi, dans tous les cas, la seule mutation 65S ralentit notablement le blanchiment irréversible des protéines ECFPr et ECFPr (72A, 145A, 148D) (Tableau 1). La protéine ECFPr (72A, 145A, 148D) subit un blanchiment un peu plus rapide que la ECFPr, montrant ainsi que cette protéine a une photostabilité diminuée à la fois en termes de réactions réversibles et irréversibles.

La mutation 65S a donc pour effet d'améliorer la photostabilité des protéines fluorescentes cyans en réponse à une irradiation.

## Conclusion

Les inventeurs ont ainsi pu constater l'importance de l'influence de la mutation 65S sur les propriétés photophysiques des protéines fluorescentes cyans. Cette mutation spécifique permet, outre le fait de réduire la sensibilité au pH de ces protéines, d'améliorer leur rendement quantique, de diminuer la complexité de leur cinétique de fluorescence, mais aussi d'inhiber les photoréactions réversibles et de ralentir le photoblanchiment irréversible.

Ainsi, des protéines fluorescentes cyans comprenant la mutation 65S pourront plus particulièrement être utilisées dans des études d'imagerie par transfert d'énergie résonant de type Förster (FRET) ou en microscopie de durées de vie de fluorescence (FLIM) dans des cellules vivantes, car elles permettront une analyse quantitative plus précise et plus sensible, et par conséquent plus fiable, des signaux de fluorescence.

## BIBLIOGRAPHIE

Prasher D.C. et al. (1992). Gene, 111: 229-33.

Day R.N., Davidson M.W. (2009). Chemical society reviews, 38: 2887-2921.

Cubitt A.B., Heim R., Adams S.R., Boyd A.E., Gross L.A., Tsien R.Y.(1995). Trends Biochem Sci., 20(11): 448-55.

Llopis J., McCaffery J.M., Miyawaki A., Farquhar M.G., Tsien R.Y.(1998). Proc Natl Acad Sci USA, 95(12): 6803-8.

Cubitt A.B., Woollenweber L.A., Heim R. (1999). Methods Cell Biol., 58:19-30. Miyawaki A., Griesbeck O., Heim R., Tsien, R.Y. (1999). Proc Natl Acad Sci USA, 96(5):2135-40.

Shaner N.C., Patterson G.H., Davidson M.W. (2007). J. Cell. Sci., 120: 4247-4260. Patterson G.H., Day R.N., Piston D. (2001). J. Cell Sci., 114: 837-838.

Tramier M., Gautier I., Piolot T., Ravalet S., Kemnitz K., Coppey J., Durieux C., Mignotte V., Coppey-Moisan M. (2002). Biophys J., 83(6):3570-7.

Miyawaki A., Tsien R. Y. (2000). Methods Enzymol., 327: 472-499.

Rizzo M.A., Springer G.H., Granada B., Piston D.W. (2004). Nat. Biotechnol., 22(4): 445-449, 2004.

Malo G.D., Pouwels L.J., Wang MT., Weichsel A., Montfort W.R., Rizzo M.A., Piston, D.W., Wachter R.M. (2007). Biochemistry, 46(35): 9865-9873.

Nguyen A.W., Daugerthy P.S. (2005). Nature Biotechnology, 23(3): 355-360.

Shaner N.C., Steinbach P.A., Tsien R.Y. (2005). Nat. Methods, 2: 905-909.

Goedhart J., van Weeren L., Hink M.A., Vischer N.O., Jalink K., Gadella T.W. Jr. (2010). Nat. Methods, 7(2):137-9.

Sawano A., Miyawaki A. (2000). Nucleic Acids Res., 28(16): E78.

Espagne A., Erard M., Madiona K., Derrien V., Jonasson G., Lévy B., Pasquier H., Melki R., et Mérola F. (2011). Biochemistry, 50(4): 437-9.

Valeur, B. (2006). Molecular Fluorescence : Principles and Applications., 3ème éd., Wiley-VCH.

O'Connor, D.V., et Philipps, D. (1984). Time-correlated single photon counting, Academic Press, London.

Needleman S. B., et Wunsch C. D. (1970). J. Mol. Biol., 48: 443-453.

Griesbeck O., Baird G.S., Campbell R.E., Zacharias D.A., Tsien R.Y. (2001). The journal of biological chemistry, 276 (31): 29188-29194.

Nagai T., Ibata K., Park E.S., Kubota M., Mikoshiba K, Miyawaki, A. (2002). Nature Biotechnology, 20: 87-90.

Sambrook et al. (2001). Molecular Cloning: A Laboratory Manual, 3ème édition, Cold Spring Harbor Laboratory Press.

Ausubel et al. (2011). Current Protocols in Molecular Biology, John Wiley & Sons. Merzlyak E.M., Goedhart J., Shcherbo D., Bulina M.E., Shcheglov A.S., Fradkov A.F., Gaintzeva A., Lukyanov K.A., Lukyanov S., Gadella T.W., Chudakov D.M. (2007). Nat Methods ; 4(7): 555-7.

Zhang J., Ma Y., Taylor S.S., Tsien R.Y. (2001). Proc Natl Acad Sci USA; 98:14997-15002.
Zhang J., Hupfeld C.J., Taylor S.S., Olefsky J.M., Tsien R.Y. (2005). Nature, 437 (7058): 569-573.
Dunn T.A., Wang C.T., Colicos M.A., Zaccolo M., DiPilato L.M., Zhang J., Tsien R.Y., and Feller M.B. (2006). The Journal of Neuroscience; 26(49):12807-12815.
Morris M.C. (2010). Cell Biochem Biophys., 56:19-37.
Frommer W. B., Davidson M. W., et Campbell R. E. (2009). Chem Soc Rev; 38: 2833-41.
Heyduk (2002). Curr Opin Biotechnol., 13 (4): 292-6
Truong et al. (2001). Curr Opin Struct Biol, 11(5) :573-8.
Issad et al. (2003). Diabetes Metab., 29 (2 Pt 1) :111-7.
Boute et al. (2002). Pharmacol Sci., 23 (8) :351-4.Trugnan G., Fontanges P., Delautier D., Ait-Slimane T. (2004). Medecine/Science, 20: 1027-34.
Kumar S., Alibhai D., Margineanu A., Laine R., Kennedy G., et al. (2011). Chem Phys Chem., 12: 209-626.Mérola F., Rigler R., Holmgren A., Brochon J.C. (1989). Biochemistry ; 28: 3383-3398.
Couprie M.E., Mérola F., Tauc P., Garzella D., Delboulbé A., Hara, T., Billardon, M. (1994). Review of Scientific Instruments ; 65: 1485-1495.
Giuliani A., Jamme F., Rouam V., Wien F., Giorgetta J.L., Lagarde B., Chubar O., Bac S., Yao L, Rey S., Herbeaux C., Marlats J.-L., Zerbib D., Polack F. et Réfrégiers M. (2009). J Synchrotron Radiat; 16(6): 835-841.
Wien F., Wallace B.A. (2005). Appl Spectrosc; 59: 1109-1113.
Lees J.G., Smith B.R., Wien F., Miles A.J., Wallace B.A. (2004). Anal Biochem; 332: 285-289.
Kelly S.M., Jess T.J., Price N.C. (2005). Biochim Biophys Acta; 1751: 119-139. Whitmore L., Wallace B.A. (2004). Nucleic Acids Res; 32: W668-673.
Lees J.G., Miles A.J., Wien F., Wallace B.A. (2006). Bioinformatics; 22: 1955-1962.

## Revendications

1. Méthode pour réduire la sensibilité au pH de protéines fluorescentes cyans, comprenant :

   - une étape a) selon laquelle une mutation est introduite en position 65 et/ou 148 de la séquence SEQ ID N°2 dans une séquence protéique comprenant ladite séquence SEQ ID N°2,

   lesdites protéines fluorescentes cyans générées par ladite méthode présentant une sensibilité réduite au pH.

2. Méthode selon la revendication 1, **caractérisée en ce que** l'acide aminé en position 65 est substitué par une sérine.

3. Méthode selon la revendication 1 ou 2, **caractérisée en ce que** l'acide aminé en position 148 est substitué par une glycine, une alanine, une sérine, ou un acide glutamique.

4. Méthode selon l'une quelconque des revendications 1 à 3, **caractérisée en ce qu'**elle comprend une étape supplémentaire, selon laquelle au moins une autre mutation sélectionnée parmi le groupe 9G, 11I, 19E, 26R, 68L, 72A, 87V, 145A, 164H, 167A, 172T, 175G, 1941, et 206K est introduite dans la séquence SEQ ID N°2, ladite étape pouvant être préalable ou postérieure à l'étape a).

5. Protéine fluorescente cyan susceptible d'être obtenue par la méthode selon l'une quelconque des revendications 1 à 4.

6. Acide nucléique codant pour la protéine fluorescente cyan selon la revendication 5.

7. Vecteur recombinant comprenant l'acide nucléique selon la revendication 6.

8. Cellule hôte exprimant la protéine fluorescente cyan selon la revendication 5.

9. Biosenseur, **caractérisé en ce que** ledit biosenseur comprend la protéine fluorescente cyan selon la revendication 5.

10. Biosenseur selon la revendication 9, **caractérisé en ce que** la séquence de ladite protéine fluorescente cyan comprend au moins les mutations 65S et 148G.

11. Biosenseur selon la revendication 9 ou 10, **caractérisé en ce qu'**il comprend en outre une protéine fluorescente dont le spectre d'absorption se recouvre partiellement avec le spectre d'émission de la protéine selon la revendication 5.

12. Biosenseur selon la revendication 11, **caractérisé en ce que** ladite protéine fluorescente dont le spectre d'absorption se recouvre partiellement avec le spectre d'émission de la protéine selon la revendication 5 est sélectionnée parmi les protéines fluorescentes YFP, Topaz, EYFP, YPET, SYFP2, Citrine, Venus, cp-Venus, Kusabira Orange, Kusabira Orange2, mOrange, mOrange2, dTomato, dTomato-Tandem, DsRed et ses variants tels que DsRed2, DsRed-Express (T1), DsRed-Express2, DsRed-Max, et DsRed-Monomer, ainsi que TagRFP, TagRFP-T, mRuby, mApple, mStrawberry, AsRed2, mRFP1, JRed, mCherry, eqFP611, tdRFP611, HcRed1, mRaspberry, tdRFP639, mKate, mKate2, Katushka, tdKatushka, HcRed-Tandem, mPlum et AQ143, de préférence parmi les protéines fluorescentes Citrine, Venus, cp-Venus, TagRFP et TagRFP-T.

13. Biosenseur selon la revendication 12, **caractérisé en ce que** ladite protéine fluorescente dont le spectre d'absorption se recouvre partiellement avec le spectre d'émission de la protéine selon la revendication 5 est la TagRFP.

14. Biosenseur selon l'une quelconque des revendications 9 à 13, **caractérisé en ce que** ladite protéine fluorescente protéine cyan est liée directement au biosenseur.

15. Biosenseur selon l'une quelconque des revendications 9 à 13, **caractérisé en ce que** ladite protéine fluorescente est liée indirectement au biosenseur.

16. Utilisation de la protéine fluorescente cyan selon la revendication 5, de l'acide nucléique selon la revendication 6, du vecteur recombinant selon la revendication 7, de la cellule hôte selon la revendication 8, du biosenseur selon l'une quelconque des revendications 9 à 15, dans des applications d'imagerie quantitative sélectionnées parmi la spectroscopie de corrélation de fluorescence (FCS) et les variations de cette méthode, le transfert d'énergie par résonance de type Fôrster (FRET), le transfert d'énergie bioluminescente par résonance (BRET), la microscopie d'imagerie de la durée de vie de fluorescence (FLIM), la redistribution de fluorescence après photoblanchiment (FRAP), la perte de fluorescence induite par photoblanchiment (FLIP), le comptage monophotonique corrélé au temps (TCSPC), l'anisotropie et la dépolarisation de fluorescence, la microscopie de localisation par photoactivation (PALM), la microscopie de reconstruction optique stochastique (STORM), la microscopie de déplétion par émission stimulée (STED).

**17.** Utilisation de la protéine fluorescente cyan selon la revendication 5, de l'acide nucléique selon la revendication 6, du vecteur recombinant selon la revendication 7, de la cellule hôte selon la revendication 8, du biosenseur selon l'une quelconque des revendications 9 à 15, dans des méthodes de criblage de composés chimiques et/ou de cellules.

**18.** Utilisation de la protéine fluorescente cyan selon la revendication 5, de l'acide nucléique selon la revendication 6, du vecteur recombinant selon la revendication 7, de la cellule hôte selon la revendication 8, du biosenseur selon l'une quelconque des revendications 9 à 15, dans des tests de toxicologie, de génotoxicité ou de détection de pollution environnementale.

**Patentansprüche**

**1.** Verfahren zur Reduzierung der Empfindlichkeit gegenüber dem pH-Wert von cyan fluoreszierenden Proteinen, umfassend:

- einen Schritt a), gemäß dem eine Mutation an Position 65 und/oder 148 der Sequenz SEQ ID Nr. 2 in eine Proteinsequenz, umfassend die Sequenz SEQ ID Nr. 2, eingeführt wird,

wobei die cyan fluoreszierenden Proteine, die durch das Verfahren generiert werden, eine reduzierte Empfindlichkeit gegenüber dem pH-Wert aufweisen.

**2.** Verfahren nach Anspruch 1, **dadurch gekennzeichnet, dass** die Aminosäure an Position 65 durch ein Serin substituiert wird.

**3.** Verfahren nach Anspruch 1 oder 2, **dadurch gekennzeichnet, dass** die Aminosäure an Position 148 durch ein Glycin, ein Alanin, ein Serin oder eine Glutaminsäure substituiert wird.

**4.** Verfahren nach einem der Ansprüche 1 bis 3, **dadurch gekennzeichnet, dass** es einen zusätzlichen Schritt umfasst, gemäß dem mindestens eine weitere Mutation, ausgewählt aus der Gruppe 9G, 11I, 19E, 26R, 68L, 72A, 87V, 145A, 164H, 167A, 172T, 175G, 1941 und 206K in die Sequenz SEQ ID Nr. 2 eingeführt wird, wobei der Schritt vor oder nach Schritt a) durchgeführt werden kann.

**5.** Cyan fluoreszierendes Protein, das durch das Verfahren nach einem der Ansprüche 1 bis 4 erhalten werden kann.

**6.** Nukleinsäure, die für das cyan fluoreszierende Protein codiert, nach Anspruch 5.

**7.** Rekombinanter Vektor, umfassend die Nukleinsäure nach Anspruch 6.

**8.** Wirtszelle, die das cyan fluoreszierende Protein exprimiert, nach Anspruch 5.

**9.** Biosensor, **dadurch gekennzeichnet, dass** der Biosensor das cyan fluoreszierende Protein nach Anspruch 5 umfasst.

**10.** Biosensor nach Anspruch 9, **dadurch gekennzeichnet, dass** die Sequenz des cyan fluoreszierenden Proteins mindestens die Mutationen 65S und 148G umfasst.

**11.** Biosensor nach Anspruch 9 oder 10, **dadurch gekennzeichnet, dass** er außerdem ein fluoreszierendes Protein umfasst, dessen Absorptionsspektrum sich teilweise mit dem Emissionsspektrum des Proteins nach Anspruch 5 überschneidet.

**12.** Biosensor nach Anspruch 11, **dadurch gekennzeichnet, dass** das fluoreszierende Protein, dessen Absorptionsspektrum sich teilweise mit dem Emissionsspektrum des Proteins nach Anspruch 5 überschneidet, ausgewählt ist aus den YFP, Topaz, EYFP, YPET, SYFP2, Citrin, Venus, cp-Venus, Kusabira Orange, Kusabira Orange2, mOrange, mOrange2, dTomato, dTomato-Tandem, DsRed und seinen Varianten wie z. B. DsRed2, DsRed-Express (T1), DsRed-Express2, DsRed-Max, und DsRed-Monomer sowie TagRFP, TagRFP-T, mRuby, mApple, mStrawberry, AsRed2, mRFP1, JRed, mCherry, eqFP611, tdRFP611, HcRed1, mRaspberry, tdRFP639, mKate, mKate2, Katushka, tdKatushka, HcRed-Tandem, mPlum und AQ143 fluoreszierenden Proteinen, vorzugweise aus den Citrin, Venus, cp-Venus, TagRFP und TagRFP-T fluoreszierenden Proteinen.

**13.** Biosensor nach Anspruch 12, **dadurch gekennzeichnet, dass** das fluoreszierende Protein, dessen Absorptionsspektrum teilweise mit dem Emissionsspektrum des Proteins nach Anspruch 5 überlappt, TagRFP ist.

**14.** Biosensor nach einem der Ansprüche 9 bis 13, **dadurch gekennzeichnet, dass** das cyan fluoreszierende Protein direkt mit dem Biosensor verbunden ist.

**15.** Biosensor nach einem der Ansprüche 9 bis 13, **dadurch gekennzeichnet, dass** das fluoreszierende Protein indirekt mit dem Biosensor verbunden ist.

**16.** Verwendung des cyan fluoreszierenden Proteins nach Anspruch 5, der Nukleinsäure nach Anspruch 6, des rekombinanten Vektors nach Anspruch 7, der Wirtszelle nach Anspruch 8, des Biosensors nach einem der Ansprüche 9 bis 15, in Anwendungen der quantitativen Bildgebung, ausgewählt aus der Fluoreszenzkorrelationsspektroskopie (FCS) und den Variationen dieser Verfahrens, dem Förster-Resonanzenergietransfer (FRET), dem Biolumineszenz-Resonanzenergietransfer (BRET), der Fluoreszenzlebensdauer-Mikroskopie (FLIM), der Fluorescence Recovery after Photobleaching (FRAP), der Fluorescence Loss in Photobleaching (FLIP), der zeitkorrelierten Einzelphotonenzählung (TCSPC), der Anisotropie und der Fluoreszenzdepolarisation, der photoaktivierten Lokalisationsmikroskopie (PALM), der stochastischen optischen Rekonstruktionsmikroskopie (STORM), der stimulierten Emissions-Depletions-Mikroskopie (STED).

**17.** Verwendung des cyan fluoreszierenden Proteins nach Anspruch 5, der Nukleinsäure nach Anspruch 6, des rekombinanten Vektors nach Anspruch 7, der Wirtszelle nach Anspruch 8, des Biosensors nach einem der Ansprüche 9 bis 15, in Verfahren zum Screening von chemischen Verbindungen und/oder Zellen.

**18.** Verwendung des cyan fluoreszierenden Proteins nach Anspruch 5, der Nukleinsäure nach Anspruch 6, des rekombinanten Vektors nach Anspruch 7, der Wirtszelle nach Anspruch 8, des Biosensors nach einem der Ansprüche 9 bis 15, in Tests der Toxikologie, der Gentoxizität oder des Nachweises der Umweltverschmutzung.

## Claims

**1.** Method for reducing the pH sensitivity of cyan fluorescent proteins, comprising:

- a step a) wherein a mutation is introduced at position 65 and/or 148 of the sequence SEQ ID No. 2 in a protein sequence comprising said sequence SEQ ID No. 2,

said cyan fluorescent proteins generated by said method having reduced pH sensitivity.

**2.** Method according to claim 1, **characterised in that** the amino acid at position 65 is substituted by serine.

**3.** Method according to claim 1 or 2, **characterised in that** the amino acid at position 148 is substituted by glycine, alanine, serine, or glutamic acid.

**4.** Method according to any one of claims 1 to 3, **characterised in that** it comprises an additional step, wherein at least one other mutation selected from the group 9G, 11I, 19E, 26R, 68L, 72A, 87V, 145A, 164H, 167A, 172T, 175G, 1941, and 206K is inserted into the sequence SEQ ID No. 2, said step being before or after step a).

**5.** Cyan fluorescent protein obtainable by the method according to any one of claims 1 to 4.

**6.** Nucleic acid encoding the cyan fluorescent protein according to claim 5.

7. Recombinant vector comprising the nucleic acid according to claim 6.

8. Host cell expressing the cyan fluorescent protein according to claim 5.

9. Biosensor, **characterised in that** said biosensor comprises the cyan fluorescent protein according to claim 5.

10. Biosensor according to claim 9, **characterised in that** the sequence of said cyan fluorescent protein comprises at least the mutations 65S and 148G.

11. Biosensor according to claim 9 or 10, **characterised in that** it further comprises a fluorescent protein of which the absorption spectrum partially overlaps with the emission spectrum of the protein according to claim 5.

12. Biosensor according to claim 11, **characterised in that** said fluorescent protein of which the absorption spectrum partially overlaps with the emission spectrum of the protein according to claim 5 is selected from the fluorescent proteins YFP, Topaz, EYFP, YPET, SYFP2, Citrine, Venus, cp-Venus, Kusabira Orange, Kusabira Orange2, mOrange, mOrange2, dTomato, dTomato-Tandem, DsRed and variants thereof such as DsRed2, DsRed-Express (T1), DsRed-Express2, DsRed-Max, and DsRed-Monomer, as well as TagRFP, TagRFP-T, mRuby, mApple, mStrawberry, AsRed2, mRFP1, JRed, mCherry, eqFP611, tdRFP611, HcRed1, mRaspberry, tdRFP639, mKate, mKate2, Katushka, tdKatushka, HcRed-Tandem, mPlum and AQ143, preferably from the fluorescent proteins Citrine, Venus, cp-Venus, TagRFP and TagRFP-T.

13. Biosensor according to claim 12, **characterised in that** said fluorescent protein of which the absorption spectrum partially overlaps with the emission spectrum of the protein according to claim 5 is TagRFP.

14. Biosensor according to any one of claims 9 to 13, **characterised in that** said cyan fluorescent protein is directly linked to the biosensor.

15. Biosensor according to any one of claims 9 to 13, **characterised in that** said fluorescent protein is indirectly linked to the biosensor.

16. Use of the cyan fluorescent protein according to claim 5, of the nucleic acid according to claim 6, of the recombinant vector according to claim 7, of the host cell according to claim 8, of the biosensor according to any one of claims 9 to 15, in quantitative imaging applications selected from fluorescence correlation spectroscopy (FCS) and variations of this method, fluorescence resonance energy transfer (FRET), bioluminescence resonance energy transfer (BRET), fluorescence-lifetime imaging microscopy (FLIM), fluorescence recovery after photobleaching (FRAP), fluorescence loss induced by photobleaching (FLIP), time-correlated single-photon counting (TCSPC), anisotropy and fluorescence depolarization, photoactivated localization microscopy (PALM), stochastic optical reconstruction microscopy (STORM), stimulated emission depletion microscopy (STED).

17. Use of the cyan fluorescent protein according to claim 5, of the nucleic acid according to claim 6, of the recombinant vector according to claim 7, of the host cell according to claim 8, of the biosensor according to any one of claims 9 to 15, in methods of screening chemical compounds and/or cells.

18. Use of the cyan fluorescent protein according to claim 5, of the nucleic acid according to claim 6, of the recombinant vector according to claim 7, of the host cell according to claim 8, of the biosensor according to any one of claims 9 to 15, in toxicology tests, genotoxicity tests or environmental pollution detection tests.

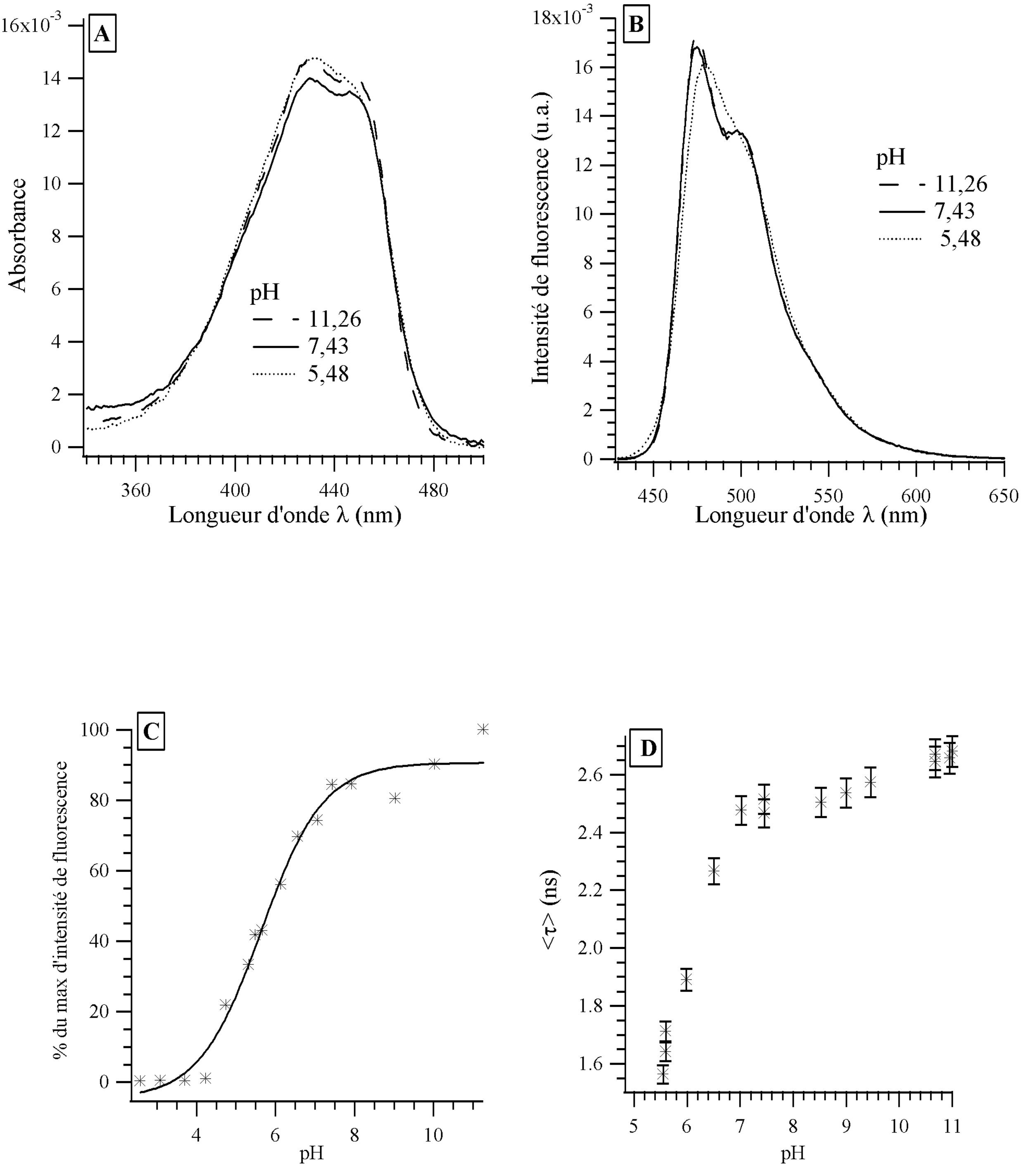
A
16x10-3
14
12
10
8
6
4
2
0
Absorbance
360
400
440
480
Longueur d'onde λ (nm)
pH
- 11,26
7,43
5,48
B
18x10-3
16
14
12
10
8
6
4
2
0
Intensité de fluorescence (u.a.)
450
500
550
600
650
Longueur d'onde λ (nm)
pH
- 11,26
7,43
5,48
C
100
80
60
40
20
0
% du max d'intensité de fluorescence
4
6
8
10
pH
D
2.6
2.4
2.2
2.0
1.8
1.6
<τ> (ns)
5
6
7
8
9
10
11
pH

**Figure 1**

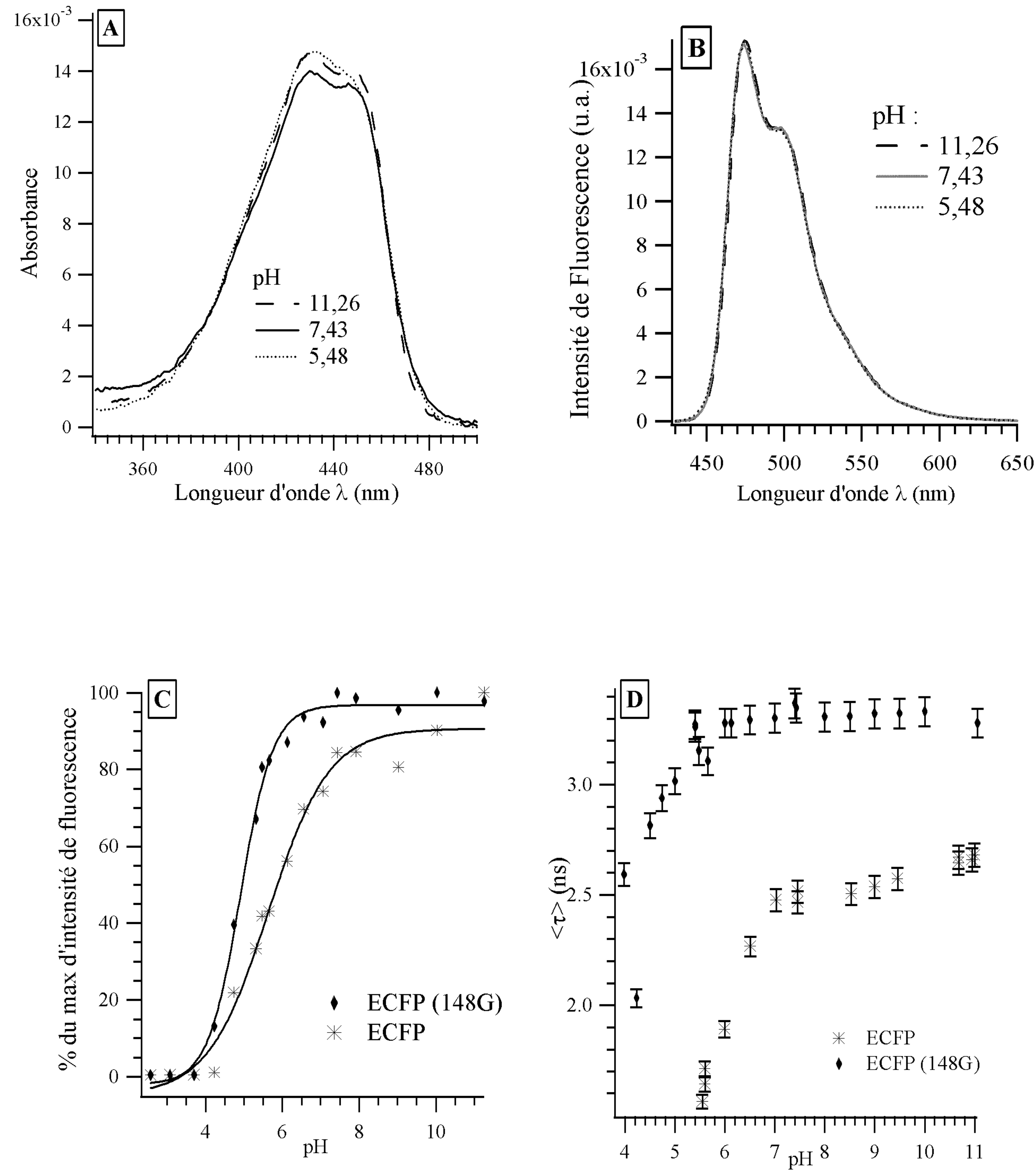
A
16x10-3
14
12
10
8
6
4
2
0
Absorbance
360
400
440
480
Longueur d'onde λ (nm)
pH
- 11,26
7,43
5,48
B
16x10-3
Intensité de Fluorescence (u.a.)
pH :
- 11,26
7,43
5,48
450
500
550
600
650
Longueur d'onde λ (nm)
C
100
80
60
40
20
0
% du max d'intensité de fluorescence
4
6
8
10
pH
ECFP (148G)
ECFP
D
3.0
2.5
2.0
<τ> (ns)
4
5
6
7
8
9
10
11
pH
ECFP
ECFP (148G)

**Figure 2**

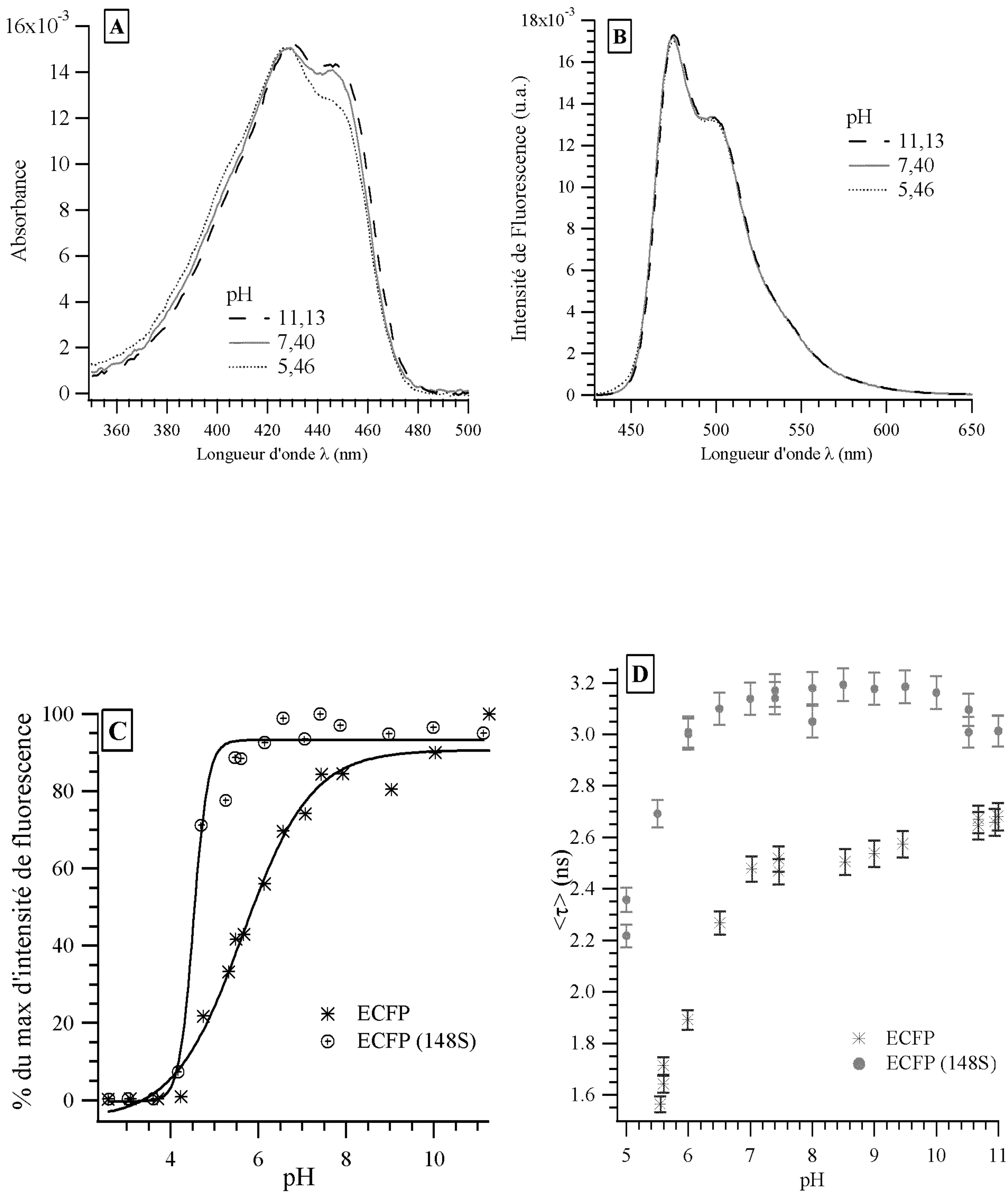
A
16x10^-3
Absorbance
pH
11,13
7,40
5,46
Longueur d'onde λ (nm)
B
18x10^-3
Intensité de Fluorescence (u.a.)
pH
11,13
7,40
5,46
Longueur d'onde λ (nm)
C
% du max d'intensité de fluorescence
ECFP
ECFP (148S)
pH
D
<τ> (ns)
ECFP
ECFP (148S)
pH

**Figure 3**

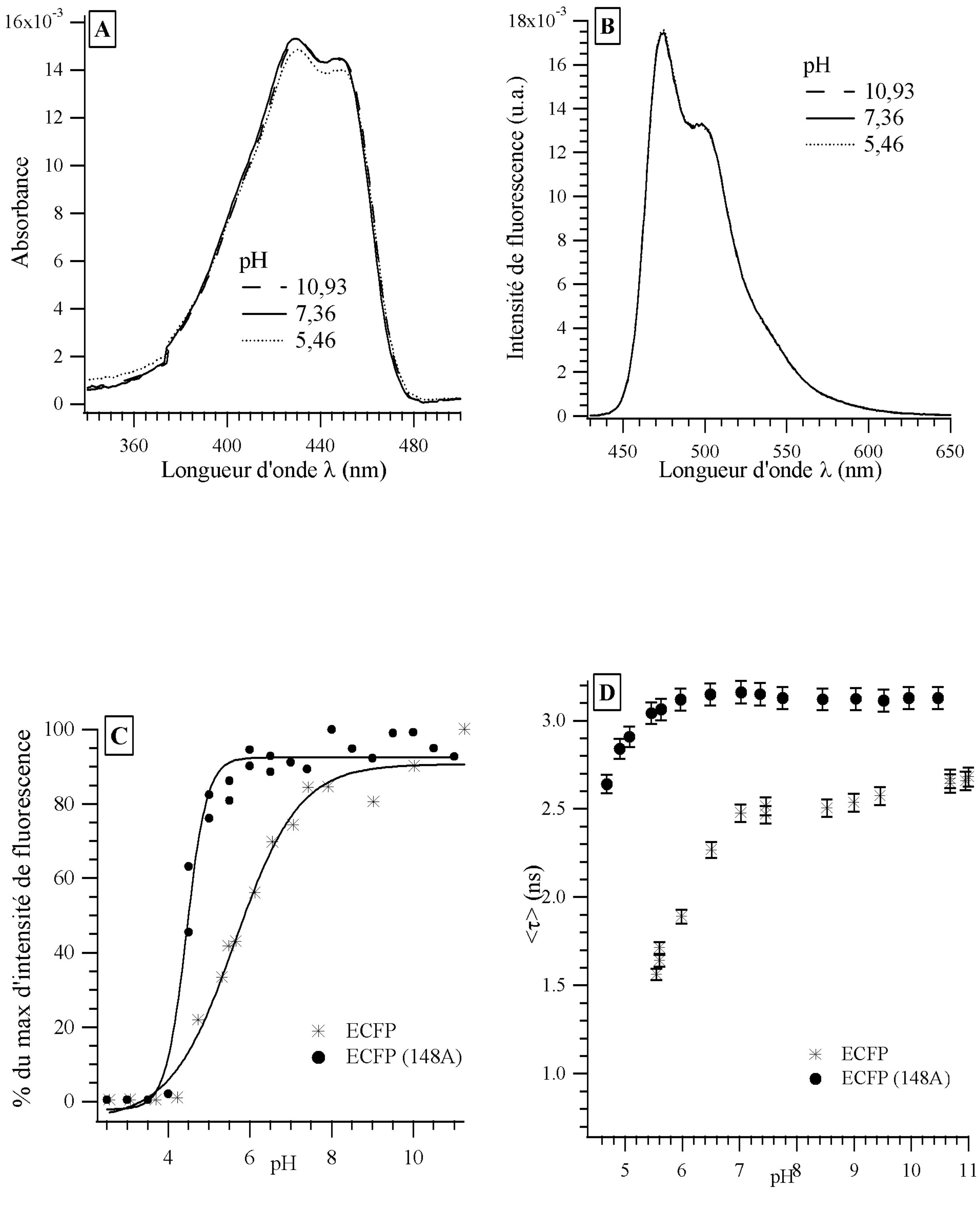
A
Absorbance
16x10^-3
Longueur d'onde λ (nm)
pH
10,93
7,36
5,46
B
Intensité de fluorescence (u.a.)
18x10^-3
Longueur d'onde λ (nm)
pH
10,93
7,36
5,46
C
% du max d'intensité de fluorescence
pH
ECFP
ECFP (148A)
D
<τ> (ns)
pH
ECFP
ECFP (148A)

**Figure 4**

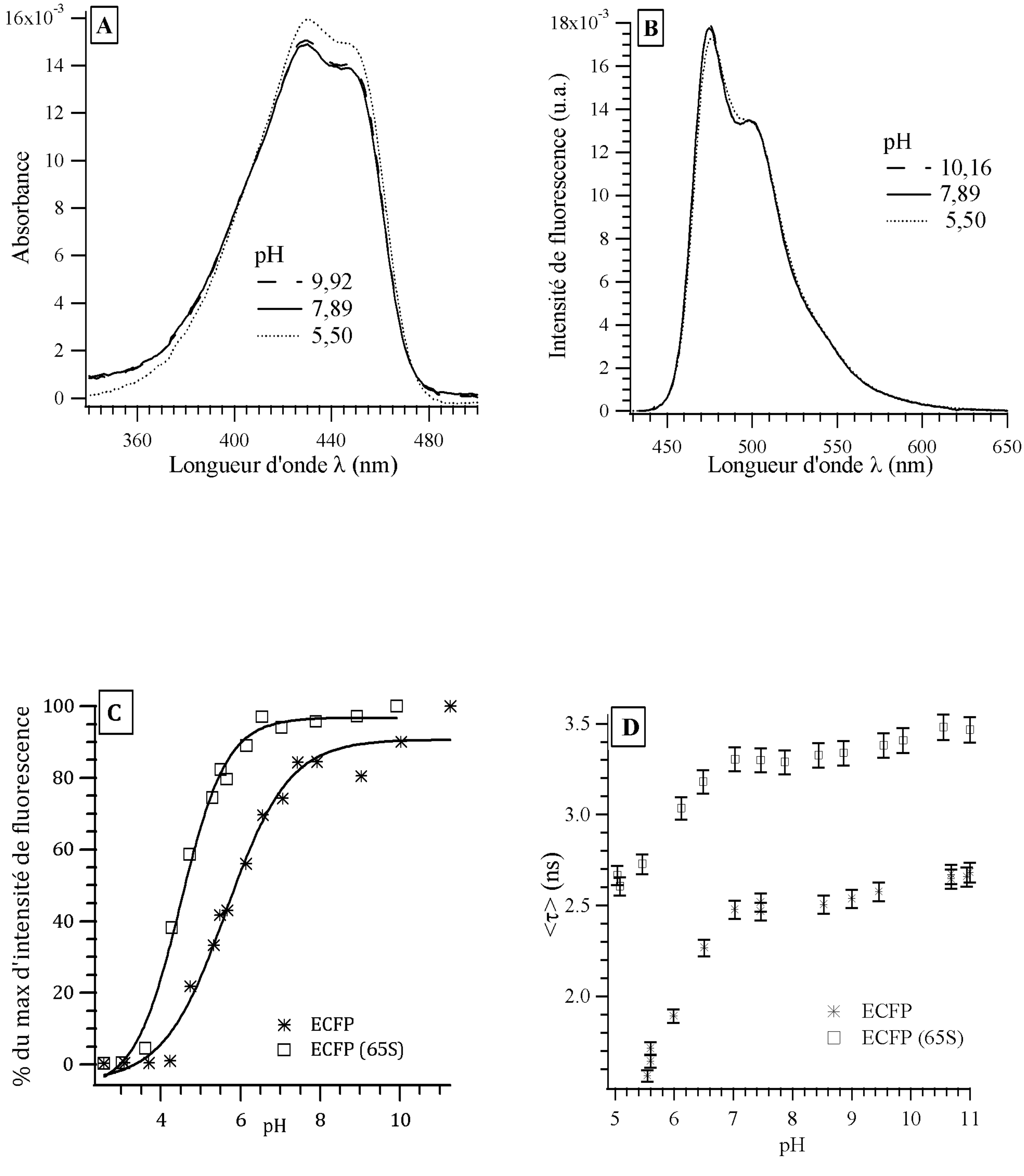
A
16x10-3
14
12
10
8
6
4
2
0
Absorbance
pH
- 9,92
7,89
5,50
360
400
440
480
Longueur d'onde λ (nm)
B
18x10-3
16
14
12
10
8
6
4
2
0
Intensité de fluorescence (u.a.)
pH
- 10,16
7,89
5,50
450
500
550
600
650
Longueur d'onde λ (nm)
C
100
80
60
40
20
0
% du max d'intensité de fluorescence
ECFP
ECFP (65S)
4
6
8
10
pH
D
3.5
3.0
2.5
2.0
<τ> (ns)
ECFP
ECFP (65S)
5
6
7
8
9
10
11
pH

**Figure 5**

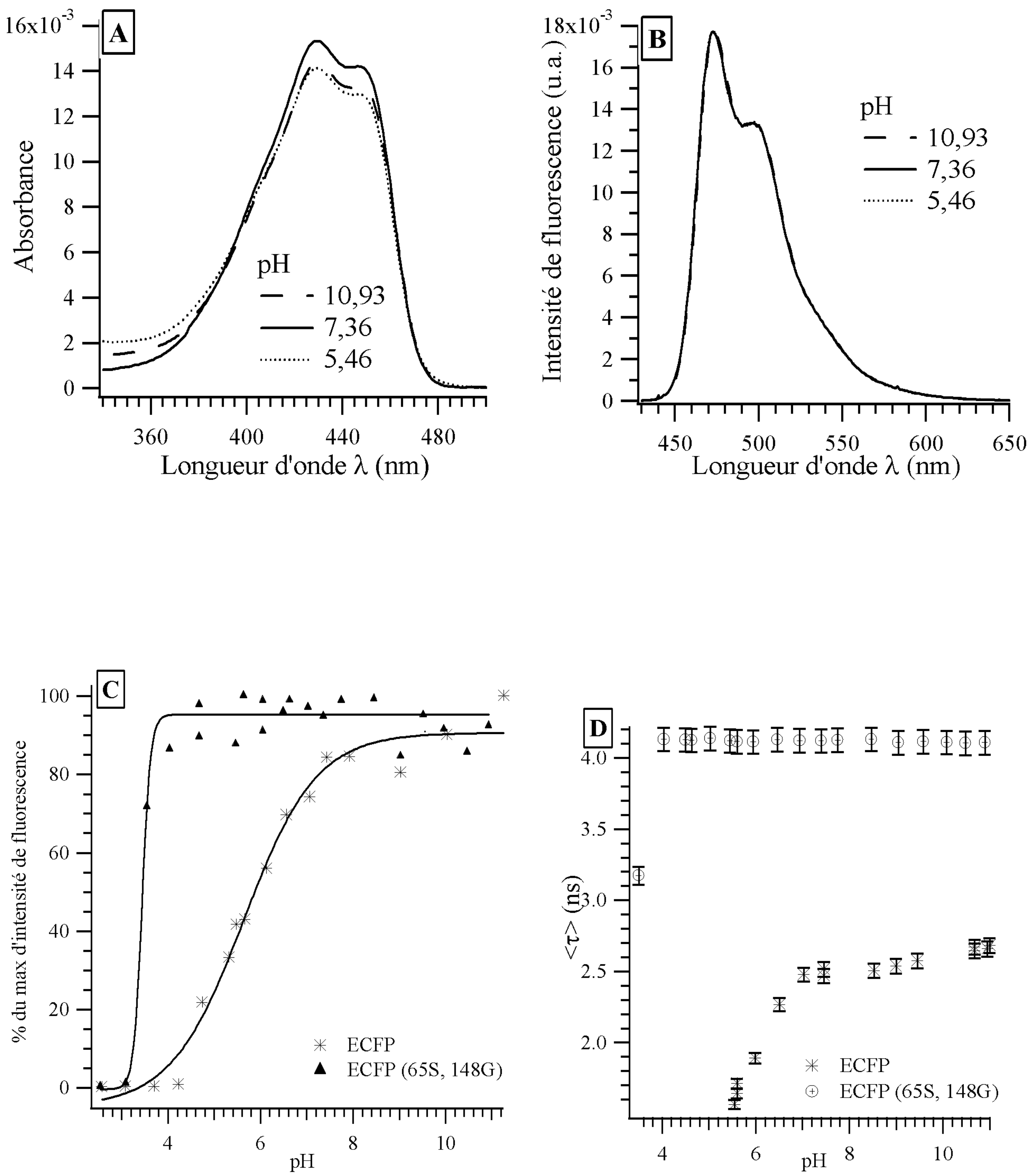
A
16x10-3
14
12
10
8
6
4
2
0
Absorbance
360
400
440
480
Longueur d'onde λ (nm)
pH
10,93
7,36
5,46
B
18x10-3
16
14
12
10
8
6
4
2
0
Intensité de fluorescence (u.a.)
450
500
550
600
650
Longueur d'onde λ (nm)
pH
10,93
7,36
5,46
C
100
80
60
40
20
0
% du max d'intensité de fluorescence
4
6
8
10
pH
ECFP
ECFP (65S, 148G)
D
4.0
3.5
3.0
2.5
2.0
<τ> (ns)
4
6
8
10
pH
ECFP
ECFP (65S, 148G)

**Figure 6**

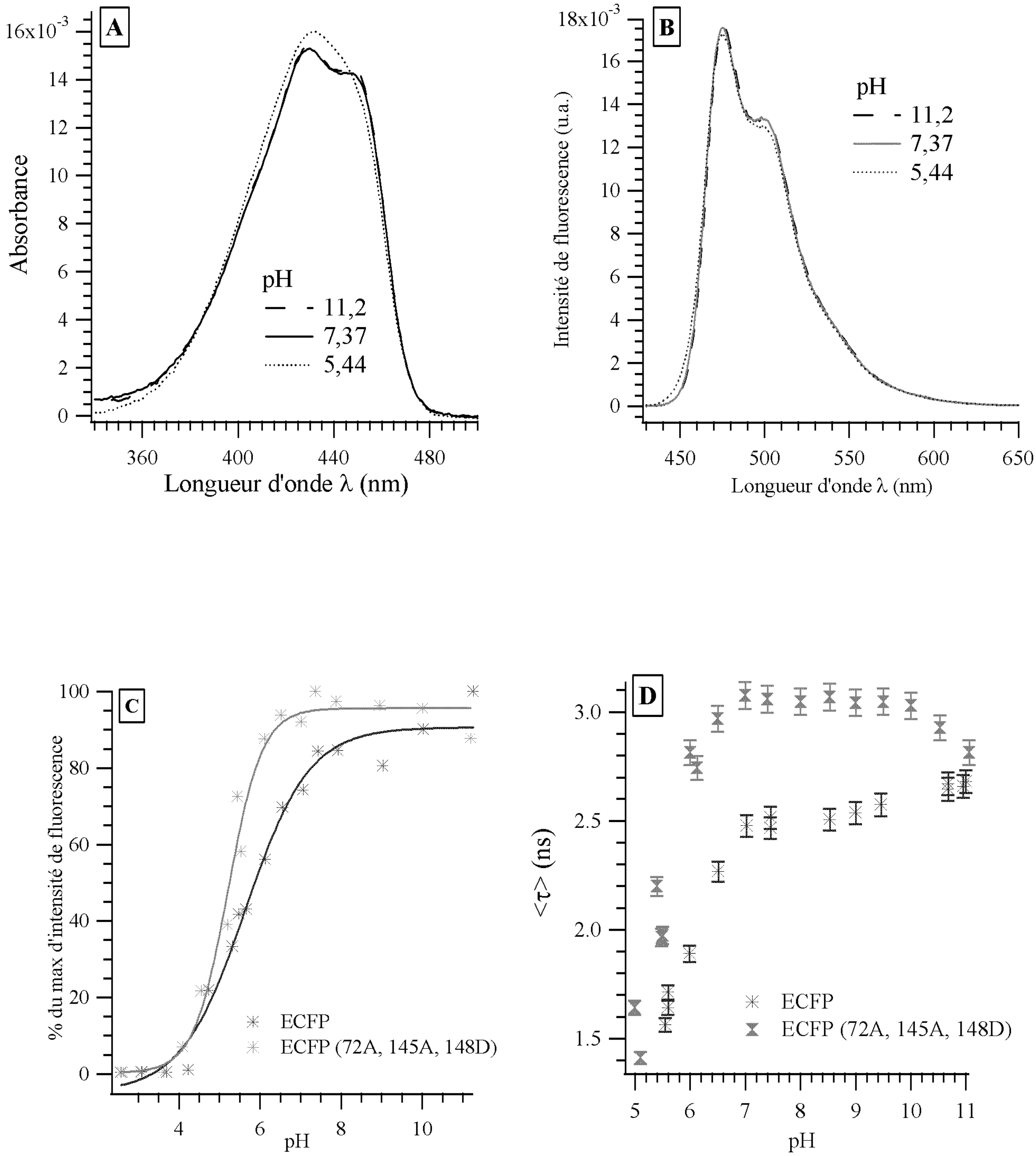
A
16x10-3
Absorbance
pH
- 11,2
7,37
5,44
Longueur d'onde λ (nm)
B
18x10-3
Intensité de fluorescence (u.a.)
pH
- 11,2
7,37
5,44
Longueur d'onde λ (nm)
C
% du max d'intensité de fluorescence
ECFP
ECFP (72A, 145A, 148D)
pH
D
<τ> (ns)
ECFP
ECFP (72A, 145A, 148D)
pH

**Figure 7**

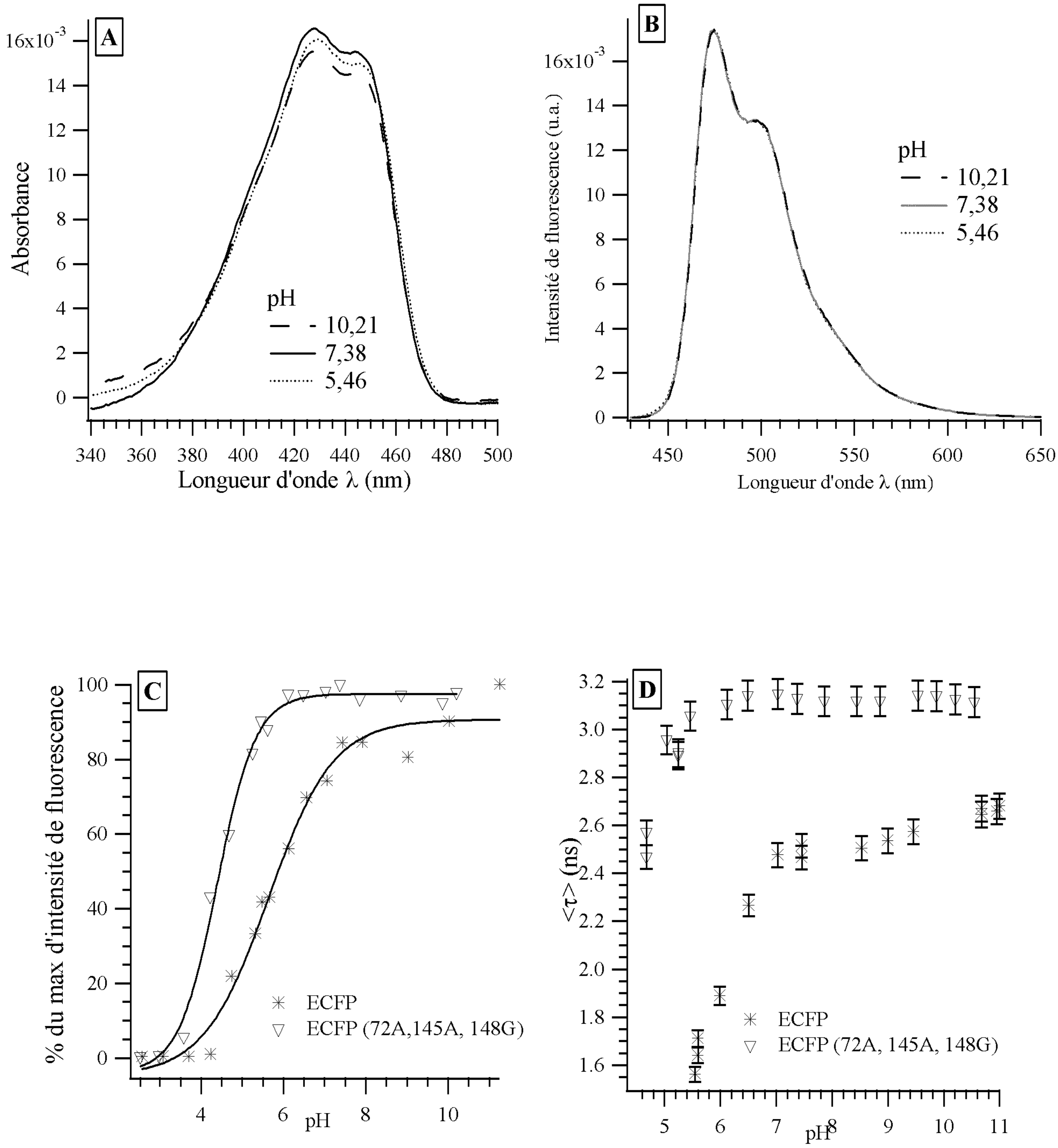
A
16x10-3
14
12
10
8
6
4
2
0
Absorbance
pH
- - 10,21
7,38
5,46
340 360 380 400 420 440 460 480 500
Longueur d'onde λ (nm)
B
16x10-3
Intensité de fluorescence (u.a.)
pH
- - 10,21
7,38
5,46
450 500 550 600 650
Longueur d'onde λ (nm)
C
% du max d'intensité de fluorescence
100
80
60
40
20
0
ECFP
ECFP (72A,145A, 148G)
4 6 8 10
pH
D
3.2
3.0
2.8
2.6
2.4
2.2
2.0
1.8
1.6
<τ> (ns)
ECFP
ECFP (72A, 145A, 148G)
5 6 7 8 9 10 11
pH

**Figure 8**

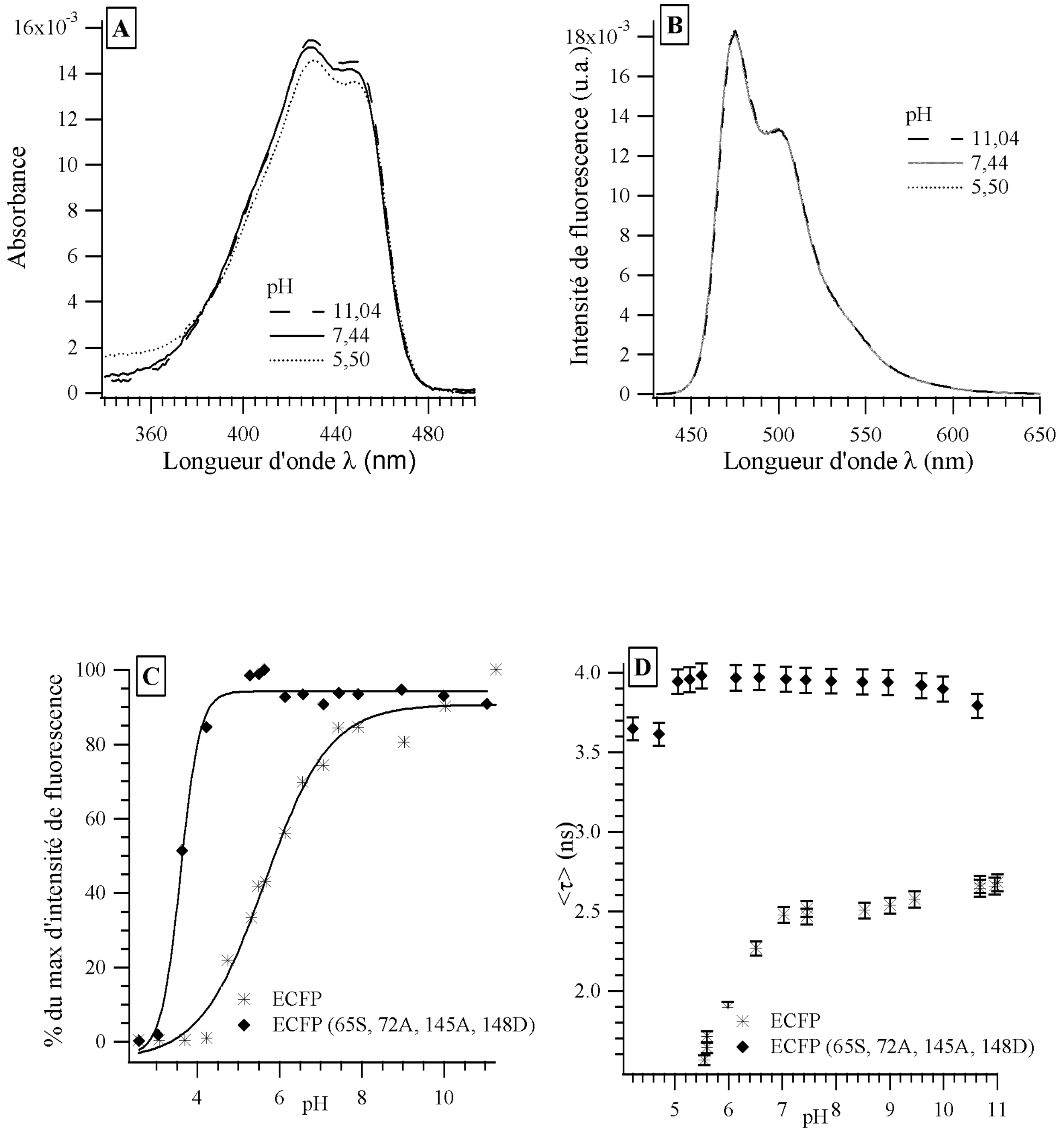
A
16x10-3
14
12
10
8
6
4
2
0
Absorbance
pH
11,04
7,44
5,50
360
400
440
480
Longueur d'onde λ (nm)
B
18x10-3
16
14
12
10
8
6
4
2
0
Intensité de fluorescence (u.a.)
pH
11,04
7,44
5,50
450
500
550
600
650
Longueur d'onde λ (nm)
C
100
80
60
40
20
0
% du max d'intensité de fluorescence
ECFP
ECFP (65S, 72A, 145A, 148D)
4
6
8
10
pH
D
4.0
3.5
3.0
2.5
2.0
<τ> (ns)
ECFP
ECFP (65S, 72A, 145A, 148D)
5
6
7
8
9
10
11
pH

**Figure 9**

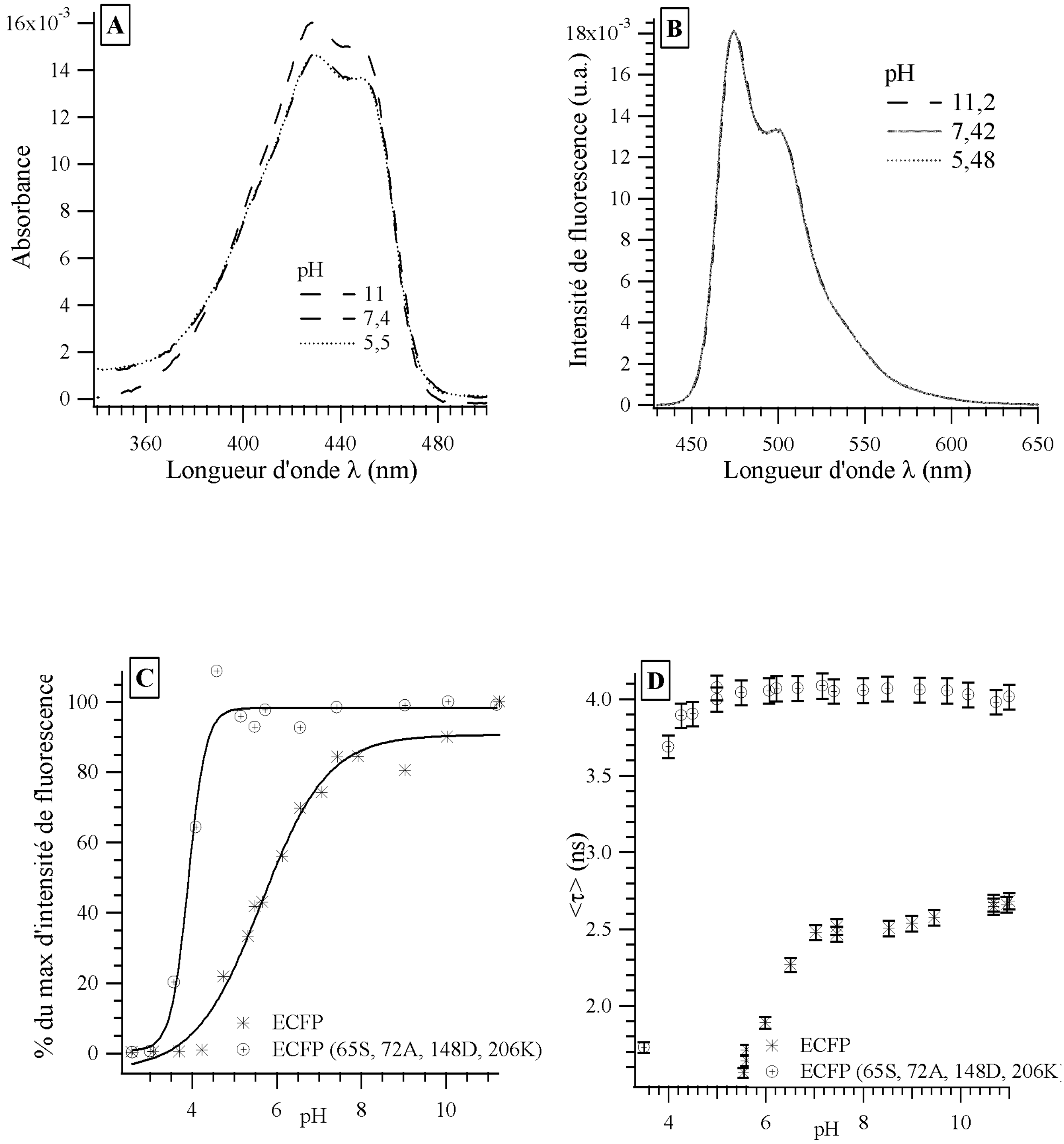
A
16x10-3
14
12
10
8
6
4
2
0
Absorbance
360
400
440
480
Longueur d'onde λ (nm)
pH
11
7,4
5,5
B
18x10-3
16
14
12
10
8
6
4
2
0
Intensité de fluorescence (u.a.)
450
500
550
600
650
Longueur d'onde λ (nm)
pH
11,2
7,42
5,48
C
100
80
60
40
20
0
% du max d'intensité de fluorescence
4
6
8
10
pH
ECFP
ECFP (65S, 72A, 148D, 206K)
D
4.0
3.5
3.0
2.5
2.0
<τ> (ns)
4
6
8
10
pH
ECFP
ECFP (65S, 72A, 148D, 206K)

**Figure 10**

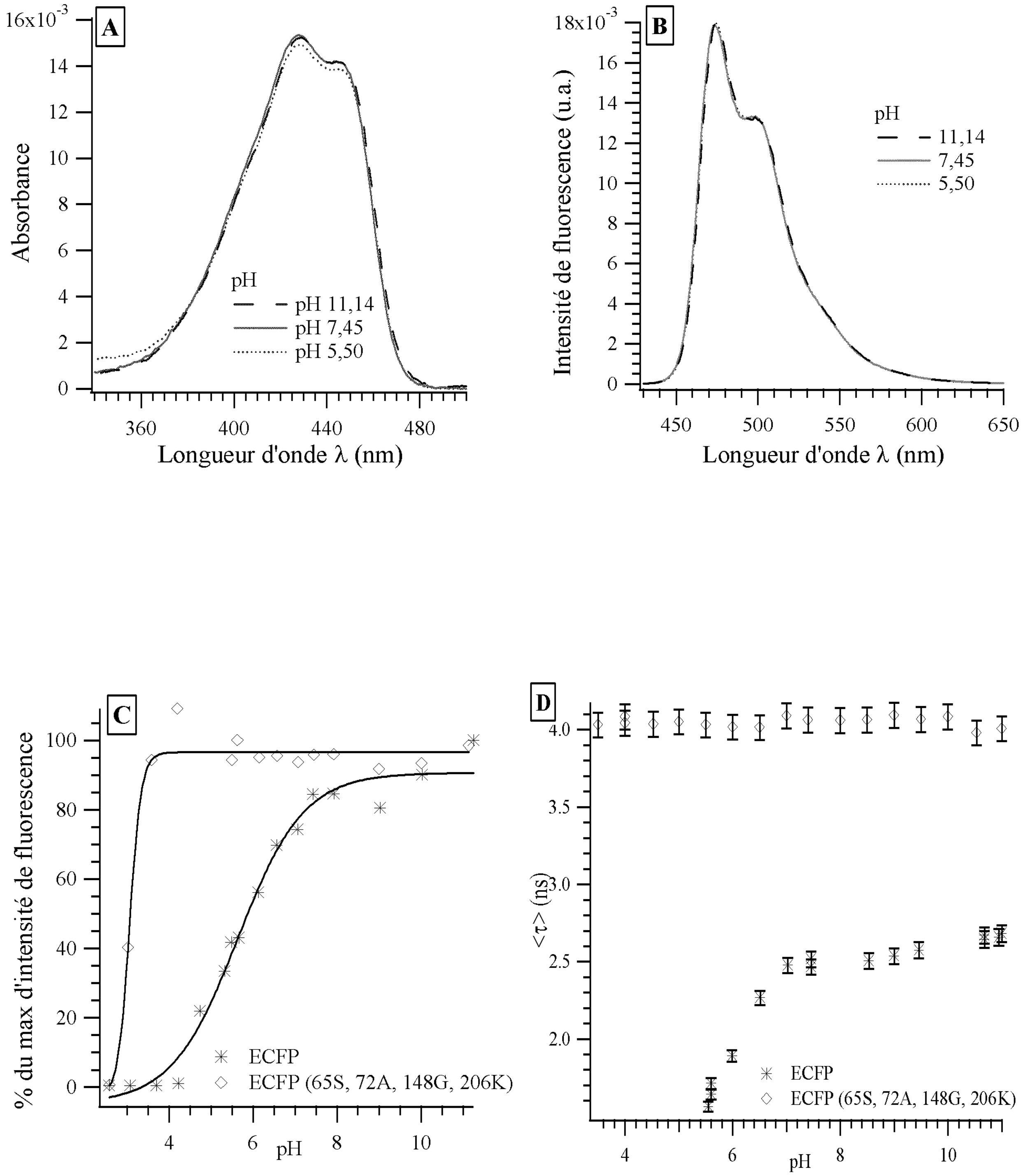
A
Absorbance
pH
pH 11,14
pH 7,45
pH 5,50
Longueur d'onde λ (nm)
B
Intensité de fluorescence (u.a.)
pH
11,14
7,45
5,50
Longueur d'onde λ (nm)
C
% du max d'intensité de fluorescence
ECFP
ECFP (65S, 72A, 148G, 206K)
pH
D
<τ> (ns)
ECFP
ECFP (65S, 72A, 148G, 206K)
pH

**Figure 11**

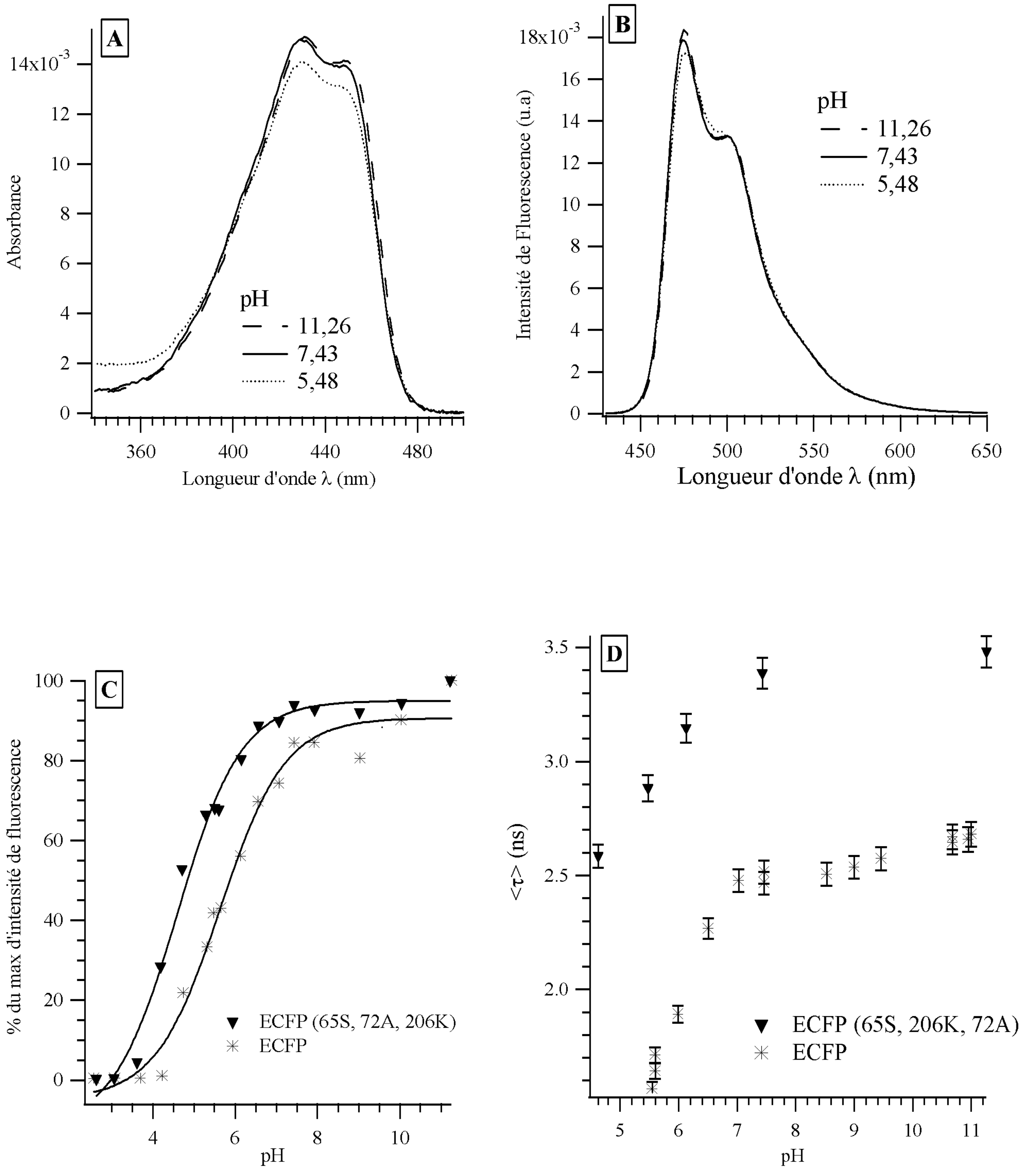
A
14x10-3
Absorbance
pH
- 11,26
7,43
5,48
Longueur d'onde λ (nm)
B
18x10-3
Intensité de Fluorescence (u.a)
pH
- 11,26
7,43
5,48
Longueur d'onde λ (nm)
C
% du max d'intensité de fluorescence
ECFP (65S, 72A, 206K)
ECFP
pH
D
<τ> (ns)
ECFP (65S, 206K, 72A)
ECFP
pH

**Figure 12**

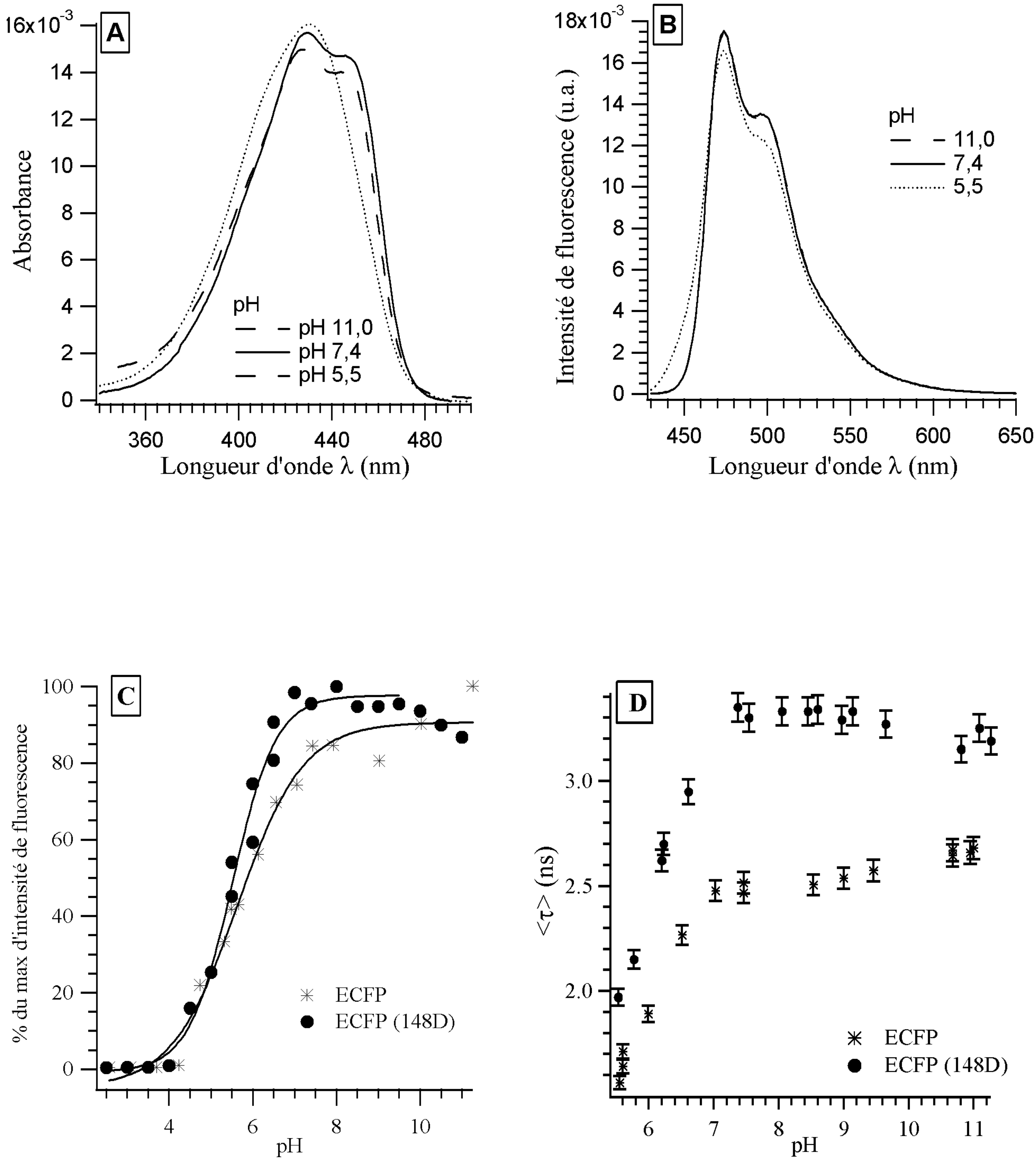
A
16x10-3
14
12
10
8
6
4
2
0
Absorbance
360
400
440
480
Longueur d'onde λ (nm)
pH
pH 11,0
pH 7,4
pH 5,5
B
18x10-3
16
14
12
10
8
6
4
2
0
Intensité de fluorescence (u.a.)
450
500
550
600
650
Longueur d'onde λ (nm)
pH
11,0
7,4
5,5
C
100
80
60
40
20
0
% du max d'intensité de fluorescence
4
6
8
10
pH
ECFP
ECFP (148D)
D
3.0
2.5
2.0
<τ> (ns)
6
7
8
9
10
11
pH
ECFP
ECFP (148D)

**Figure 13**

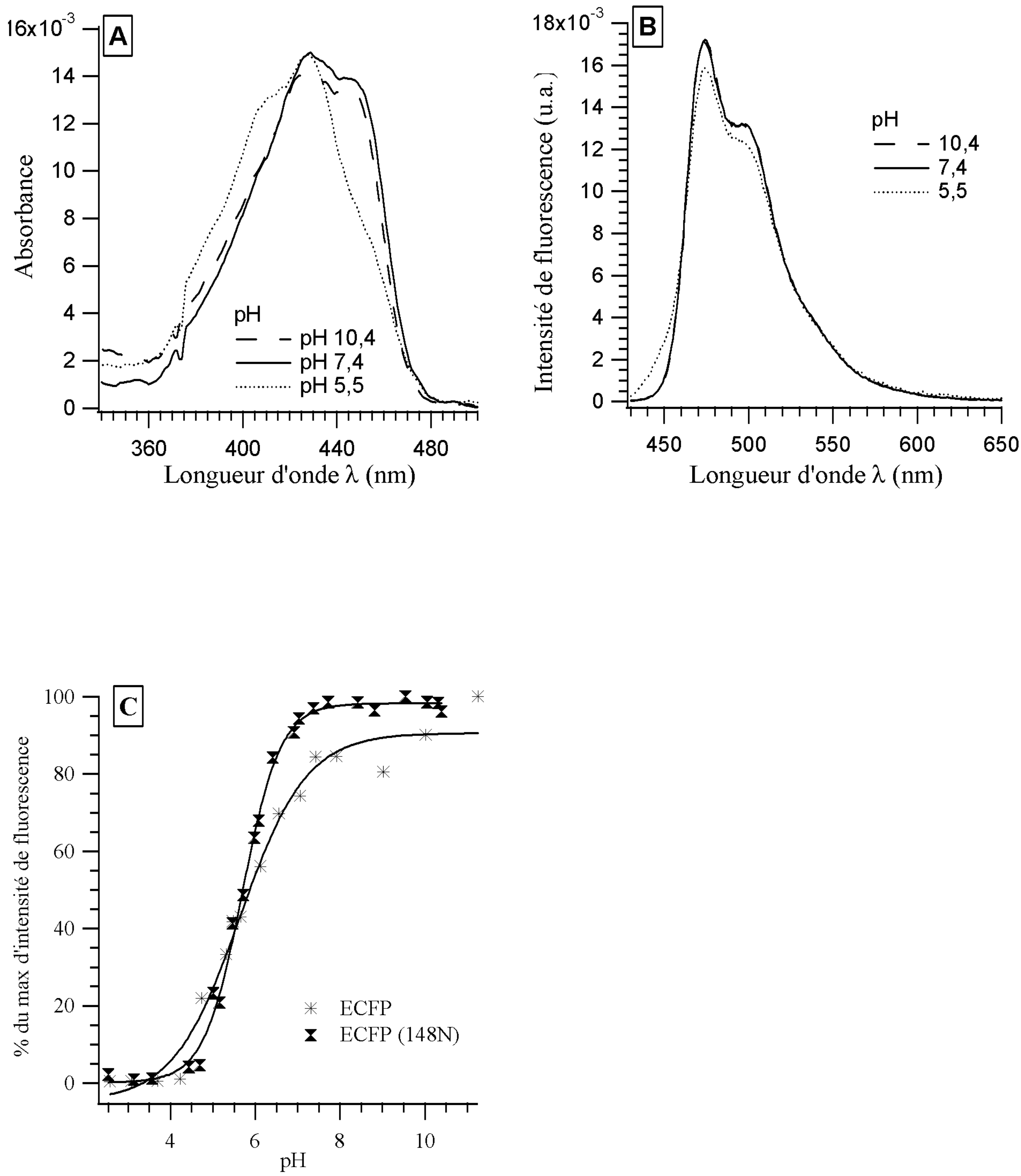
A
16x10-3
14
12
10
8
6
4
2
0
Absorbance
360
400
440
480
Longueur d'onde λ (nm)
pH
pH 10,4
pH 7,4
pH 5,5
B
18x10-3
16
14
12
10
8
6
4
2
0
Intensité de fluorescence (u.a.)
450
500
550
600
650
Longueur d'onde λ (nm)
pH
10,4
7,4
5,5
C
100
80
60
40
20
0
% du max d'intensité de fluorescence
4
6
8
10
pH
ECFP
ECFP (148N)

**Figure 14**

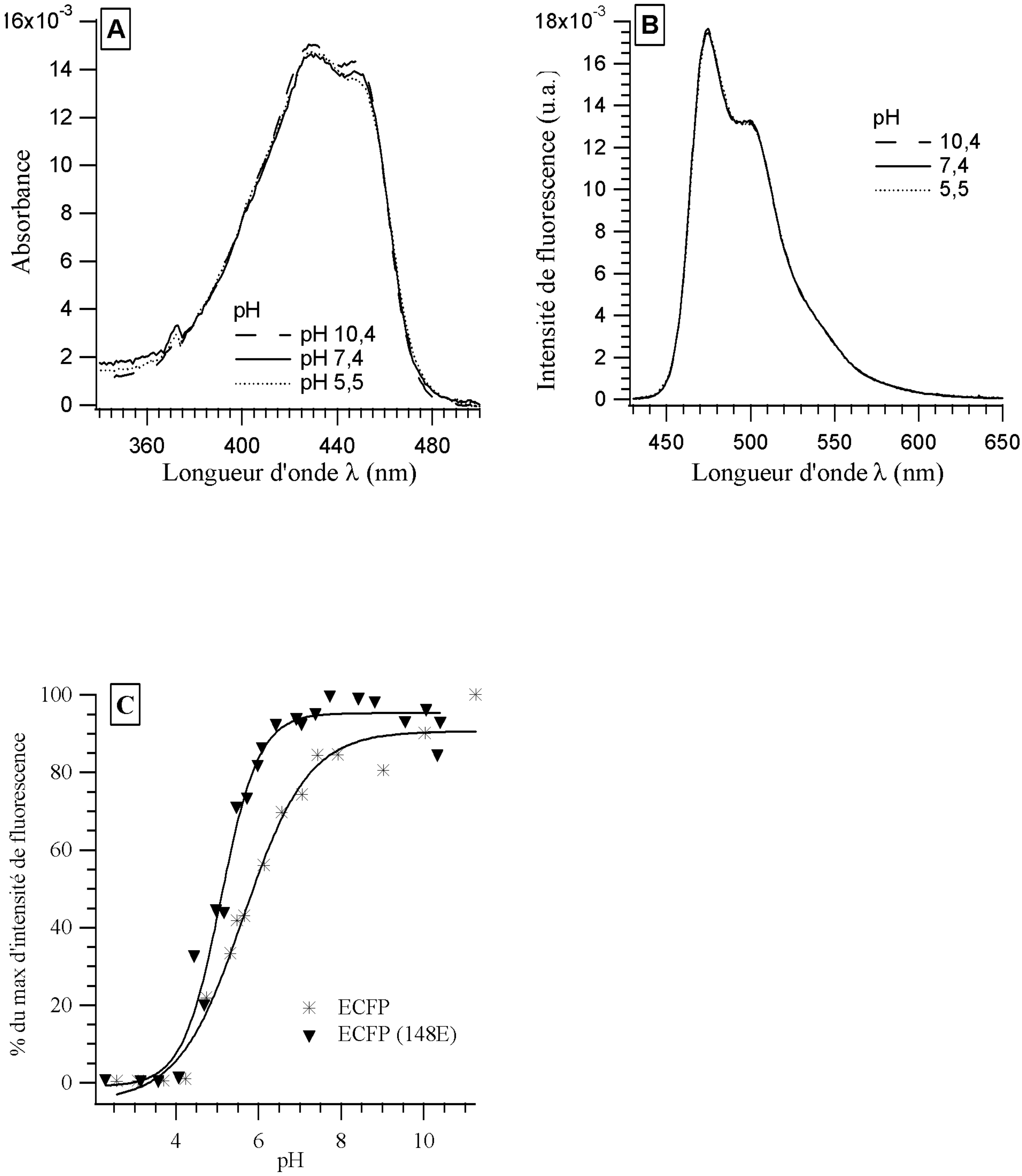
A
16x10-3
Absorbance
pH
pH 10,4
pH 7,4
pH 5,5
360
400
440
480
Longueur d'onde λ (nm)
B
18x10-3
Intensité de fluorescence (u.a.)
pH
10,4
7,4
5,5
450
500
550
600
650
Longueur d'onde λ (nm)
C
% du max d'intensité de fluorescence
ECFP
ECFP (148E)
pH

**Figure 15**

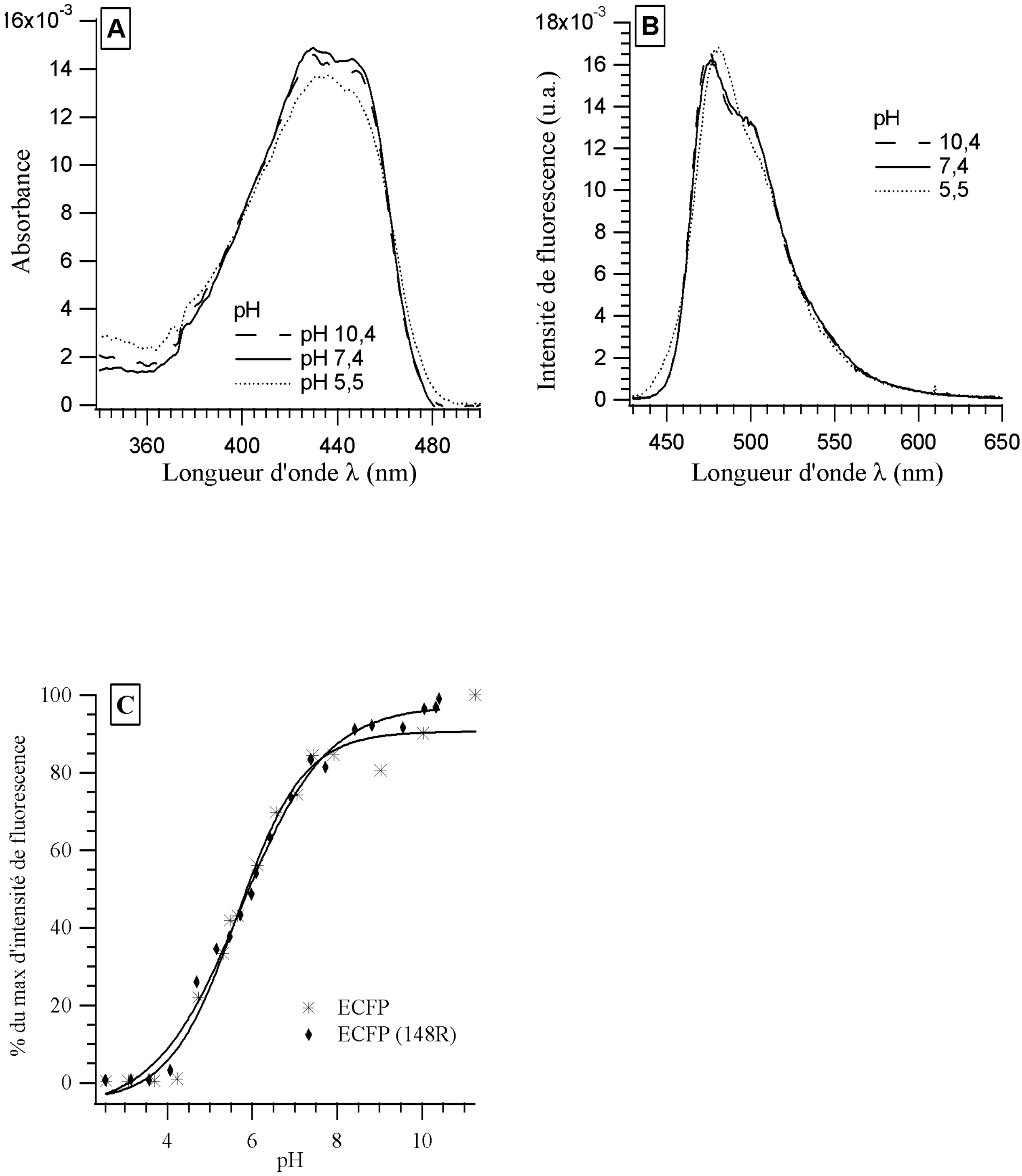
A
16x10-3
14
12
10
8
6
4
2
0
Absorbance
360
400
440
480
Longueur d'onde λ (nm)
pH
pH 10,4
pH 7,4
pH 5,5
B
18x10-3
16
14
12
10
8
6
4
2
0
Intensité de fluorescence (u.a.)
450
500
550
600
650
Longueur d'onde λ (nm)
pH
10,4
7,4
5,5
C
100
80
60
40
20
0
% du max d'intensité de fluorescence
4
6
8
10
pH
ECFP
ECFP (148R)

**Figure 16**

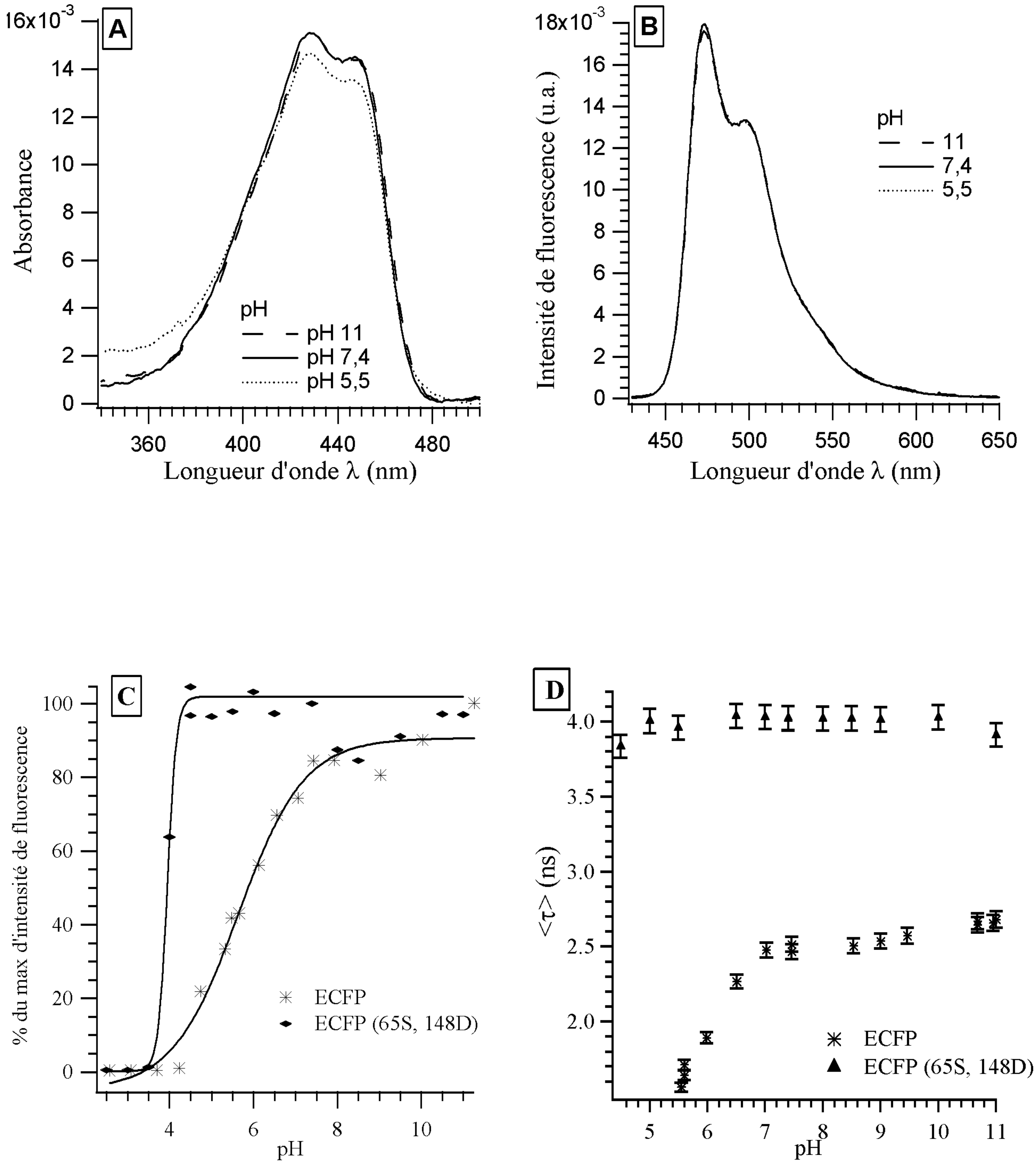
A
16x10-3
Absorbance
Longueur d'onde λ (nm)
pH
pH 11
pH 7,4
pH 5,5
B
18x10-3
Intensité de fluorescence (u.a.)
Longueur d'onde λ (nm)
pH
11
7,4
5,5
C
% du max d'intensité de fluorescence
pH
ECFP
ECFP (65S, 148D)
D
<τ> (ns)
pH
ECFP
ECFP (65S, 148D)

**Figure 17**

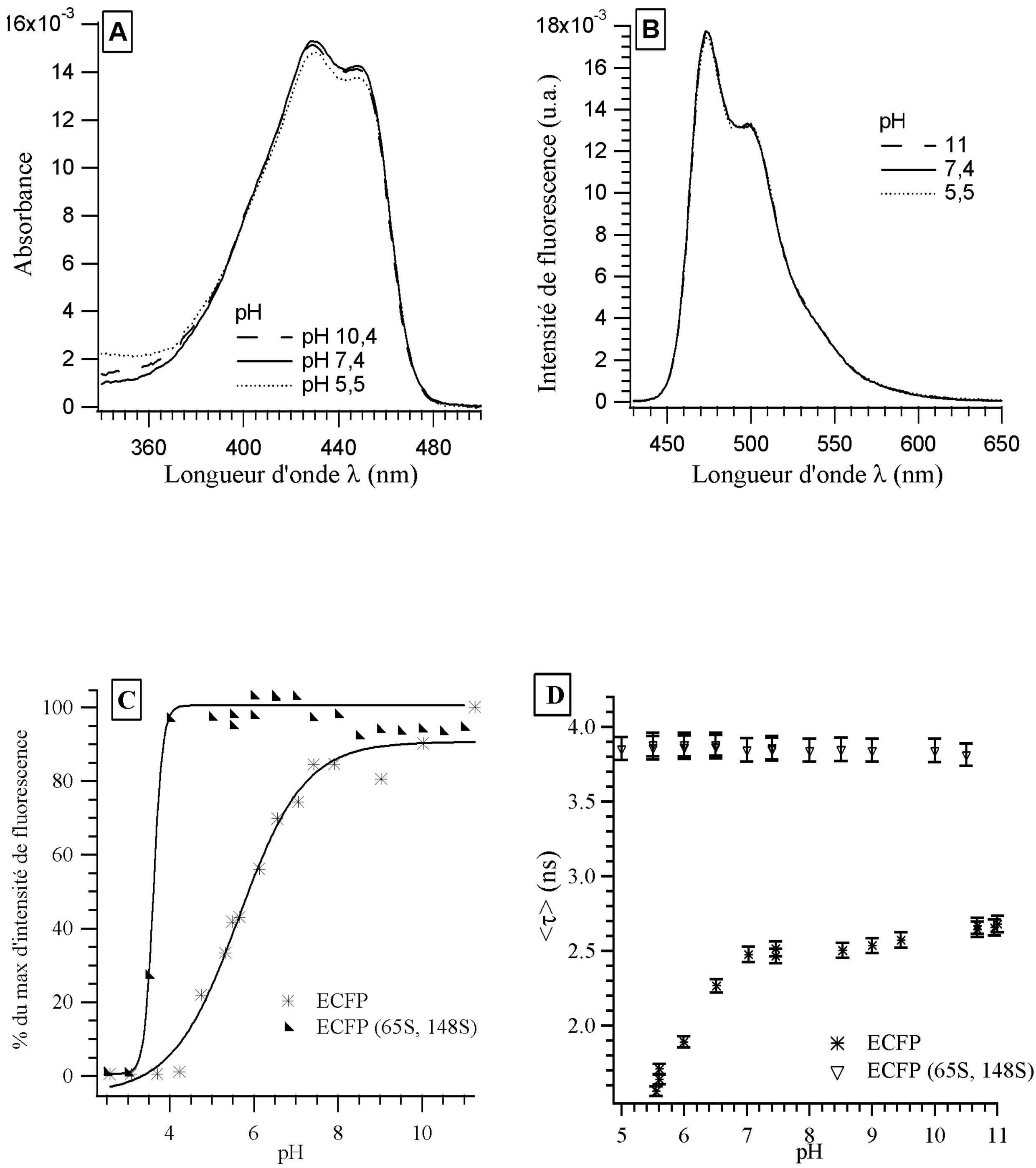
A
16x10-3
Absorbance
Longueur d'onde λ (nm)
pH
pH 10,4
pH 7,4
pH 5,5
B
18x10-3
Intensité de fluorescence (u.a.)
Longueur d'onde λ (nm)
pH
11
7,4
5,5
C
% du max d'intensité de fluorescence
pH
ECFP
ECFP (65S, 148S)
D
<τ> (ns)
pH
ECFP
ECFP (65S, 148S)

**Figure 18**

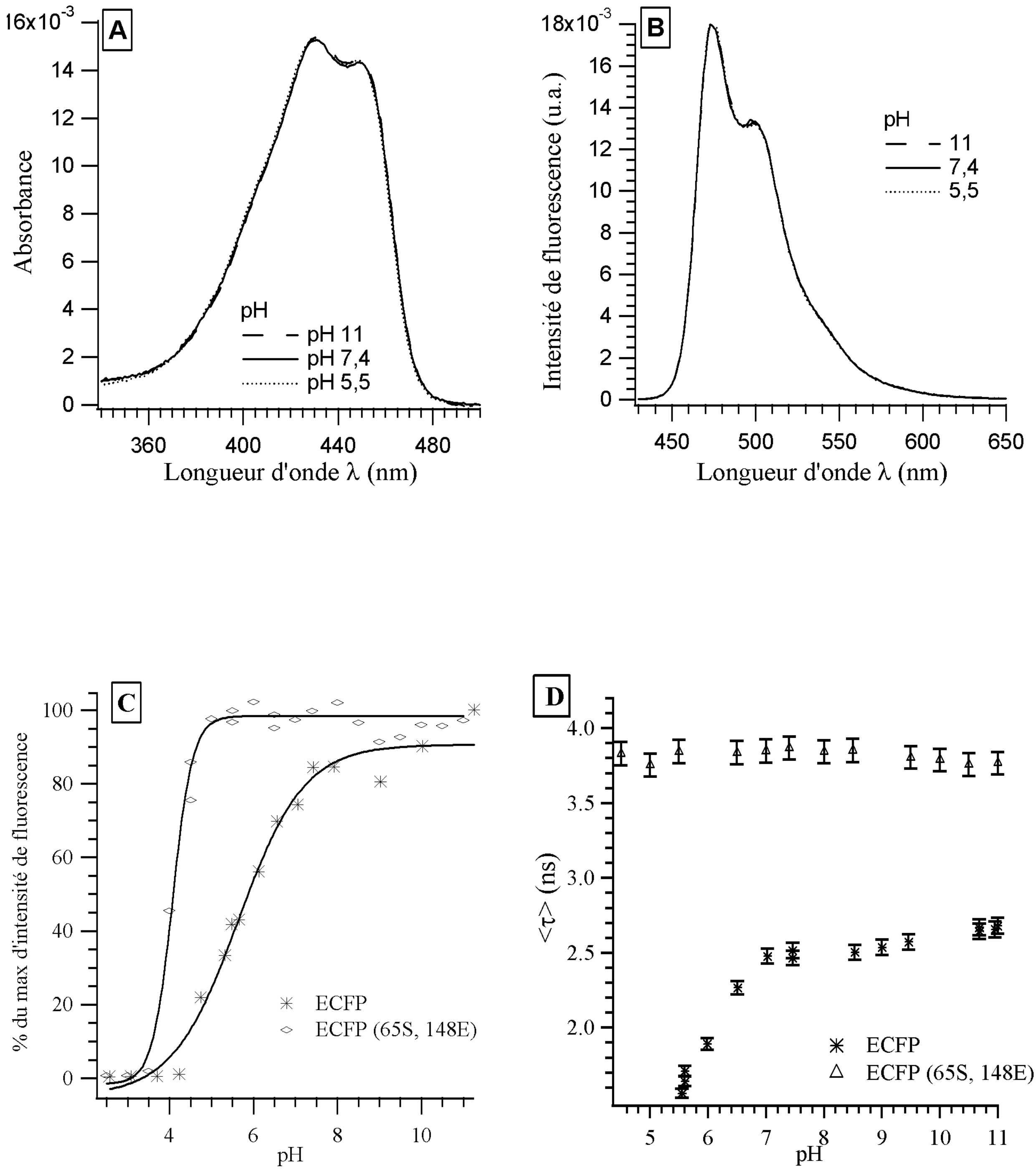
A
16x10^-3
Absorbance
Longueur d'onde λ (nm)
pH
pH 11
pH 7,4
pH 5,5
B
18x10^-3
Intensité de fluorescence (u.a.)
Longueur d'onde λ (nm)
pH
11
7,4
5,5
C
% du max d'intensité de fluorescence
pH
ECFP
ECFP (65S, 148E)
D
<τ> (ns)
pH
ECFP
ECFP (65S, 148E)

**Figure 19**

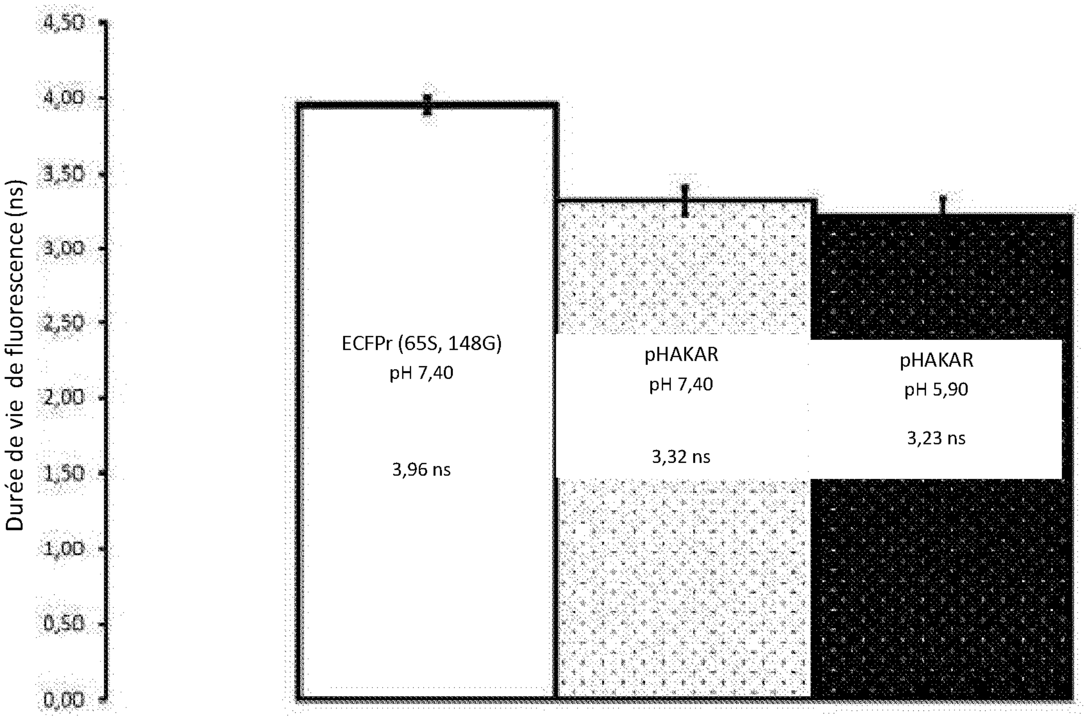
4,50
4,00
3,50
3,00
2,50
2,00
1,50
1,00
0,50
0,00
Durée de vie de fluorescence (ns)
ECFPr (65S, 148G)
pH 7,40
3,96 ns
pHAKAR
pH 7,40
3,32 ns
pHAKAR
pH 5,90
3,23 ns

**Figure 20**

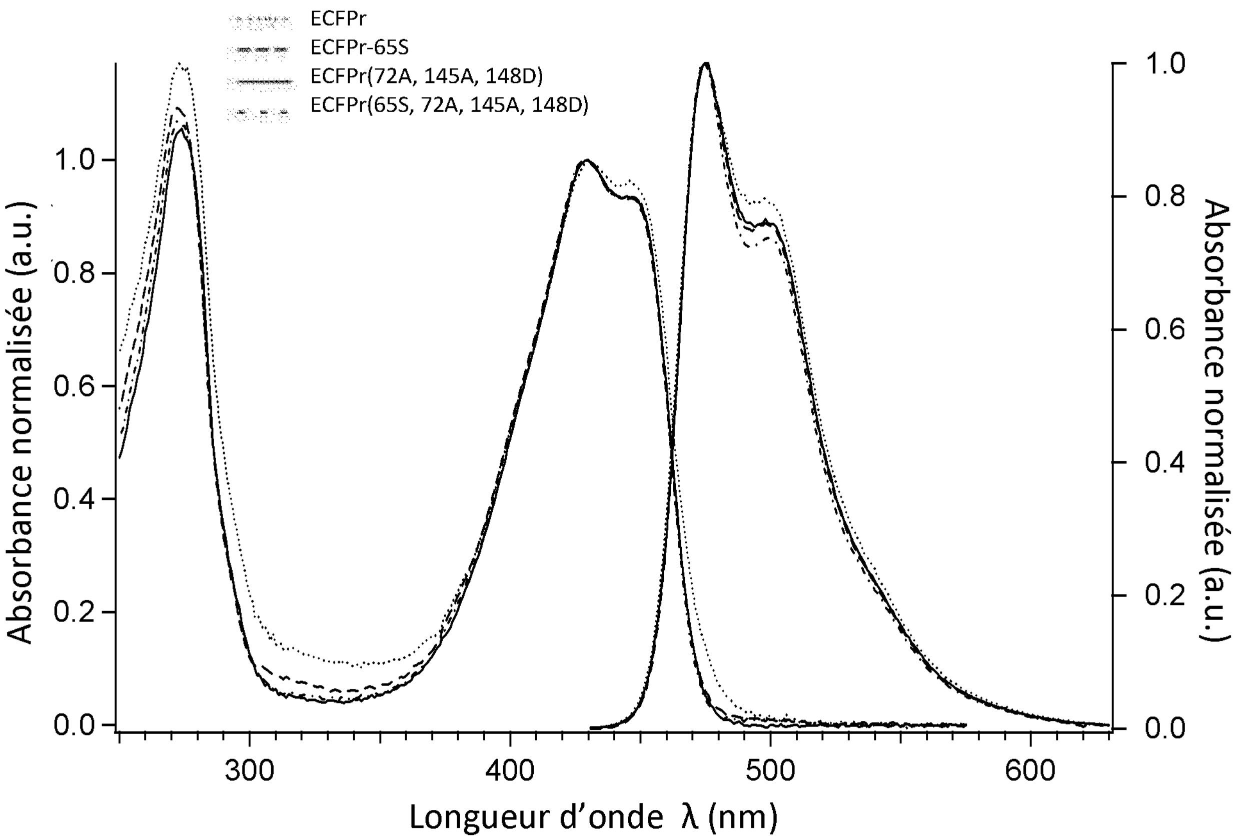
ECFPr
ECFPr-65S
ECFPr(72A, 145A, 148D)
ECFPr(65S, 72A, 145A, 148D)
Absorbance normalisée (a.u.)
Absorbance normalisée (a.u.)
0.0
0.2
0.4
0.6
0.8
1.0
300
400
500
600
Longueur d'onde λ (nm)

**Figure 21**

| | | | | | Photostabilité | | | |
|---|---|---|---|---|---|---|---|---|
| | | | | | sur billes d'agarose | | | en cellules vivantes |
| **Protéines étudiées** | **ε ($10^3$ $M^{-1}$ $cm^{-1}$)** | **Rend.Q.** | **$\tau_L$ (ns) ± SD** | **$c_L$ (%) ± Dev Std** | **% Rev ± 2%** | **$\tau_{Rev}$ (s) ± 0.1s** | **$\tau_{Irrev}$ (s) ± 5%** | **$\tau_{Irrev}$ (s) ± Dev Std** |
| ECFPr (SEQ ID N° 4) | 29 | 0,40 | 3,51 ± 0,12 | 52± 7 | 23 | 0.6 | 700 | 726 ± 88 (N=37) |
| ECFPr (65S ) | 28,5 | 0,59 | 3,78 ± 0,11 | 77± 8 | 3 | 1 | 940 | 828 ± 89 (N=23) |
| ECFPr (72A, 145A, 148D) | 29,2 | 0,67 | 3,80± 0,13 | 64± 7 | 33 | 1 | 610 | 643 ± 97 (N=21) |
| ECFPr (65S, 72A, 145A, 148D) | 34,8 | 0,84 | 4,17± 0,09 | 83± 11 | 3 | 0.8 | 772 | - |

**Figure 22**

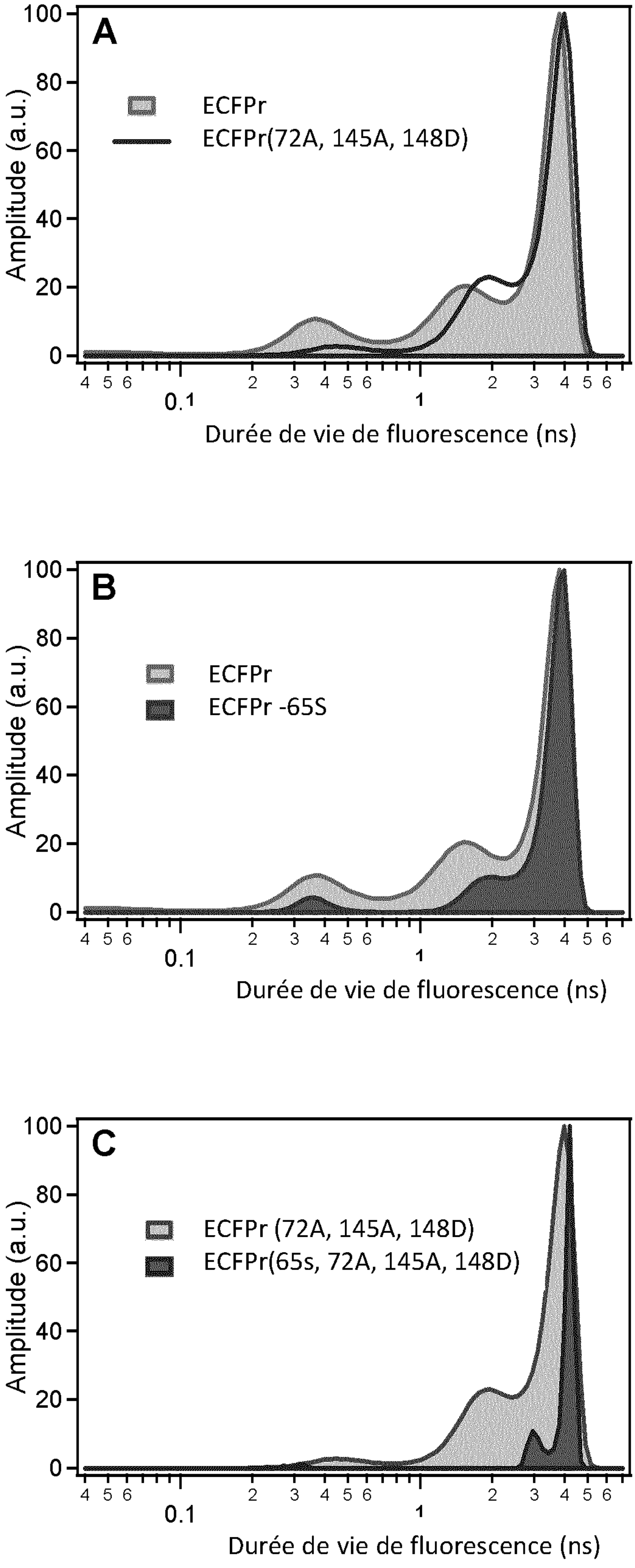
A
ECFPr
ECFPr(72A, 145A, 148D)
B
ECFPr
ECFPr -65S
C
ECFPr (72A, 145A, 148D)
ECFPr(65s, 72A, 145A, 148D)
Amplitude (a.u.)
Durée de vie de fluorescence (ns)

**Figure 23**

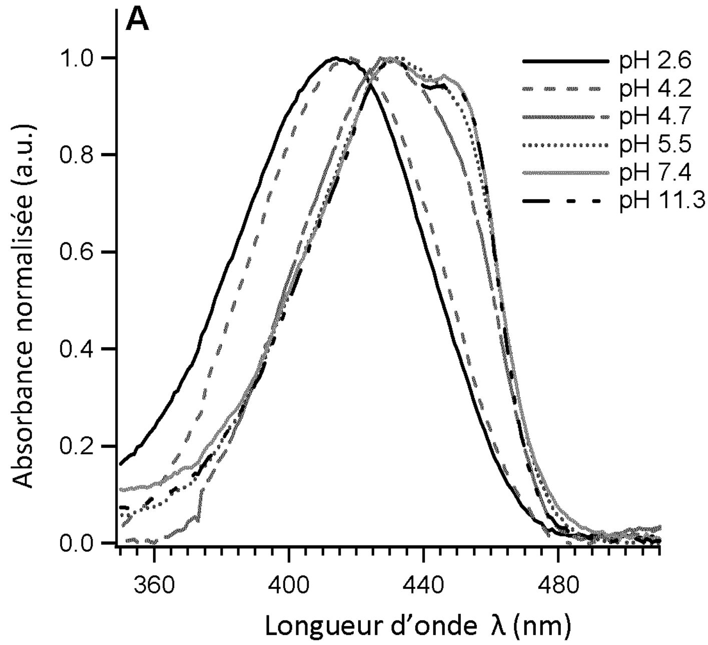
A
Absorbance normalisée (a.u.)
1.0
0.8
0.6
0.4
0.2
0.0
360
400
440
480
Longueur d'onde λ (nm)
pH 2.6
pH 4.2
pH 4.7
pH 5.5
pH 7.4
pH 11.3

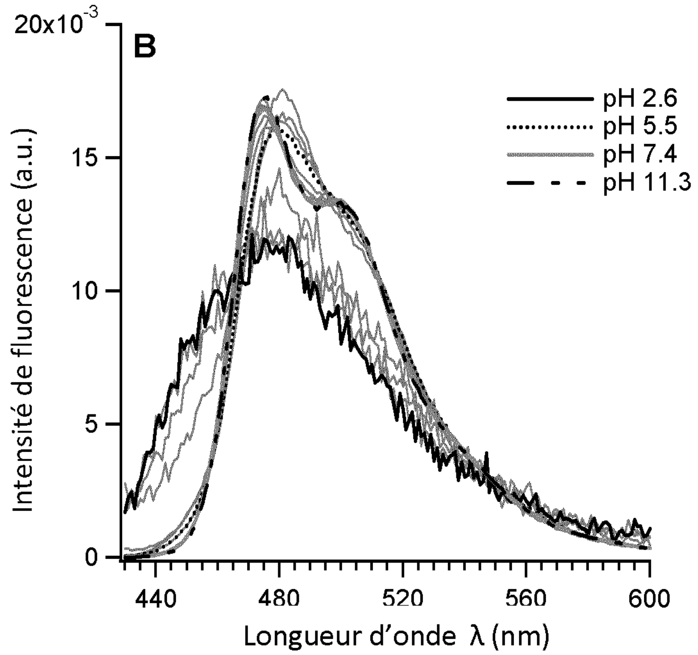
B
20x10^-3
Intensité de fluorescence (a.u.)
15
10
5
0
440
480
520
560
600
Longueur d'onde λ (nm)
pH 2.6
pH 5.5
pH 7.4
pH 11.3

**Figure 24**

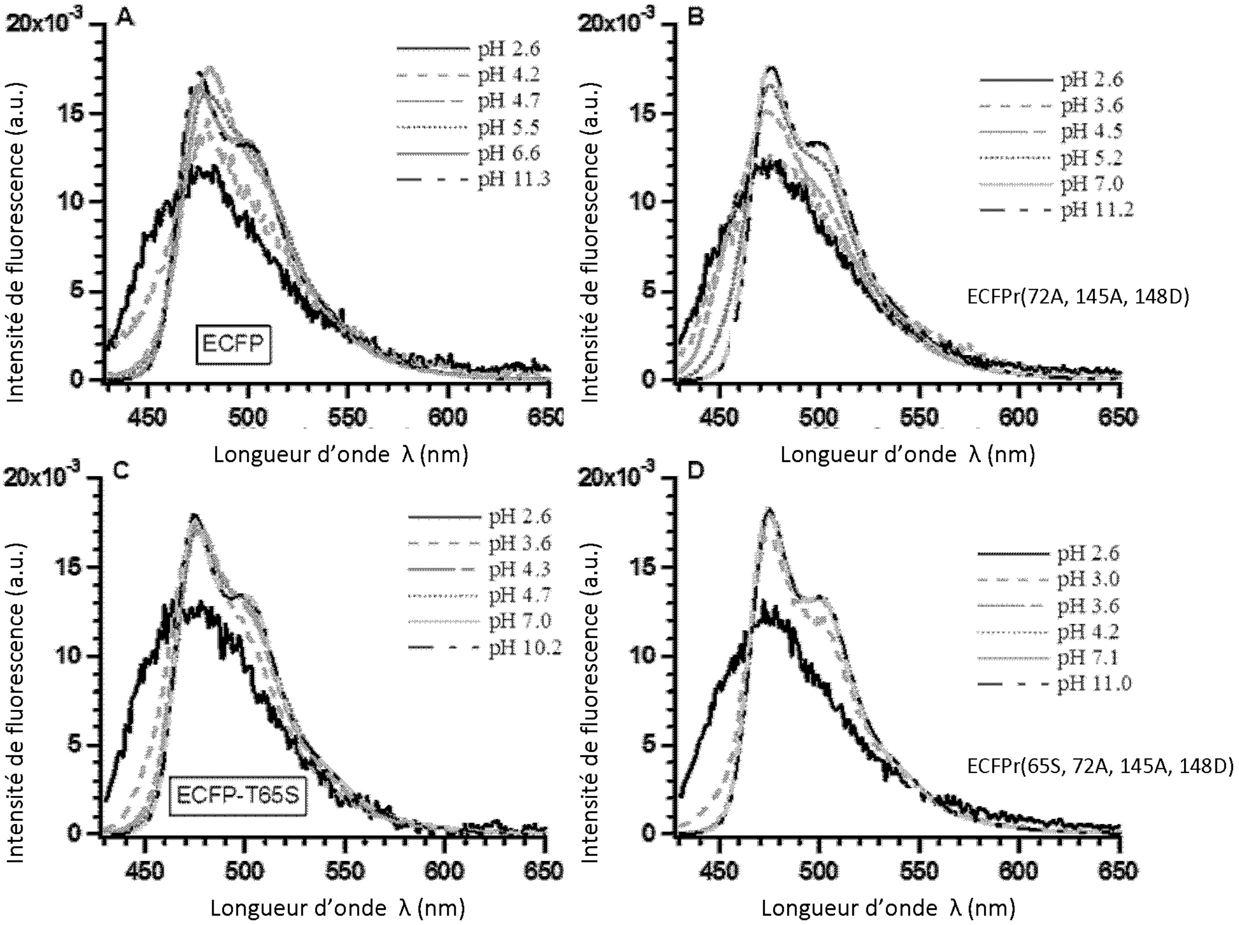

A
B
C
D
20x10⁻³
15
10
5
0
450
500
550
600
650
Intensité de fluorescence (a.u.)
Longueur d'onde λ (nm)
pH 2.6
pH 4.2
pH 4.7
pH 5.5
pH 6.6
pH 11.3
ECFP
pH 2.6
pH 3.6
pH 4.5
pH 5.2
pH 7.0
pH 11.2
ECFPr(72A, 145A, 148D)
pH 2.6
pH 3.6
pH 4.3
pH 4.7
pH 7.0
pH 10.2
ECFP-T65S
pH 2.6
pH 3.0
pH 3.6
pH 4.2
pH 7.1
pH 11.0
ECFPr(65S, 72A, 145A, 148D)


**Figure 25**

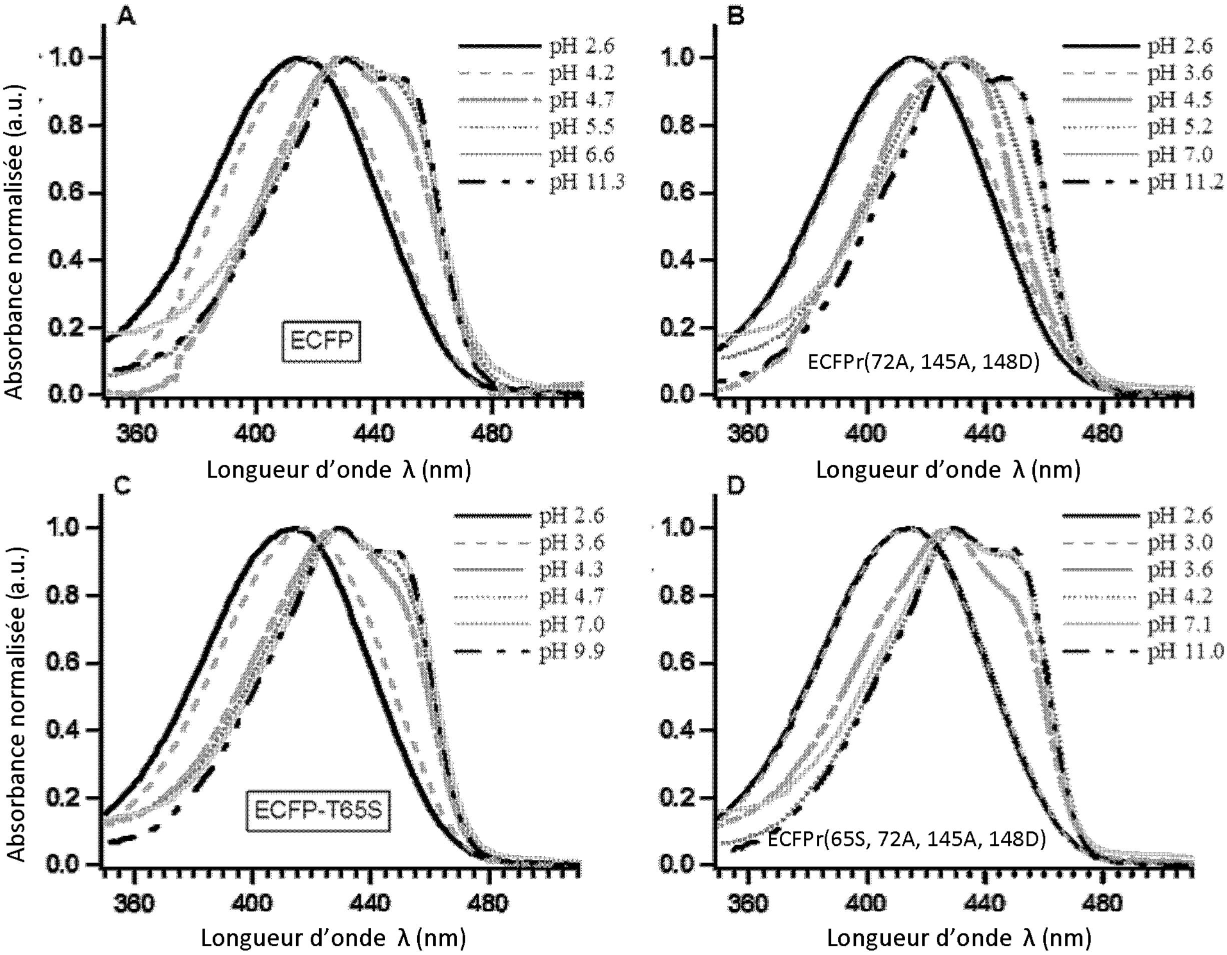
A
B
C
D
Absorbance normalisée (a.u.)
Longueur d'onde λ (nm)
ECFP
ECFPr(72A, 145A, 148D)
ECFP-T65S
ECFPr(65S, 72A, 145A, 148D)
pH 2.6
pH 4.2
pH 4.7
pH 5.5
pH 6.6
pH 11.3
pH 3.6
pH 4.5
pH 5.2
pH 7.0
pH 11.2
pH 4.3
pH 9.9
pH 3.0
pH 7.1
pH 11.0

Figure 26

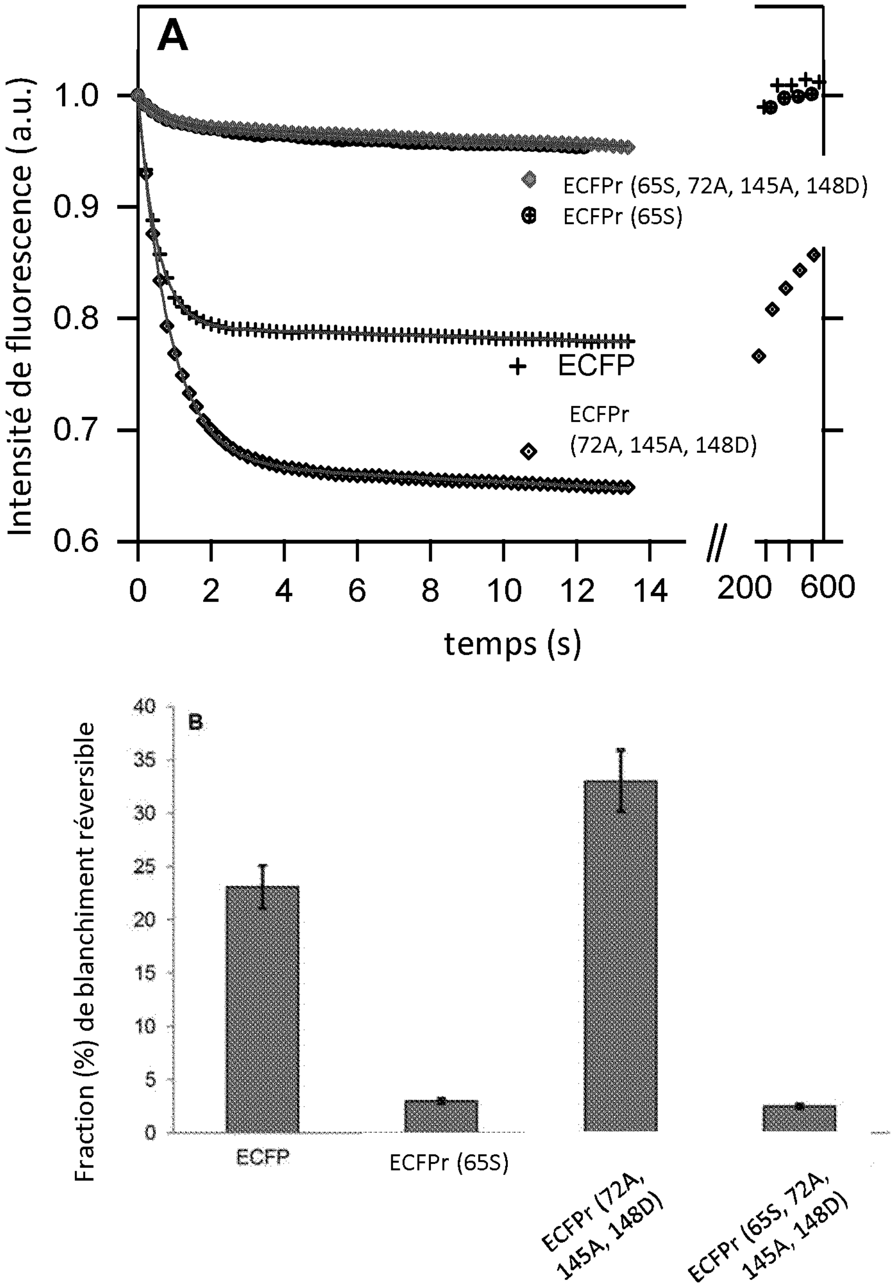
A
Intensité de fluorescence (a.u.)
1.0
0.9
0.8
0.7
0.6
ECFPr (65S, 72A, 145A, 148D)
ECFPr (65S)
ECFP
ECFPr
(72A, 145A, 148D)
0
2
4
6
8
10
12
14
200
600
temps (s)
B
Fraction (%) de blanchiment réversible
40
35
30
25
20
15
10
5
0
ECFP
ECFPr (65S)
ECFPr (72A, 145A, 148D)
ECFPr (65S, 72A, 145A, 148D)

**Figure 27**

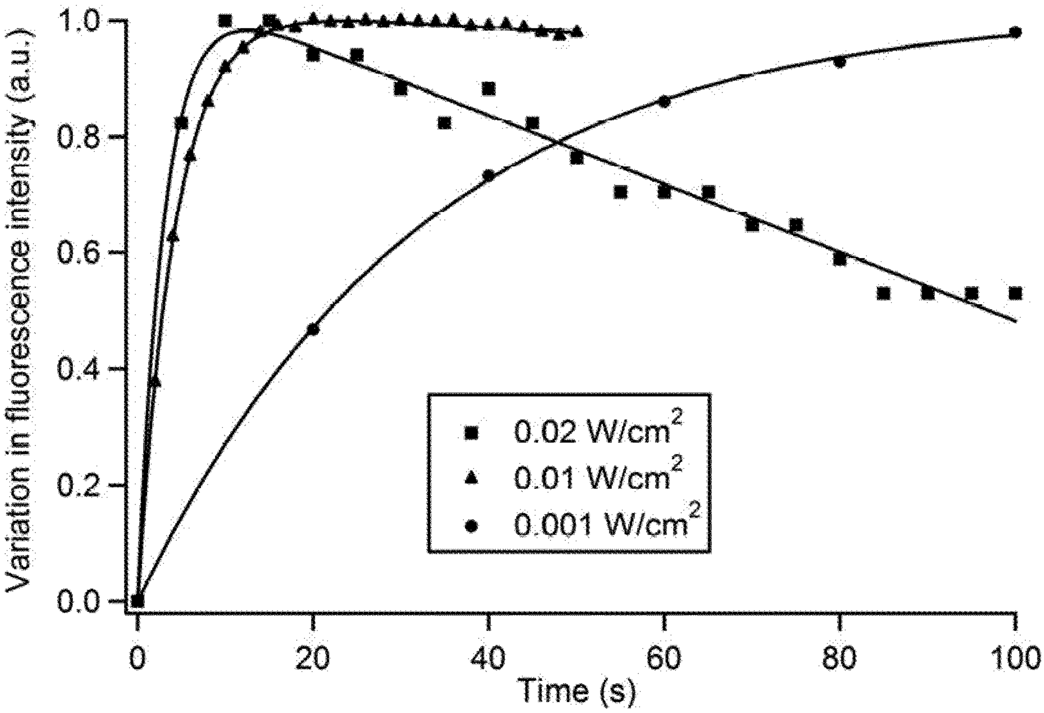
Variation in fluorescence intensity (a.u.)
1.0
0.8
0.6
0.4
0.2
0.0
0
20
40
60
80
100
Time (s)
0.02 W/cm2
0.01 W/cm2
0.001 W/cm2

**Figure 28**

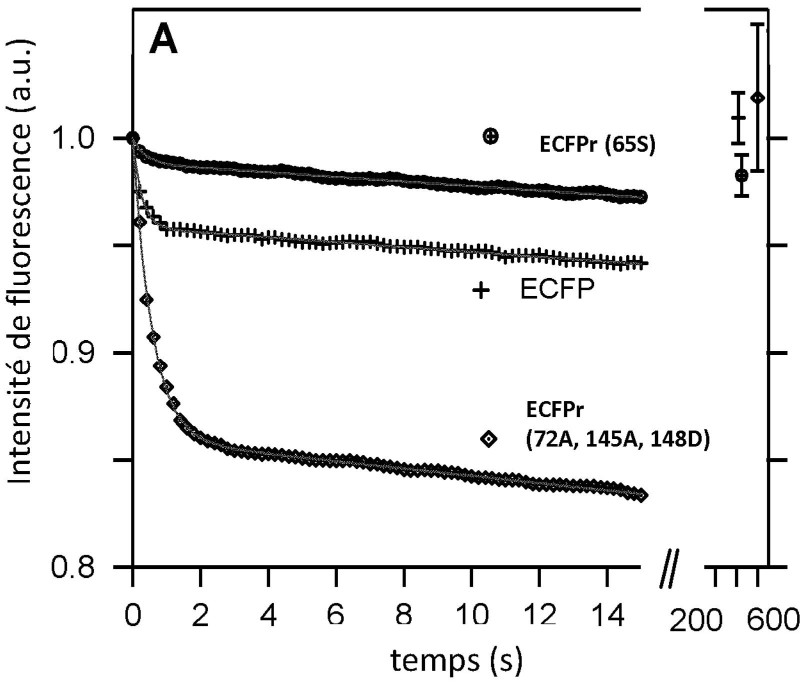
A
Intensité de fluorescence (a.u.)
1.0
0.9
0.8
ECFPr (65S)
+ ECFP
ECFPr
(72A, 145A, 148D)
0
2
4
6
8
10
12
14
200
600
temps (s)

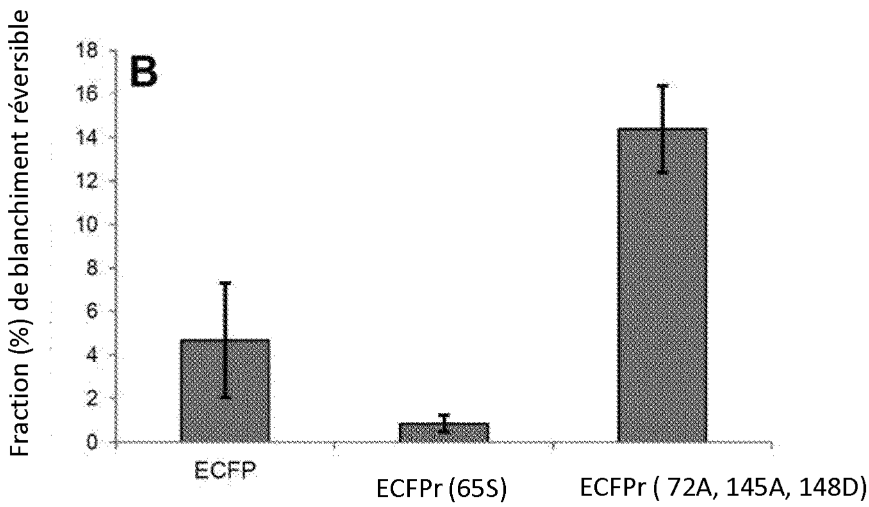
B
Fraction (%) de blanchiment réversible
18
16
14
12
10
8
6
4
2
0
ECFP
ECFPr (65S)
ECFPr ( 72A, 145A, 148D)

**Figure 29**

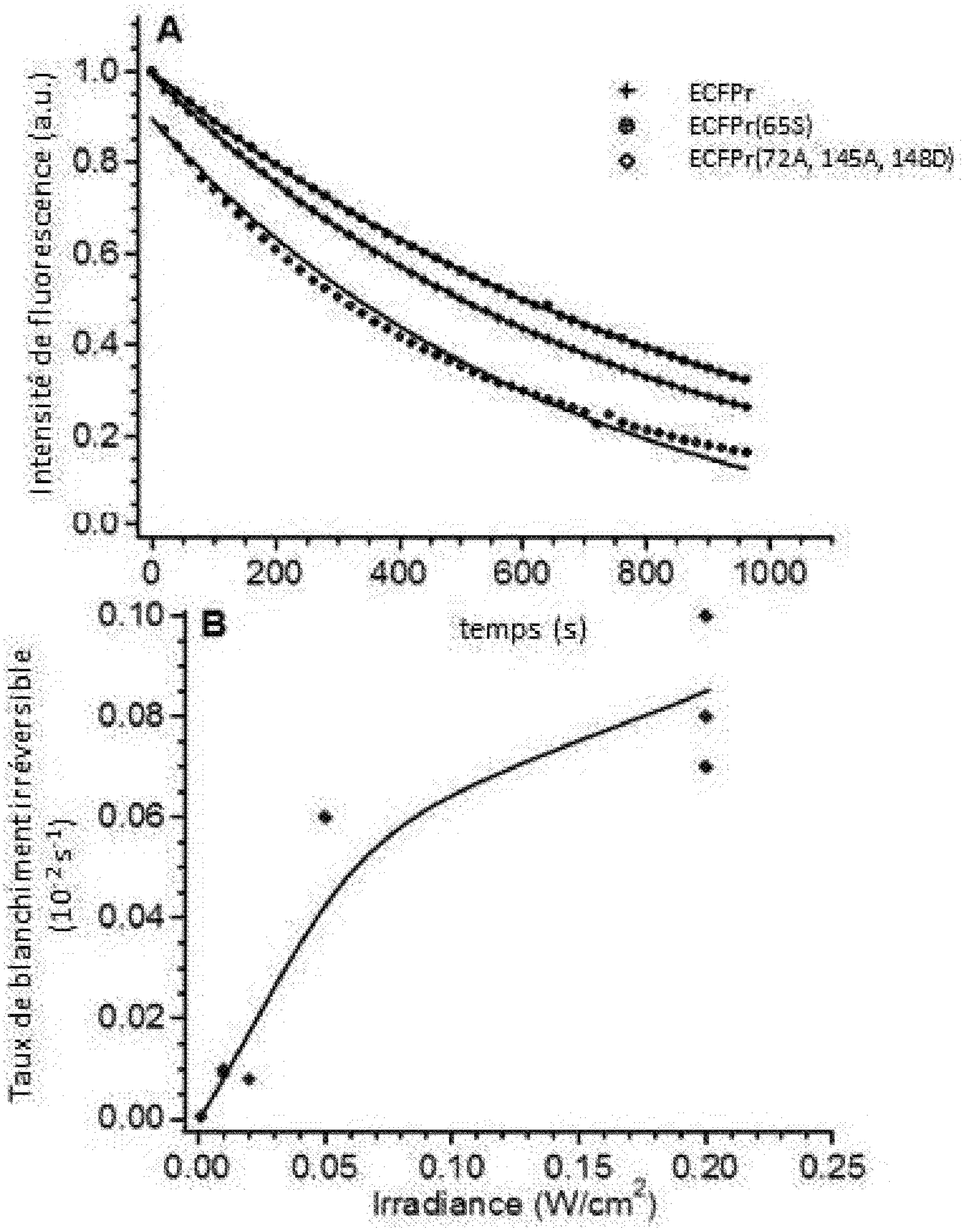
A
Intensité de fluorescence (a.u.)
1.0
0.8
0.6
0.4
0.2
0.0
ECFPr
ECFPr(65S)
ECFPr(72A, 145A, 148D)
0
200
400
600
800
1000
temps (s)
B
Taux de blanchiment irréversible (10⁻² s⁻¹)
0.10
0.08
0.06
0.04
0.02
0.00
0.00
0.05
0.10
0.15
0.20
0.25
Irradiance (W/cm²)

**Figure 30**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 1155227


### Littérature non-brevet citée dans la description


- **DAVID R. O'REILLY et al.** Baculovirus Expression vectors, A Laboratory Manual. Oxford University Press, 1992
- **PRASHER D.C. et al.** *Gene,* 1992, vol. 111, 229-33
- **DAY R.N. ; DAVIDSON M.W.** *Chemical society reviews,* 2009, vol. 38, 2887-2921
- **CUBITT A.B. ; HEIM R. ; ADAMS S.R. ; BOYD A.E. ; GROSS L.A. ; TSIEN R.Y.** *Trends Biochem Sci.,* 1995, vol. 20 (11), 448-55
- **LLOPIS J. ; MCCAFFERY J.M. ; MIYAWAKI A. ; FARQUHAR M.G. ; TSIEN R.Y.** *Proc Natl Acad Sci USA,* 1998, vol. 95 (12), 6803-8
- **CUBITT A.B. ; WOOLLENWEBER L.A. ; HEIM R.** *Methods Cell Biol.,* 1999, vol. 58, 19-30
- **MIYAWAKI A. ; GRIESBECK O. ; HEIM R. ; TSIEN, R.Y.** *Proc Natl Acad Sci USA,* 1999, vol. 96 (5), 2135-40
- **SHANER N.C. ; PATTERSON G.H. ; DAVIDSON M.W.** *J. Cell. Sci.,* 2007, vol. 120, 4247-4260
- **PATTERSON G.H. ; DAY R.N. ; PISTON D.** *J. Cell Sci.,* 2001, vol. 114, 837-838
- **TRAMIER M. ; GAUTIER I. ; PIOLOT T. ; RAVALET S. ; KEMNITZ K. ; COPPEY J. ; DURIEUX C. ; MIGNOTTE V. ; COPPEY-MOISAN M.** *Biophys J.,* 2002, vol. 83 (6), 3570-7
- **MIYAWAKI A. ; TSIEN R. Y.** *Methods Enzymol.,* 2000, vol. 327, 472-499
- **RIZZO M.A ; SPRINGER G.H. ; GRANADA B. ; PISTON D.W.** *Nat. Biotechnol.,* 2004, vol. 22 ((4)), 445-449
- **MALO G.D. ; POUWELS L.J. ; WANG MT. ; WEICHSEL A. ; MONTFORT W.R. ; RIZZO M.A. ; PISTON, D.W. ; WACHTER R.M.** *Biochemistry,* 2007, vol. 46 (35), 9865-9873
- **NGUYEN A.W. ; DAUGERTHY P.S.** *Nature Biotechnology,* 2005, vol. 23 (3), 355-360
- **SHANER N.C. ; STEINBACH P.A. ; TSIEN R.Y.** *Nat. Methods,* 2005, vol. 2, 905-909
- **GOEDHART J. ; VAN WEEREN L. ; HINK M.A. ; VISCHER N.O. ; JALINK K. ; GADELLA T.W. JR.** *Nat. Methods,* 2010, vol. 7 (2), 137-9
- **SAWANO A. ; MIYAWAKI A.** *Nucleic Acids Res.,* 2000, vol. 28 (16), E78
- **ESPAGNE A. ; ERARD M. ; MADIONA K. ; DERRIEN V. ; JONASSON G. ; LÉVY B. ; PASQUIER H. ; MELKI R. ; MÉROLA F.** *Biochemistry,* 2011, vol. 50 (4), 437-9
- **VALEUR, B.** Molecular Fluorescence : Principles and Applications. Wiley-VCH, 2006
- **O'CONNOR, D.V. ; PHILIPPS, D.** Time-correlated single photon counting. Academic Press, 1984
- **NEEDLEMAN S. B. ; WUNSCH C. D.** *J. Mol. Biol.,* 1970, vol. 48, 443-453
- **GRIESBECK O. ; BAIRD G.S. ; CAMPBELL R.E. ; ZACHARIAS D.A. ; TSIEN R.Y.** *The journal of biological chemistry,* 2001, vol. 276 (31), 29188-29194
- **NAGAI T. ; IBATA K. ; PARK E.S. ; KUBOTA M. ; MIKOSHIBA K ; MIYAWAKI, A.** *Nature Biotechnology,* 2002, vol. 20, 87-90
- **SAMBROOK et al.** Molecular Cloning: A Laboratory Manual,. Cold Spring Harbor Laboratory Press, 2001
- **AUSUBEL et al.** Current Protocols in Molecular Biology. John Wiley & Sons, 2011
- **MERZLYAK E.M. ; GOEDHART J. ; SHCHERBO D. ; BULINA M.E. ; SHCHEGLOV A.S. ; FRADKOV A.F. ; GAINTZEVA A. ; LUKYANOV K.A. ; LUKYANOV S. ; GADELLA T.W.** *Nat Methods,* 2007, vol. 4 (7), 555-7
- **ZHANG J. ; MA Y. ; TAYLOR S.S. ; TSIEN R.Y.** *Proc Natl Acad Sci USA,* 2001, vol. 98, 14997-15002
- **ZHANG J. ; HUPFELD C.J. ; TAYLOR S.S. ; OLEFSKY J.M. ; TSIEN R.Y.** *Nature,* 2005, vol. 437 (7058), 569-573
- **DUNN T.A. ; WANG C.T. ; COLICOS M.A. ; ZACCOLO M. ; DIPILATO L.M. ; ZHANG J. ; TSIEN R.Y. ; FELLER M.B.** *The Journal of Neuroscience,* 2006, vol. 26 (49), 12807-12815
- **MORRIS M.C.** *Cell Biochem Biophys.,* 2010, vol. 56, 19-37
- **FROMMER W. B. ; DAVIDSON M. W. ; CAMPBELL R. E.** *Chem Soc Rev,* 2009, vol. 38, 2833-41
- **HEYDUK.** *Curr Opin Biotechnol.,* 2002, vol. 13 (4), 292-6

- **TRUONG et al.** *Curr Opin Struct Biol,* 2001, vol. 11 (5), 573-8
- **ISSAD et al.** *Diabetes Metab.,* 2003, vol. 29, 111-7
- **BOUTE et al.** *Pharmacol Sci.,* 2002, vol. 23 (8), 351-4
- **TRUGNAN G. ; FONTANGES P. ; DELAUTIER D. ; AIT-SLIMANE T.** *Medecine/Science,* 2004, vol. 20, 1027-34
- **KUMAR S. ; ALIBHAI D. ; MARGINEANU A. ; LAINE R. ; KENNEDY G. et al.** *Chem Phys Chem.,* 2011, vol. 12, 209-626
- **MÉROLA F. ; RIGLER R. ; HOLMGREN A. ; BROCHON J.C.** *Biochemistry,* 1989, vol. 28, 3383-3398
- **COUPRIE M.E. ; MÉROLA F. ; TAUC P. ; GARZELLA D. ; DELBOULBÉ A. ; HARA, T. ; BILLARDON, M.** *Review of Scientific Instruments,* 1994, vol. 65, 1485-1495
- **GIULIANI A. ; JAMME F. ; ROUAM V. ; WIEN F. ; GIORGETTA J.L. ; LAGARDE B. ; CHUBAR O. ; BAC S. ; YAO L ; REY S.** *J Synchrotron Radiat,* 2009, vol. 16 (6), 835-841
- **WIEN F. ; WALLACE B.A.** *Appl Spectrosc,* 2005, vol. 59, 1109-1113
- **LEES J.G. ; SMITH B.R. ; WIEN F. ; MILES A.J. ; WALLACE B.A.** *Anal Biochem,* 2004, vol. 332, 285-289
- **KELLY S.M. ; JESS T.J. ; PRICE N.C.** *Biochim Biophys Acta,* 2005, vol. 1751, 119-139
- **WHITMORE L. ; WALLACE B.A.** *Nucleic Acids Res,* 2004, vol. 32, W668-673
- **LEES J.G. ; MILES A.J. ; WIEN F. ; WALLACE B.A.** *Bioinformatics,* 2006, vol. 22, 1955-1962

SEQUENCE LISTING

<110> CENTRE NATIONAL DE LA RECHERCHE SCIENTIFIQUE (CNRS) UNIVERSITE PARIS-SUD 11

<120> METHODE POUR GENERER DES PROTEINES FLUORESCENTES CYANS AYANT UNE SENSIBILITE REDUITE AU PH

<130> 360942D29024

<150> FR 1155227
<151> 2011-06-15

<160> 82

<170> PatentIn version 3.5

<210> 1
<211> 714
<212> DNA
<213> artificial

<220>
<223> Séquence nucléotidique de la ECFP

<400> 1

```
gtgagcaagg gcgaggagct gttcaccggg gtggtgccca tcctggtcga gctggacggc        60
gacgtaaacg gccacaagtt cagcgtgtcc ggcgagggcg agggcgatgc cacctacggc       120
aagctgaccc tgaagttcat ctgcaccacc ggcaagctgc ccgtgccctg gcccaccctc       180
gtgaccaccc tgacctgggg cgtgcagtgc ttcagccgct accccgacca catgaagcag       240
cacgacttct tcaagtccgc catgcccgaa ggctacgtcc aggagcgcac catcttcttc       300
aaggacgacg gcaactacaa gacccgcgcc gaggtgaagt tcgagggcga caccctggtg       360
aaccgcatcg agctgaaggg catcgacttc aaggaggacg gcaacatcct ggggcacaag       420
ctggagtaca actacatcag ccacaacgtc tatatcaccg ccgacaagca gaagaacggc       480
atcaaggcca acttcaagat ccgccacaac atcgaggacg gcagcgtgca gctcgccgac       540
cactaccagc agaacacccc catcggcgac ggccccgtgc tgctgcccga caaccactac       600
ctgagcaccc agtccgccct gagcaaagac cccaacgaga agcgcgatca catggtcctg       660
ctggagttcg tgaccgccgc cgggatcact ctcggcatgg acgagctgta caag             714
```

<210> 2
<211> 238
<212> PRT
<213> artificial

<220>
<223> Séquence d’acides aminés de la ECFP

<400> 2

```
Val Ser Lys Gly Glu Glu Leu Phe Thr Gly Val Val Pro Ile Leu Val
1               5                   10                  15
```

Glu Leu Asp Gly Asp Val Asn Gly His Lys Phe Ser Val Ser Gly Glu
20 25 30

Gly Glu Gly Asp Ala Thr Tyr Gly Lys Leu Thr Leu Lys Phe Ile Cys
35 40 45

Thr Thr Gly Lys Leu Pro Val Pro Trp Pro Thr Leu Val Thr Thr Leu
50 55 60

Thr Trp Gly Val Gln Cys Phe Ser Arg Tyr Pro Asp His Met Lys Gln
65 70 75 80

His Asp Phe Phe Lys Ser Ala Met Pro Glu Gly Tyr Val Gln Glu Arg
85 90 95

Thr Ile Phe Phe Lys Asp Asp Gly Asn Tyr Lys Thr Arg Ala Glu Val
100 105 110

Lys Phe Glu Gly Asp Thr Leu Val Asn Arg Ile Glu Leu Lys Gly Ile
115 120 125

Asp Phe Lys Glu Asp Gly Asn Ile Leu Gly His Lys Leu Glu Tyr Asn
130 135 140

Tyr Ile Ser His Asn Val Tyr Ile Thr Ala Asp Lys Gln Lys Asn Gly
145 150 155 160

Ile Lys Ala Asn Phe Lys Ile Arg His Asn Ile Glu Asp Gly Ser Val
165 170 175

Gln Leu Ala Asp His Tyr Gln Gln Asn Thr Pro Ile Gly Asp Gly Pro
180 185 190

Val Leu Leu Pro Asp Asn His Tyr Leu Ser Thr Gln Ser Ala Leu Ser
195 200 205

Lys Asp Pro Asn Glu Lys Arg Asp His Met Val Leu Leu Glu Phe Val
210 215 220

Thr Ala Ala Gly Ile Thr Leu Gly Met Asp Glu Leu Tyr Lys
225 230 235

<210> 3
<211> 789
<212> DNA
<213> artificial

<220>

<223> Séquence nucléotidique de la ECFP recombinante (EFCPr)

<400> 3

```
atgtcgtact accatcacca tcaccatcac gattacgata tcccaacgac cgaaaacctg     60
tattttcagg gcgccgtgag caagggcgag gagctgttca ccggggtggt gcccatcctg    120
gtcgagctgg acggcgacgt aaacggccac aagttcagcg tgtccggcga gggcgagggc    180
gatgccacct acggcaagct gaccctgaag ttcatctgca ccaccggcaa gctgcccgtg    240
ccctggccca ccctcgtgac caccctgacc tggggcgtgc agtgcttcag ccgctacccc    300
gaccacatga agcagcacga cttcttcaag tccgccatgc ccgaaggcta cgtccaggag    360
cgcaccatct tcttcaagga cgacggcaac tacaagaccc gcgccgaggt gaagttcgag    420
ggcgacaccc tggtgaaccg catcgagctg aagggcatcg acttcaagga ggacggcaac    480
atcctggggc acaagctgga gtacaactac atcagccaca acgtctatat caccgccgac    540
aagcagaaga acggcatcaa ggccaacttc aagatccgcc acaacatcga ggacggcagc    600
gtgcagctcg ccgaccacta ccagcagaac acccccatcg gcgacggccc cgtgctgctg    660
cccgacaacc actacctgag cacccagtcc gccctgagca aagaccccaa cgagaagcgc    720
gatcacatgg tcctgctgga gttcgtgacc gccgccggga tcactctcgg catggacgag    780
ctgtacaag                                                            789
```

<210> 4
<211> 263
<212> PRT
<213> artificial

<220>
<223> Séquence d’acides aminés de la ECFP recombinante (ECFPr)

<400> 4

```
Met Ser Tyr Tyr His His His His His His Asp Tyr Asp Ile Pro Thr
1               5                   10                  15

Thr Glu Asn Leu Tyr Phe Gln Gly Ala Val Ser Lys Gly Glu Glu Leu
            20                  25                  30

Phe Thr Gly Val Val Pro Ile Leu Val Glu Leu Asp Gly Asp Val Asn
        35                  40                  45

Gly His Lys Phe Ser Val Ser Gly Glu Gly Glu Gly Asp Ala Thr Tyr
    50                  55                  60

Gly Lys Leu Thr Leu Lys Phe Ile Cys Thr Thr Gly Lys Leu Pro Val
65                  70                  75                  80

Pro Trp Pro Thr Leu Val Thr Thr Leu Thr Trp Gly Val Gln Cys Phe
                85                  90                  95

Ser Arg Tyr Pro Asp His Met Lys Gln His Asp Phe Phe Lys Ser Ala
            100                 105                 110
```

```
Met Pro Glu Gly Tyr Val Gln Glu Arg Thr Ile Phe Phe Lys Asp Asp
        115                 120                 125

Gly Asn Tyr Lys Thr Arg Ala Glu Val Lys Phe Glu Gly Asp Thr Leu
    130                 135                 140

Val Asn Arg Ile Glu Leu Lys Gly Ile Asp Phe Lys Glu Asp Gly Asn
145                 150                 155                 160

Ile Leu Gly His Lys Leu Glu Tyr Asn Tyr Ile Ser His Asn Val Tyr
                165                 170                 175

Ile Thr Ala Asp Lys Gln Lys Asn Gly Ile Lys Ala Asn Phe Lys Ile
            180                 185                 190

Arg His Asn Ile Glu Asp Gly Ser Val Gln Leu Ala Asp His Tyr Gln
        195                 200                 205

Gln Asn Thr Pro Ile Gly Asp Gly Pro Val Leu Leu Pro Asp Asn His
    210                 215                 220

Tyr Leu Ser Thr Gln Ser Ala Leu Ser Lys Asp Pro Asn Glu Lys Arg
225                 230                 235                 240

Asp His Met Val Leu Leu Glu Phe Val Thr Ala Ala Gly Ile Thr Leu
                245                 250                 255

Gly Met Asp Glu Leu Tyr Lys
            260
```

<210> 5
<211> 714
<212> DNA
<213> artificial

<220>
<223> Séquence nucléotidique de la ECFP (148G)

<400> 5

gtgagcaagg gcgaggagct gttcaccggg gtggtgccca tcctggtcga gctggacggc 60

gacgtaaacg gccacaagtt cagcgtgtcc ggcgagggcg agggcgatgc cacctacggc 120

aagctgaccc tgaagttcat ctgcaccacc ggcaagctgc ccgtgccctg gcccaccctc 180

gtgaccaccc tgacctgggg cgtgcagtgc ttcagccgct accccgacca catgaagcag 240

cacgacttct tcaagtccgc catgcccgaa ggctacgtcc aggagcgcac catcttcttc 300

aaggacgacg gcaactacaa gacccgcgcc gaggtgaagt tcgagggcga caccctggtg 360

aaccgcatcg agctgaaggg catcgacttc aaggaggacg gcaacatcct ggggcacaag 420

ctggagtaca actacatcag cggcaacgtc tatatcaccg ccgacaagca gaagaacggc 480

atcaaggcca acttcaagat ccgccacaac atcgaggacg gcagcgtgca gctcgccgac 540

cactaccagc agaacacccc catcggcgac ggccccgtgc tgctgcccga caaccactac 600

ctgagcaccc agtccgccct gagcaaagac cccaacgaga agcgcgatca catggtcctg 660

ctggagttcg tgaccgccgc cgggatcact ctcggcatgg acgagctgta caag 714

<210> 6
<211> 238
<212> PRT
<213> artificial

<220>
<223> Séquence d’acides aminés de la ECFP (148G)

<400> 6

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Gly Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 7
<211> 714

<212> DNA
<213> artificial

<220>
<223> Séquence nucléotidique de la ECFP (148S)

<400> 7

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcagccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag ctccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 8
<211> 238
<212> PRT
<213> artificial

<220>
<223> Séquence d'acides aminés de la ECFP (148S)

<400> 8

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
```

```
                20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Ser Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 9
<211> 714
<212> DNA
<213> artificial

<220>
<223> Séquence nucléotidique de la ECFP (65S)

<400> 9

```
gtgagcaagg gcgaggagct gttcaccggg gtggtgccca tcctggtcga gctggacggc     60
gacgtaaacg gccacaagtt cagcgtgtcc ggcgagggcg agggcgatgc cacctacggc    120
aagctgaccc tgaagttcat ctgcaccacc ggcaagctgc ccgtgccctg gcccaccctc    180
gtgaccaccc tgagctgggg cgtgcagtgc ttcagccgct accccgacca catgaagcag    240
cacgacttct tcaagtccgc catgcccgaa ggctacgtcc aggagcgcac catcttcttc    300
aaggacgacg gcaactacaa gacccgcgcc gaggtgaagt tcgagggcga caccctggtg    360
aaccgcatcg agctgaaggg catcgacttc aaggaggacg gcaacatcct ggggcacaag    420
ctggagtaca actacatcag ccacaacgtc tatatcaccg ccgacaagca gaagaacggc    480
atcaaggcca acttcaagat ccgccacaac atcgaggacg gcagcgtgca gctcgccgac    540
cactaccagc agaacacccc catcggcgac ggccccgtgc tgctgcccga caaccactac    600
ctgagcaccc agtccgccct gagcaaagac cccaacgaga agcgcgatca catggtcctg    660
ctggagttcg tgaccgccgc cgggatcact ctcggcatgg acgagctgta caag          714
```

<210> 10
<211> 238
<212> PRT
<213> artificial

<220>
<223> Séquence d’acides aminés de la ECFP (65S)

<400> 10

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser His Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 11
<211> 714

<212> DNA
<213> artificial

<220>
<223> Séquence nucléotidique de la ECFP (65S, 148G)

<400> 11

```
gtgagcaagg gcgaggagct gttcaccggg gtggtgccca tcctggtcga gctggacggc     60
gacgtaaacg gccacaagtt cagcgtgtcc ggcgagggcg agggcgatgc cacctacggc    120
aagctgaccc tgaagttcat ctgcaccacc ggcaagctgc ccgtgccctg gcccaccctc    180
gtgaccaccc tgagctgggg cgtgcagtgc ttcagccgct accccgacca catgaagcag    240
cacgacttct tcaagtccgc catgcccgaa ggctacgtcc aggagcgcac catcttcttc    300
aaggacgacg gcaactacaa gacccgcgcc gaggtgaagt tcgagggcga caccctggtg    360
aaccgcatcg agctgaaggg catcgacttc aaggaggacg gcaacatcct ggggcacaag    420
ctggagtaca actacatcag cggcaacgtc tatatcaccg ccgacaagca gaagaacggc    480
atcaaggcca acttcaagat ccgccacaac atcgaggacg gcagcgtgca gctcgccgac    540
cactaccagc agaacacccc catcggcgac ggccccgtgc tgctgcccga caaccactac    600
ctgagcaccc agtccgccct gagcaaagac cccaacgaga agcgcgatca catggtcctg    660
ctggagttcg tgaccgccgc cgggatcact ctcggcatgg acgagctgta caag          714
```

<210> 12
<211> 238
<212> PRT
<213> artificial

<220>
<223> Séquence d'acides aminés de la ECFP (65S, 148G)

<400> 12

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Gly Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys


         225                 230                 235
```

<210> 13
<211> 714
<212> DNA
<213> artificial

<220>
<223> Séquence nucléotidique de la ECFP (65S, 148S)

<400> 13

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcagccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag ctccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 14
<211> 238
<212> PRT
<213> artificial

<220>
<223> Séquence d’acides aminés de la ECFP (65S, 148S)

<400> 14

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Ser Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 15
<211> 714
<212> DNA

<213> artificial

<220>
<223> Séquence nucléotidique de la ECFP (72A, 145A, 148D)

<400> 15

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag cgacaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 16
<211> 238
<212> PRT
<213> artificial

<220>
<223> Séquence d’acides aminés de la ECFP (72A, 145A, 148D)

<400> 16

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Asp Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 17
<211> 714

<212> DNA
<213> artificial

<220>
<223> Séquence nucléotidique de la ECFP (72A, 145A, 148G)

<400> 17

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag cggcaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 18
<211> 238
<212> PRT
<213> artificial

<220>
<223> Séquence d'acides aminés de la ECFP (72A, 145A, 148G)

<400> 18

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
```

```
                20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Gly Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 19
<211> 714
<212> DNA
<213> artificial

<220>
<223> Séquence nucléotidique de la ECFP (72A, 145A, 148S)

<400> 19

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag ctccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 20
<211> 238
<212> PRT
<213> artificial

<220>
<223> Séquence d’acides aminés de la ECFP (72A, 145A, 148S)

<400> 20

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Ser Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 21
<211> 714

<212> DNA
<213> artificial

<220>
<223> Séquence nucléotidique de la ECFP (65S, 72A, 145A, 148D)

<400> 21

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag cgacaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 22
<211> 238
<212> PRT
<213> artificial

<220>
<223> Séquence d’acides aminés de la ECFP (65S, 72A, 145A, 148D)

<400> 22

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Asp Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys

          225                 230                 235
```

<210> 23
<211> 714
<212> DNA
<213> artificial

<220>
<223> Séquence nucléotidique de la ECFP (65S, 72A, 145A, 148G)

<400> 23

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag cggcaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 24
<211> 238
<212> PRT
<213> artificial

<220>
<223> Séquence d’acides aminés de la ECFP (65S, 72A, 145A, 148G)

<400> 24

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Gly Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 25
<211> 714
<212> DNA

<213> artificial

<220>
<223> Séquence nucléotidique de la ECFP (65S, 72A, 145A, 148S)

<400> 25

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag ctccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 26
<211> 238
<212> PRT
<213> artificial

<220>
<223> Séquence d’acides aminés de la ECFP (65S, 72A, 145A, 148S)

<400> 26

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Ser Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 27
<211> 714

<212> DNA
<213> artificial

<220>
<223> Séquence nucléotidique de la ECFP (65S, 72A, 148D, 206K)

<400> 27

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cgacaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccaagct gagcaaagac cccaacgaga agcgcgatca catggtcctg     660
ctggagttcg tgaccgccgc cgggatcact ctcggcatgg acgagctgta caag           714
```

<210> 28
<211> 238
<212> PRT
<213> artificial

<220>
<223> Séquence d’acides aminés de la ECFP (65S, 72A, 148D, 206K)

<400> 28

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
```

```
        20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Asp Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Lys Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 29
<211> 714
<212> DNA
<213> artificial

<220>
<223> Séquence nucléotidique de la ECFP (65S, 72A, 148G, 206K)

<400> 29

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cggcaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccaagct gagcaaagac cccaacgaga agcgcgatca catggtcctg     660
ctggagttcg tgaccgccgc cgggatcact ctcggcatgg acgagctgta caag           714
```

<210> 30
<211> 238
<212> PRT
<213> artificial

<220>
<223> Séquence d’acides aminés de la ECFP (65S, 72A, 148G, 206K)

<400> 30

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Gly Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Lys Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 31
<211> 714

<212> DNA
<213> artificial

<220>
<223> Séquence nucléotidique de la ECFP (65S, 72A, 148S, 206K)

<400> 31

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag ctccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccaagct gagcaaagac cccaacgaga agcgcgatca catggtcctg     660
ctggagttcg tgaccgccgc cgggatcact ctcggcatgg acgagctgta caag           714
```

<210> 32
<211> 238
<212> PRT
<213> artificial

<220>
<223> Séquence d’acides aminés de la ECFP (65S, 72A, 148S, 206K)

<400> 32

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Ser Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Lys Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys

        225                 230                 235
```

<210> 33
<211> 714
<212> DNA
<213> artificial

<220>
<223> Séquence nucléotidique de la ECFP (65S, 72A, 206K)

<400> 33

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag ccacaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccaagct gagcaaagac cccaacgaga agcgcgatca catggtcctg     660
ctggagttcg tgaccgccgc cgggatcact ctcggcatgg acgagctgta caag           714
```

<210> 34
<211> 238
<212> PRT
<213> artificial

<220>
<223> Séquence d’acides aminés de la ECFP (65S, 72A, 206K)

<400> 34

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser His Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Lys Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 35
<211> 29
<212> DNA

<213> artificial

<220>
<223> Amorce sens

<400> 35
aaggcgccgt gagcaagggc gaggagctg 29

<210> 36
<211> 30
<212> DNA
<213> artificial

<220>
<223> Amorce antisens

<400> 36
ttaagcttac ttgtacagct cgtccatgcc 30

<210> 37
<211> 31
<212> DNA
<213> artificial

<220>
<223> Séquence nucléotidique de 148Gf

<400> 37
caactacatc agcggcaacg tctatatcac c 31

<210> 38
<211> 31
<212> DNA
<213> artificial

<220>
<223> Séquence nucléotidique de 148Gr

<400> 38
ggtgatatag acgttgccgc tgatgtagtt g 31

<210> 39
<211> 31
<212> DNA
<213> artificial

<220>
<223> Séquence nucléotidique de 148Sf

<400> 39
caactacatc agctccaacg tctatatcac c 31

<210> 40
<211> 31
<212> DNA
<213> artificial

<220>

<223> Séquence nucléotidique de 148Sr

<400> 40
ggtgatatag acgttggagc tgatgtagtt g 31

<210> 41
<211> 31
<212> DNA
<213> artificial

<220>
<223> Séquence nucléotidique de 148Df

<400> 41
caactacatc agcgacaacg tctatatcac c 31

<210> 42
<211> 31
<212> DNA
<213> artificial

<220>
<223> Séquence nucléotidique de 148Dr

<400> 42
ggtgatatag acgttgtcgc tgatgtagtt g 31

<210> 43
<211> 31
<212> DNA
<213> artificial

<220>
<223> Séquence nucléotidique de 148Rf

<400> 43
caactacatc agccgcaacg tctatatcac c 31

<210> 44
<211> 31
<212> DNA
<213> artificial

<220>
<223> Séquence nucléotidique de 148Rr

<400> 44
ggtgatatag acgttgcggc tgatgtagtt g 31

<210> 45
<211> 31
<212> DNA
<213> artificial

<220>
<223> Séquence nucléotidique de 148Nf

<400> 45

gtacaactac atctccaaca acgtctatat c 31

<210> 46
<211> 31
<212> DNA
<213> artificial

<220>
<223> Séquence nucléotidique de 148Nr

<400> 46
gatatagacg ttgttggaga tgtagttgta c 31

<210> 47
<211> 31
<212> DNA
<213> artificial

<220>
<223> Séquence nucléotidique de 148Ef

<400> 47
caactacatc agcgagaacg tctatatcac c 31

<210> 48
<211> 31
<212> DNA
<213> artificial

<220>
<223> Séquence nucléotidique de 148Er

<400> 48
ggtgatatag acgttctcgc tgatgtagtt g 31

<210> 49
<211> 31
<212> DNA
<213> artificial

<220>
<223> Séquence nucléotidique de 65Sf

<400> 49
cgtgaccacc ctgagctggg gcgtgcagtg c 31

<210> 50
<211> 31
<212> DNA
<213> artificial

<220>
<223> Séquence nucléotidique de 65Sr

<400> 50
gcactgcacg ccccagctca gggtggtcac g 31

<210> 51

<211> 31
<212> DNA
<213> artificial

<220>
<223> Séquence nucléotidique de 72Af

<400> 51
cgtgcagtgc ttcgcccgct accccgacca c 31

<210> 52
<211> 31
<212> DNA
<213> artificial

<220>
<223> Séquence nucléotidique de 72Ar

<400> 52
gtggtcgggg tagcgggcga agcactgcac g 31

<210> 53
<211> 31
<212> DNA
<213> artificial

<220>
<223> Séquence nucléotidique de 145Af

<400> 53
gctggagtac aacgccatca gcgacaacgt c 31

<210> 54
<211> 31
<212> DNA
<213> artificial

<220>
<223> Séquence nucléotidique de 145Ar

<400> 54
gacgttgtcg ctgatggcgt tgtactccag c 31

<210> 55
<211> 34
<212> DNA
<213> artificial

<220>
<223> Séquence nucléotidique de 206Kf

<400> 55
cctgagcacc cagtccaagc tgagcaaaga cccc 34

<210> 56
<211> 34
<212> DNA
<213> artificial

<220>
<223> Séquence nucléotidique de 206Kr

<400> 56
ggggtctttg ctcagcttgg actgggtgct cagg 34

<210> 57
<211> 714
<212> DNA
<213> artificial

<220>
<223> Séquence nucléotidique de la ECFP (148A)

<400> 57

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcagccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cgccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 58
<211> 238
<212> PRT
<213> artificial

<220>
<223> Séquence d’acides aminés de la ECFP (148A)

<400> 58

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Ala Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 59
<211> 714
<212> DNA

<213> artificial

<220>
<223> Séquence nucléotidique de la ECFP (65S, 148A)

<400> 59

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcagccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cgccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 60
<211> 238
<212> PRT
<213> artificial

<220>
<223> Séquence d’acides aminés de la ECFP (65S, 148A)

<400> 60

```
Val Ser Lys Gly Glu Glu Leu Phe Thr Gly Val Val Pro Ile Leu Val
```

```
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Ala Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 61
<211> 714
<212> DNA

<213> artificial

<220>
<223> Séquence nucléotidique de la ECFP (72A, 145A, 148A)

<400> 61

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag cgccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 62
<211> 238
<212> PRT
<213> artificial

<220>
<223> Séquence d’acides aminés de la ECFP (72A, 145A, 148A)

<400> 62

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Ala Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 63
<211> 714

<212> DNA
<213> artificial

<220>
<223> Séquence nucléotidique de la ECFP (65S, 72A, 145A, 148A)

<400> 63

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca acgccatcag cgccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 64
<211> 238
<212> PRT
<213> artificial

<220>
<223> Séquence d’acides aminés de la ECFP (65S, 72A, 145A, 148A)

<400> 64

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Ala Ile Ser Ala Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val

        210                 215                 220

    Thr Ala Ala Gly Ile Thr Leu Gly Met Asp Glu Leu Tyr Lys
    225                 230                 235
```

<210> 65

<211> 714
<212> DNA
<213> artificial

<220>
<223> Séquence nucléotidique de la ECFP (65S, 72A, 148A, 206K)

<400> 65

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcgcccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cgccaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccaagct gagcaaagac cccaacgaga agcgcgatca catggtcctg     660
ctggagttcg tgaccgccgc cgggatcact ctcggcatgg acgagctgta caag           714
```

<210> 66
<211> 238
<212> PRT
<213> artificial

<220>
<223> Séquence d’acides aminés de la ECFP (65S, 72A, 148A, 206K)

<400> 66

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60
```

```
Ser Trp Gly Val Gln Cys Phe Ala Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Ala Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Lys Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 67
<211> 31
<212> DNA
<213> artificial

<220>
<223> Séquence nucléotidique de 148Af

<400> 67
caactacatc agcgccaacg tctatatcac c 31

<210> 68
<211> 31
<212> DNA
<213> artificial

<220>

<223> Séquence nucléotidique de 148Ar

<400> 68
ggtgatatag acgttggcgc tgatgtagtt g 31

<210> 69
<211> 75
<212> DNA
<213> artificial

<220>
<223> séquence polyhistidine et une séquence de site de clivage par la protéase TEV

<400> 69

atgtcgtact accatcacca tcaccatcac gattacgata tcccaacgac cgaaaacctg 60

tattttcagg gcgcc 75

<210> 70
<211> 25
<212> PRT
<213> artificial

<220>
<223> séquence polyhistidine et une séquence de site de clivage par la protéase TEV

<400> 70

Met Ser Tyr Tyr His His His His His His Asp Tyr Asp Ile Pro Thr
1               5                   10                  15

Thr Glu Asn Leu Tyr Phe Gln Gly Ala
            20                  25

<210> 71
<211> 714
<212> DNA
<213> artificial

<220>
<223> séquence nucléotidique de la ECFP (148E)

<400> 71

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgacctgggg cgtgcagtgc ttcagccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cgaaaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 72
<211> 238
<212> PRT
<213> artificial

<220>
<223> séquence d’acides aminés de la ECFP (148E)

<400> 72

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Glu Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 73
<211> 714
<212> DNA

<213> artificial

<220>
<223> séquence nucléotidique de la ECFP (65S, 148E)

<400> 73

```
gtgagcaagg gcgaggagct gttcaccggg gtggtgccca tcctggtcga gctggacggc      60
gacgtaaacg gccacaagtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcagccgct accccgacca catgaagcag     240
cacgacttct tcaagtccgc catgcccgaa ggctacgtcc aggagcgcac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cgaaaacgtc tatatcaccg ccgacaagca gaagaacggc     480
atcaaggcca acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccgccct gagcaaagac cccaacgaga agcgcgatca catggtcctg     660
ctggagttcg tgaccgccgc cgggatcact ctcggcatgg acgagctgta caag           714
```

<210> 74
<211> 238
<212> PRT
<213> artificial

<220>
<223> séquence d’acides aminés de la ECFP (65S, 148E)

<400> 74

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30
```

```
Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Glu Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 75
<211> 714
<212> DNA
<213> artificial

<220>
<223> séquence nucléotidique de la ECFP (65S, 148D)

<400> 75

```
gtgagcaagg gcgaggagct gttcaccggg gtggtgccca tcctggtcga gctggacggc        60
gacgtaaacg gccacaagtt cagcgtgtcc ggcgagggcg agggcgatgc cacctacggc       120
aagctgaccc tgaagttcat ctgcaccacc ggcaagctgc ccgtgccctg gcccaccctc       180
gtgaccaccc tgagctgggg cgtgcagtgc ttcagccgct accccgacca catgaagcag       240
cacgacttct tcaagtccgc catgcccgaa ggctacgtcc aggagcgcac catcttcttc       300
aaggacgacg gcaactacaa gacccgcgcc gaggtgaagt tcgagggcga caccctggtg       360
aaccgcatcg agctgaaggg catcgacttc aaggaggacg gcaacatcct ggggcacaag       420
ctggagtaca actacatcag cgacaacgtc tatatcaccg ccgacaagca gaagaacggc       480
atcaaggcca acttcaagat ccgccacaac atcgaggacg gcagcgtgca gctcgccgac       540
cactaccagc agaacacccc catcggcgac ggccccgtgc tgctgcccga caaccactac       600
ctgagcaccc agtccgccct gagcaaagac cccaacgaga agcgcgatca catggtcctg       660
ctggagttcg tgaccgccgc cgggatcact ctcggcatgg acgagctgta caag             714
```

<210> 76
<211> 238
<212> PRT
<213> artificial

<220>
<223> séquence d'acides aminés de la ECFP (65S, 148D)

<400> 76

```
Val Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Asp Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235
```

<210> 77
<211> 28
<212> DNA

<213> artificial

<220>
<223> séquence nucléotidique TagRFPf

<400> 77
attagagctc atggtgtcta agggcgaa 28

<210> 78
<211> 30
<212> DNA
<213> artificial

<220>
<223> séquence nucléotidique TagFRPr

<400> 78
ataatgaatt cttaattaag tttgtgcccc 30

<210> 79
<211> 714
<212> DNA
<213> artificial

<220>
<223> séquence nucléotidique de la ECFP (26R, 65S, 148G, 164H, 206K)

<400> 79

```
gtgagcaagg gcgaggagct gttcaccggg gtggtgccca tcctggtcga gctggacggc      60
gacgtaaacg gccacaggtt cagcgtgtcc ggcgagggcg agggcgatgc cacctacggc     120
aagctgaccc tgaagttcat ctgcaccacc ggcaagctgc ccgtgccctg gcccaccctc     180
gtgaccaccc tgagctgggg cgtgcagtgc ttcagccgct accccgacca catgaagcag     240
cacgacttct tcaagtccgc catgcccgaa ggctacgtcc aggagcgtac catcttcttc     300
aaggacgacg gcaactacaa gacccgcgcc gaggtgaagt tcgagggcga caccctggtg     360
aaccgcatcg agctgaaggg catcgacttc aaggaggacg gcaacatcct ggggcacaag     420
ctggagtaca actacatcag cggcaacgtc tatatcaccg ccgacaagca gaagaacggc     480
atcaaggccc acttcaagat ccgccacaac atcgaggacg gcagcgtgca gctcgccgac     540
cactaccagc agaacacccc catcggcgac ggccccgtgc tgctgcccga caaccactac     600
ctgagcaccc agtccaagct gagcaaagac cccaacgaga agcgcgatca catggtcctg     660
ctggagttcg tgaccgccgc cgggatcact ctcggcatgg acgagctgta caag           714
```

<210> 80
<211> 238
<212> PRT
<213> artificial

<220>
<223> séquence d'acides aminés de la ECFP (26R, 65S, 148G, 164H, 206K)

<400> 80

```
Val Ser Lys Gly Glu Glu Leu Phe Thr Gly Val Val Pro Ile Leu Val
1               5                   10                  15

Glu Leu Asp Gly Asp Val Asn Gly His Arg Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140
```

Tyr Ile Ser Gly Asn Val Tyr Ile Thr Ala Asp Lys Gln Lys Asn Gly
145 150 155 160

Ile Lys Ala His Phe Lys Ile Arg His Asn Ile Glu Asp Gly Ser Val
165 170 175

Gln Leu Ala Asp His Tyr Gln Gln Asn Thr Pro Ile Gly Asp Gly Pro
180 185 190

Val Leu Leu Pro Asp Asn His Tyr Leu Ser Thr Gln Ser Lys Leu Ser
195 200 205

Lys Asp Pro Asn Glu Lys Arg Asp His Met Val Leu Leu Glu Phe Val
210 215 220

Thr Ala Ala Gly Ile Thr Leu Gly Met Asp Glu Leu Tyr Lys
225 230 235

<210> 81
<211> 681
<212> DNA
<213> artificial

<220>
<223> séquence nucléotidique de la ECFP (26R, 65S, 148G, 164H, 206K) tronquée en C-terminal

<400> 81

gtgagcaagg gcgaggagct gttcaccggg gtggtgccca tcctggtcga gctggacggc 60

gacgtaaacg gccacaggtt cagcgtgtcc ggcgagggcg agggcgatgc cacctacggc 120

aagctgaccc tgaagttcat ctgcaccacc ggcaagctgc ccgtgccctg gcccaccctc 180

gtgaccaccc tgagctgggg cgtgcagtgc ttcagccgct accccgacca catgaagcag 240

cacgacttct tcaagtccgc catgcccgaa ggctacgtcc aggagcgtac catcttcttc 300

aaggacgacg gcaactacaa gacccgcgcc gaggtgaagt tcgagggcga caccctggtg 360

aaccgcatcg agctgaaggg catcgacttc aaggaggacg gcaacatcct ggggcacaag 420

ctggagtaca actacatcag cggcaacgtc tatatcaccg ccgacaagca gaagaacggc 480

atcaaggccc acttcaagat ccgccacaac atcgaggacg gcagcgtgca gctcgccgac 540

cactaccagc agaacacccc catcggcgac ggccccgtgc tgctgcccga caaccactac 600

ctgagcaccc agtccaagct gagcaaagac cccaacgaga agcgcgatca catggtcctg 660

ctggagttcg tgaccgccgc c 681

<210> 82
<211> 227
<212> PRT

<213> artificial

<220>
<223> séquence d’acides aminés de la ECFP (26R, 65S, 148G, 164H, 206K) tronquée en C-terminal

<400> 82

```
Val Ser Lys Gly Glu Glu Leu Phe Thr Gly Val Val Pro Ile Leu Val
1               5                   10                  15

Glu Leu Asp Gly Asp Val Asn Gly His Arg Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Ser Trp Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Ile Ser Gly Asn Val Tyr Ile Thr Ala Asp Lys Gln Lys Asn Gly
145                 150                 155                 160

Ile Lys Ala His Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Lys Leu Ser
        195                 200                 205

Lys Asp Pro Asn Glu Lys Arg Asp His Met Val Leu Leu Glu Phe Val
    210                 215                 220

Thr Ala Ala
225
```